# EUROPEAN PATENT APPLICATION

(11) **EP 4 834 901 A1**
(43) Date of publication of application: **04.11.2026**
(21) Application number: 24910182.5
(22) Date of filing: 18.10.2024
(51) Int. Cl.: C07D 471/04, A61K 31/506, A61K 31/44, A61K 31/47, A61P 35/00, A61P 29/00, A61P 3/10, A61P 11/06, A61P 37/06, A61P 19/02, A61P 19/08, A61P 21/00, A61P 17/06

(54) **PARP7 INHIBITOR AND USE THEREOF**

(30) Priority: 26.12.2023 CN 202311806914
(71) Applicant: Novostar Pharmaceuticals, Ltd., Shanghai 201210 (CN)
(72) Inventor: LI, Bing, Shanghai 201210 (CN); ZHANG, Ke, Shanghai 201210 (CN); ZUO, Xiaofei, Shanghai 201210 (CN); NING, Wenxing, Shanghai 201210 (CN); HUANG, Qionglin, Shanghai 201210 (CN); CHEN, Qin, Shanghai 201210 (CN)
(74) Representative: EIP
(86) International application number: PCT/CN2024/125765
(87) International publication number: WO 2025/139240

(57) **Abstract**

A PARP7 inhibitor, which is a compound of formula (I) or a pharmaceutically acceptable salt, solvate, ester, acid, metabolite or prodrug thereof. The present application also relates to the use of the PARP7 inhibitor in the selective inhibition of PARP7 activity, or in the treatment or prevention of diseases, disorders, or conditions regulated or affected by PARP7 activity, or in which PARP7 activity or overexpression is involved.

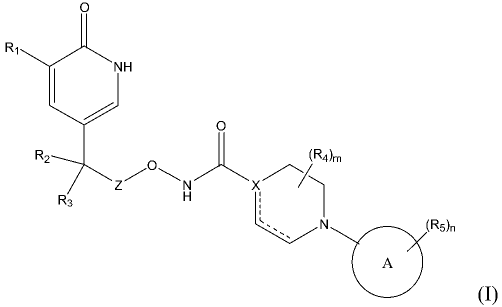

## Description

### Technical Field

This invention belongs to the pharmaceutical field, specifically relating to compounds having multiple nitrogen heterocycles and nitrogen-containing aryl structures such as 2-pyridone, their preparation methods, and methods and uses for treating and/or preventing diseases.

### Background Art

PARP (poly-ADP-ribose polymerase) is a family of 17 enzymes that use nicotinamide adenine dinucleotide (NAD+) as a substrate to transfer ADP-ribose (ADPR) groups onto target proteins. As a multifunctional post-translational modification enzyme, PARP exists in most eukaryotic cells. It is activated by recognizing structurally damaged DNA fragments and is considered a DNA damage sensor, participating in a series of cellular processes such as DNA repair and genome stability. The PARP family is divided into two categories: poly-PARPs and mono-PARPs. Poly-PARPs catalyze the sequential transfer of multiple ADPR molecules to substrate proteins, with poly-ADPR chains reaching hundreds of units, while mono-PARPs catalyze the transfer of a single ADPR group onto the substrate. Therefore, poly-PARPs and mono-PARPs have different ADP-ribosylation modification substrates and play different roles in cell signal transduction and protein function regulation, representing two different types of therapeutic targets.

PARP7 (TIPARP) is a mono-PARP that is a key factor regulating innate immunity, transcription factor activity, and cellular stress responses. Multiple studies have shown that PARP7 plays a crucial role in innate immune signaling pathways, particularly as a negative regulator of type I interferon antiviral responses. Knockout of PARP7 can enhance the expression of nucleic acid sensor agonists or virus-induced interferon-β (IFN-β) in different cell types. PARP7 acts as an inhibitor of aryl hydrocarbon receptor (AHR) activity and is upregulated in cellular stress activated by AHR following viral infection or exposure to cigarette smoke. Furthermore, PARP7 is also regulated by liver X receptors (LXRs) and hypoxia-inducible factor 1 (HIF-1).

In cancer cells or virus-infected cells, intracellular free nucleic acid fragments trigger innate immune sensing through the TBK1-mediated type I IFN pathway. This leads to phosphorylation of the cytokine regulator IRF-3, which then translocates into the nucleus and binds to transcriptional coactivators, thereby promoting the transcription of type I IFNs. The transcription factor STAT1 is a key downstream effector in the IFN signaling pathway. Once IFN binds to its receptor, STAT1 is phosphorylated by the kinase JAK1, forming a dimer that translocates to the nucleus, regulating the transcription of the interferon-stimulated genes (ISGs). This, in turn, recruits dendritic cells, macrophages, lymphocytes, and other immune cells to recognize and kill cancer cells. PARP7 is expressed at low levels in normal cells but is overactive in various types of tumors. Highly expressed PARP7 acts as a brake in cancer cells, modifying the key kinase TBK1 through single ADP-ribosylation, preventing the phosphorylation of IRF-3, and consequently preventing the production of type I IFNs, thus allowing them to evade recognition and killing by immune cells. Therefore, inhibition of PARP7 can restore the response of the type I interferon signaling pathway to nucleic acids, directly inhibit tumor cell proliferation, and activate the immune system, effectively preventing tumor immune escape, thereby making tumor elimination possible.

PARP7 may also be a target for inflammation therapy. PARP7 negatively regulates aryl hydrocarbon receptor (AHR) and type I interferon-I (IFN-I) signaling, both of which are involved in intestinal homeostasis and immunity. Since the loss of PARP7 expression enhances the AHR and IFN-I signaling pathways, in a mouse model of dextran sulfate (DSS)-induced colitis, the loss of PARP7 expression reduced the expression of inflammatory genes, including interleukin IL-6, CXCL1, and lipocalin-2. Experimental results suggest that PARP7 may be involved in the recruitment of immune cells to inflammatory sites. Therefore, inhibiting PARP7 can reduce the severity of DSS-induced colitis in mice and inhibit the production of pro-inflammatory cytokines in tissues of patients with inflammatory bowel disease.

PARP7 is a highly attractive therapeutic target. However, to date, only one PARP7 selective inhibitor, RBN-2397, is in Phase I clinical trials for the treatment of solid tumors. Preclinical studies have shown that compound RBN-2397 can selectively inhibit the enzymatic activity of PARP7 and inhibit the growth and proliferation of various cancer cells, leading to tumor regression in mouse xenograft tumor models and syngeneic tumor models (Gozgit JM et al., Cancer Cell 2021, 39, 1-13). Phase I clinical trial data show that RBN-2397 can induce IFN pathway activation and increase the infiltration of immune cells in tumors, indicating adaptive immune induction after RBN-2397 treatment. However, RBN-2397 has poor pharmacokinetic properties in vivo, such as low plasma exposure, short plasma half-life, and low oral bioavailability.

Therefore, there is a need to develop PARP7 inhibitors with higher selectivity, improved in vivo pharmacokinetic properties, better biological activity and therapeutic effects, reduced clinical side effects, and the ability to expand to more indications such as tumors, autoimmune diseases, or inflammatory diseases.

### Summary of the Invention

This invention aims to provide a compound capable of selectively inhibiting the enzymatic activity of PARP7.

According to one aspect of the invention, a PARP7 inhibitor is provided, which is a compound of formula (I) or a pharmaceutically acceptable salt, solvate, ester, acid, metabolite or prodrug thereof,

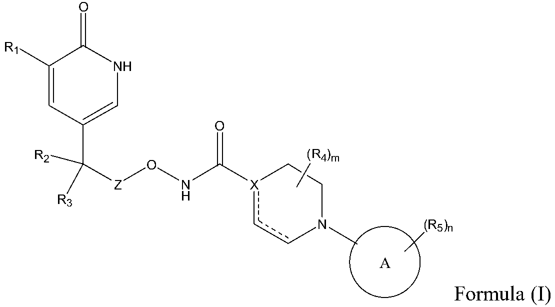

Wherein,
R₁ is selected from halogens, C1-6 haloalkyl groups, and C2-6 alkanoyl groups;
R₂ is selected from hydrogen and deuterium;
R₃ is selected from hydrogen, C1-6 alkyl groups, C1-6 hydroxyalkyl groups, and C1-6 haloalkyl; or,
R₂ and R₃ together with adjacent carbon atoms form C3-6 cycloalkyl; or
Z is selected from direct bond or methylene;
X is selected from carbon and nitrogen, indicates a double bond or a single bond, provided that when X is nitrogen, the bond connected to X is a single bond, and when X is carbon, the bond connected to X is a double bond or a single bond, and at most one double bond is connected to any atom;
m is 0, 1, or 2, and when m is 1, R₄ is selected from C1-6 alkyl; when m is 2, the two R₄ are located on different ring atoms, and the two R₄ together form an alkylene bridging bond containing 1-3 carbon atoms;
Ring A is a 5-6 membered monocyclic heteroaryl or a 9-10 membered bicyclic heteroaryl having 1-3 nitrogen atoms;
n is 0, 1, or 2, and each R₅ is independently selected from nitrile, halogen, C3-6 cycloalkyl, C1-6 alkyl, C2-6 alkenyl, C2-6 alkynyl, C1-6 haloalkyl, C2-6 alkylamido, C1-4 alkylsulfinamide, C1-4 alkylsulfonamide, and phenyl optionally substituted with one or two halogens.

In another embodiment, ring A is selected from pyrimidinyl, pyridinyl, pyrrolopyridinyl, imidazopyridinyl, and quinazolinyl; ring A is more preferably selected from pyrimidin-2-yl, pyridin-2-yl, pyrrolo[2,3-c]pyridin-7-yl, imidazo[4,5-c]pyridin-4-yl, and quinazolin-2-yl; ring A is most preferably pyrimidin-2-yl or imidazo[4,5-c]pyridin-4-yl.

In a preferred embodiment, n is 1.

In another preferred embodiment, R₅ is selected from halogen, C3-6 cycloalkyl, C1-3 haloalkyl, and phenyl optionally substituted with one halogen; R₅ is more preferably selected from trifluoromethyl or cyclopropyl; and /or, the substitution position of R₅ on ring A is para-position relative to the connection position of ring A with the nitrogen-containing six-membered heterocyclic group.

In another embodiment, X is N, and both are single bonds; and /or,
m is 0, 1, or 2, more preferably 0 or 1;
when m is 1, R₄ is a C1-3 alkyl, more preferably methyl; and /or, R₄ is located ortho to the X atom of the nitrogen-containing six-membered heterocyclic group;
when m is 2, the two R₄ on different ring atoms together form a bridging bond selected from methylene or ethylene; and /or, the two R₄ are respectively located ortho to the X atom of the nitrogen-containing six-membered heterocyclic group or respectively located meta to the X atom of the nitrogen-containing six-membered heterocyclic group.

In other embodiments, X is C or CH, and one of the two is a double bond and the other is a single bond; more preferably, X is C, and one of the two bonds that connects to the X atom is a double bond, and the other is a single bond; and /or,
m is 0 or 1, more preferably 0;
when m is 1, R₄ is C1-3 alkyl, more preferably methyl.

In a preferred aspect of the invention, Z is a direct bond.

In another preferred embodiment, R₂ is hydrogen; and /or,

R₃ is selected from hydrogen, C1-3 alkyl, and C1-3 hydroxyalkyl; R₃ is more preferably methyl or ethyl.

In other preferred embodiments, R₁ is C1-3 haloalkyl, more preferably trifluoromethyl.

In another aspect of the invention, a pharmaceutical composition is provided, comprising the compound of the invention, and a pharmaceutically acceptable carrier or excipient, and optionally other therapeutic agents.

Other aspects of the invention also relate to methods or uses of the compounds of the invention, or pharmaceutically acceptable salts, solvates, esters, acids, metabolites, or prodrugs thereof, to selectively inhibit PARP7 activity, or the use of the compounds of the invention, or pharmaceutically acceptable salts, solvates, esters, acids, metabolites, or prodrugs thereof, in the preparation of medicaments for selectively inhibiting PARP7 activity.

Further aspects of the invention also relate to methods or uses of the compound of the invention, or a pharmaceutically acceptable salt, solvate, ester, acid, metabolite, or prodrug thereof, for treating or preventing diseases, disorders, or conditions regulated or affected by PARP7 activity, or wherein PARP7 activity or overexpression is involved, or the use of the compound of the invention, or a pharmaceutically acceptable salt, solvate, ester, acid, metabolite, or prodrug thereof, in the preparation of medicaments for treating or preventing diseases, disorders, or conditions regulated or affected by PARP7 activity, or wherein PARP7 activity or overexpression is involved.

Preferably, diseases, disorders, or conditions regulated or affected by PARP7 activity, or wherein PARP7 activity or overexpression is a hyperproliferative disease, particularly cancer, autoimmune, or inflammatory diseases.

In a more preferred aspect, the disease, disorder, or ailment is selected from cancer, including but not limited to squamous cell carcinoma of the lung, adenocarcinoma of the lung, large cell lung cancer, small cell lung cancer, head and neck squamous cell carcinoma, breast cancer, pancreatic cancer, pancreatic ductal adenocarcinoma, colorectal cancer, melanoma, ovarian cancer, esophageal squamous cell carcinoma, gastric cancer, liver cancer, oral cancer, urothelial carcinoma, prostate cancer, bladder cancer, renal cell carcinoma, gastrointestinal stromal tumor, cervical cancer, endometrial cancer, rhabdomyosarcoma, fibrosarcoma, neuroendocrine tumor, mesothelioma, brain cancer, and malignant glioma, etc.

In other preferred aspects, the disease, disorder or symptom is selected from autoimmune or inflammatory diseases, including but not limited to ulcerative colitis, Crohn's disease, multiple sclerosis, autoimmune liver disease, type I diabetes, bronchial asthma, systemic lupus erythematosus, rheumatoid arthritis, ankylosing spondylitis, juvenile idiopathic arthritis, psoriasis, polymyositis, and dermatomyositis, etc.

### Brief Description of the Drawings

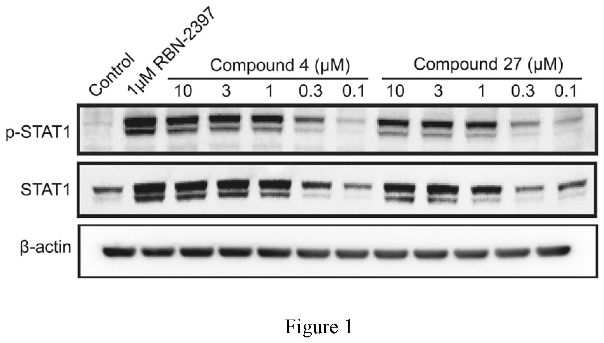

Figure 1 is a diagram showing the dose-dependent restoration of the type I interferon signaling pathway in CT26 cells by the preferred compound.

### Detailed Description of the Embodiments

### Definitions

Unless otherwise defined, all technical and scientific terms used herein have the meanings commonly understood by one of ordinary skill in the art to which this invention pertains. In this specification, the singular form also includes the plural form unless the context clearly indicates otherwise. All publications, patent applications, patents, or other references mentioned herein are incorporated herein by reference. In the event of any conflict, this specification (including the definitions) shall prevail. Furthermore, the materials, methods, and examples are illustrative only and are not intended to limit the scope of the invention.

Unless otherwise stated, the invention employs conventional methods within the scope of the art, such as mass spectrometry, NMR, HPLC, protein chemistry, biochemistry, recombinant DNA technology, and pharmacology. Unless specifically defined, the nomenclature and laboratory procedures and techniques relating to analytical chemistry, synthetic organic chemistry, and medicinal chemistry described herein are known to those skilled in the art. Generally, the foregoing techniques and procedures can be performed by conventional methods well known in the art and described in various general and more specific literatures, which are cited and discussed herein.

The term "alkyl" refers to an aliphatic hydrocarbon group, which may be a branched or straight-chain alkyl group. Depending on the structure, an alkyl group may be a monovalent or divalent group (i.e., an alkylene group). In this invention, the alkyl group is preferably an alkyl group having 1-8 carbon atoms, more preferably a "lower alkyl group" having 1-6 carbon atoms, and even more preferably an alkyl group having 1-4 carbon atoms. Typical alkyl groups include, but are not limited to, methyl, ethyl, propyl, butyl, pentyl, hexyl, etc. It should be understood that "alkyl" as used herein includes all possible configurations and conformations of the alkyl group, for example, "propyl" as used herein includes n-propyl and isopropyl, "butyl" includes n-butyl, isobutyl, and tert-butyl, "pentyl" includes n-pentyl, isopentyl, neopentyl, tert-pentyl, and pent-3-yl, etc.

The term "alkoxy" means -O-alkyl, where alkyl is as defined herein. Typical alkoxy groups include, but are not limited to, methoxy, ethoxy, propoxy, butoxy, pentoxy, hexoxy, etc.

The term "alkoxyalkyl" means that an alkyl group as defined herein is substituted with an alkoxy group as defined herein.

The term "cycloalkyl" refers to a monocyclic or polycyclic group containing only carbon and hydrogen. Cycloalkyl groups include groups having 3-12 ring atoms. Depending on the structure, a cycloalkyl group can be a monovalent or bivalent group (e.g., a cycloalkylene group). In this invention, the cycloalkyl group is preferably a cycloalkyl group having 3-8 carbon atoms, more preferably a "lower cycloalkyl group" having 3-6 carbon atoms. Examples of cycloalkyl groups include, but are not limited to, cyclopropyl, cyclobutyl, cyclopentyl, cyclohexyl, cycloheptyl, cyclooctyl, cyclopentenyl, cyclohexenyl, cycloheptenyl, and adamantyl.

The term "alkyl (cycloalkyl)" or "cycloalkylalkyl" means that an alkyl group as defined herein is substituted with a cycloalkyl group as defined herein. Non-limiting cycloalkylalkyl groups include cyclopropylmethyl, cyclobutylmethyl, cyclopentylmethyl, cyclohexylmethyl, etc.

The term "aromatic group" refers to a planar ring having a delocalized π-electron system and containing 4n+2 π electrons, where n is an integer. An aromatic ring can consist of five, six, seven, eight, nine, or more than nine atoms. The aromatic group can be optionally substituted. The term "aromatic group" includes carbocyclic aryl (e.g., phenyl) and heterocyclic aryl (or "heteroaryl" or "heteroaromatic") groups (e.g., pyridine). The term includes monocyclic or fused-ring polycyclic (i.e., rings sharing adjacent carbon atom pairs) groups.

As used herein, the term "aryl" means that each atom constituting the ring in an aromatic ring is a carbon atom. The aryl ring can consist of five, six, seven, eight, nine, or more than nine atoms. The aryl group can be optionally substituted. Examples of aryl groups include, but are not limited to, phenyl, naphthyl, phenanthryl, anthracenyl, fluorenyl, and indenyl. Depending on the structure, the aryl group can be a monovalent or bivalent group (i.e., arylene).

The term "aryloxy" means -O-aryl, where aryl is as defined herein.

The term "heteroaryl" refers to an aryl group that includes one or more cyclic heteroatoms selected from nitrogen, oxygen, and sulfur. An N-containing "heteroaryl" moiety means that at least one skeletal atom on the ring of the aryl group is a nitrogen atom. Depending on the structure, a heteroaryl group can be a monovalent group or a bivalent group (i.e., a heteroarylene). Examples of heteroaryl groups include, but are not limited to, pyridinyl, imidazolyl, pyrimidinyl, pyrazolyl, triazolyl, pyrazinyl, tetrazolyl, furanyl, thienyl, isoxazolyl, thiazolyl, oxazolyl, isothiazolyl, pyrrolyl, quinolinyl, isoquinolinyl, indolyl, benzimidazolyl, benzofuranyl, indazolyl, indazinyl, phthalazinyl, pyridazinyl, isoindolyl, pteridinyl, purinyl, oxadiazolyl, thiadiazolyl, furazonyl, benzofuranyl, benzothiopheneyl, benzothiazolyl, benzooxazolyl, quinazolinyl, naphthyridinyl, and furanopyridinyl.

The term "alkyl (aryl)" or "aralkyl" means that an alkyl group as defined herein is substituted with an aryl group as defined herein. Non-limiting alkyl (aryl) includes benzyl, phenethyl, etc.

The term "alkyl (heteroaryl)" or "heteroarylalkyl" means that an alkyl group as defined herein is substituted with a heteroaryl group as defined herein.

As used herein, the term "heteroalkyl" means that one or more skeletal chain atoms in an alkyl group as defined herein are heteroatoms, such as oxygen, nitrogen, sulfur, silicon, phosphorus, or combinations thereof. The heteroatoms (one or more) can be located at any position within the heteroalkyl group or at a position where the heteroalkyl group is attached to the rest of the molecule.

As used herein, the term "heterocyclic alkyl" or "heterocyclic group" refers to a non-aromatic ring in which one or more of the constituent atoms of the ring are heteroatoms selected from nitrogen, oxygen, and sulfur. Heterocyclic alkyl rings can be monocyclic or polycyclic, consisting of three, four, five, six, seven, eight, nine, or more atoms. The heterocyclic alkyl ring can be optionally substituted. Examples of heterocyclic alkyl groups include, but are not limited to, lactams, lactones, cycloimides, cyclothioimides, cyclocarbamates, tetrahydrothiarans, 4H-pyran, tetrahydropyran, piperidine, 1,3-dioxins, 1,3-dioxanes, 1,4-dioxins, 1,4-dioxanes, piperazines, 1,3-oxothiacyclohexane, 1,4-oxothiacyclohexadiene, 1,4-oxothiacyclohexane, tetrahydro-1,4-thiazine, 2H-1,2-oxazines, maleimides, succinimides, barbiturates, and thiobarbiturates. Acids, dioxopiperazine, hydantoin, dihydrouracil, morpholine, trioxane, hexahydro-1,3,5-triazine, tetrahydrothiophene, THF, pyrrolidone, pyrrolidine, imidazoline, pyrrolidone, imidazoline, pyrrolidine, imidazoline, imidazoline, 1,3-dioxacyclopentene, 1,3-dioxacyclopentene, 1,3-dithiocyclopentene, isoxazoline, isoxazoline, oxazoline, oxazoline, oxazoline, oxazoline ketone, thiazoline, thiazoline, and 1,3-oxothiocyclopentane. Depending on the structure, heterocyclic alkyl groups can be monovalent or bivalent (i.e., heterocyclic alkylene).

The term "alkyl (heterocyclic alkyl)" or "heterocyclic alkyl alkyl" means that an alkyl group as defined herein is substituted with a heterocyclic alkyl group as defined herein.

The term "alkoxy (heterocyclic alkyl)" or "heterocyclic alkyl alkoxy" means that an alkoxy group as defined herein is substituted with a heterocyclic alkyl group as defined herein.

The term "halogen" means fluorine, chlorine, bromine, and iodine.

The terms "haloalkyl", "haloalkoxy", and "haloheteroalkyl" include structures of alkyl, alkoxy, or heteroalkyl groups in which at least one hydrogen atom is substituted with a halogen atom. In some embodiments, if two or more hydrogen atoms are substituted with halogen atoms, the halogen atoms may be the same or different from each other.

The term "hydroxyl group" refers to the -OH group.

The term "cyano" means -CN group.

The term "ester group" refers to a chemical moiety having the formula -COOR, where R is selected from alkyl, cycloalkyl, aryl, heteroaryl (linked by a ring carbon), and heterocyclic (linked by a ring carbon).

The term "amino" means -NH₂ group.

The term "aminoacyl" means -CO-NH₂ group.

The term "alkylaminoacyl" means -CO-NH-R group, where R is an alkyl group as defined herein.

The term "amide group" or "acylamino" refers to -NR-CO-R', where R and R' are each independently hydrogen or alkyl.

The term "alkylamino" refers to an amino substituent further replaced by one or two alkyl groups, specifically the group -NRR', wherein R and R' are each independently selected from hydrogen or a lower alkyl group, provided that -NRR' is not -NH₂. "Alkylamino" includes groups of compounds in which the nitrogen of -NH₂ is attached to at least one alkyl group. Examples of alkylamino groups include, but are not limited to, methylamino, ethylamino, etc. "Dialkylamino" includes groups in which the nitrogen of -NH₂ is attached to at least two other alkyl groups. Examples of dialkylamino groups include, but are not limited to, dimethylamino, diethylamino, etc.

The terms "arylamino" and "diarylamino" refer to amino substituents further replaced by one or two aryl groups, specifically the group -NRR', wherein R and R' are each independently selected from hydrogen, a lower alkyl group, or an aryl group, wherein N is attached to at least one or two aryl groups respectively.

The term "cycloalkylamino" means an amino substituent further substituted with one or two cycloalkyl groups as defined herein.

The term "heteroalkylamino" means an amino substituent further substituted with one or two heteroalkyl groups as defined herein.

The term "aralkylamino" in this article refers to a group in which R is a lower aralkyl group and R' is a hydrogen, lower alkyl, aryl, or lower aryl group -NRR'.

The term "heteroarylamino" means an amino substituent further substituted with one or two heteroaryl groups as defined herein.

The term "heterocyclic alkylamino" means an amino group as defined herein substituted with a heterocyclic alkyl group as defined herein.

The term "alkylaminoalkyl" means an alkyl group as defined herein substituted with an alkylamino group as defined herein.

The term "aminoalkyl" means an alkyl substituent further substituted with one or more amino groups.

The term "aminoalkoxy" means an alkoxy substituent further substituted with one or more amino groups.

The term "hydroxyalkyl" or "hydroxyalkyl" means an alkyl substituent further substituted with one or more hydroxyl groups.

The term "cyanoalkyl" means an alkyl substituent further substituted with one or more cyano groups.

The term "acyl" refers to the monovalent group remaining after the removal of the hydroxyl group from an organic or inorganic oxyacid, with the general formula RM(O)-, where M is usually C.

The term 'carbonyl' refers to carbon and oxygen atoms connected by a double bond (C=O).

The terms "alkanoyl" or "alkylcarbonyl" refer to a carbonyl group further substituted with an alkyl group. Typical alkanoyl groups include, but are not limited to, acetyl, propionyl, butyryl, valeryl, and hexanoyl.

The term "arylcarbonyl" refers to a carbonyl group as defined herein that is substituted with an aryl group as defined herein.

The term "alkoxycarbonyl" refers to a carbonyl group further substituted with an alkoxy group.

The term "heterocyclic alkylcarbonyl" refers to a carbonyl group further substituted with a heterocyclic alkyl group.

The terms "alkylaminocarbonyl," "cycloalkylaminocarbonyl," "arylaminocarbonyl," "aralkylaminocarbonyl," and "heteroarylaminocarbonyl" refer to carbonyl groups as defined herein that are substituted with alkylamino, cycloalkylamino, arylamino, aralkylamino, or heteroarylamino groups as defined herein.

The terms "alkylcarbonylalkyl" or "alkanoylalkyl" refer to an alkyl group further substituted with an alkylcarbonyl group.

The terms "alkylcarbonylalkoxy" or "alkanoylalkoxy" refer to an alkoxy group further substituted with an alkylcarbonyl group.

The term "heterocyclic alkylcarbonylalkyl" refers to an alkyl group further substituted with a heterocyclic alkylcarbonyl group.

The term "thiol" refers to the -SH group. The term "alkathiol" refers to the thiol group as defined herein being replaced by an alkyl group as defined herein.

The term "sulfonyl" or "sulfonyl" refers to the functional group remaining after the removal of the hydroxyl group from a sulfonic acid, specifically the -S(=O)₂- group.

The term "sulfonylene" or "sulfinyl" refers to -S(=O)-.

The term "aminosulfonyl" or "aminosulfonyl" refers to the -S(=O)₂-NH₂ group.

The term "alkylsulfonyl" or "alkylsulfinyl" refers to the alkyl-S(=O)-.

The term "alkyl sulfonyl" or "alkyl sulfonyl" refers to -S(=O)₂-R, where R is an alkyl group.

The term "alkylaminosulfonyl" refers to a sulfonyl group as defined herein that is substituted with an alkylamino group as defined herein.

The terms "alkyl sulfonyl amino(amine)" or "alkyl sulfonyl amino(amine)" and "cycloalkyl sulfonyl amino(amine)" or "cycloalkyl sulfonyl amino(amine)" refer to an amino group as defined herein being replaced by an alkyl sulfonyl or cycloalkyl sulfonyl group as defined herein, i.e., -NH-S(=O)₂-R, where R is an alkyl group and a cycloalkyl group, respectively.

The terms "cycloalkyl sulfonyl" and "cycloalkyl sulfonyl" refer to -S(=O)₂-R, where R is a cycloalkyl group.

The term "quaternary ammonium" refers to -N⁺RR'R", where R, R', and R" are each independently selected from alkyl groups having 1 to 8 carbon atoms.

The term "optional" means that one or more events described below may or may not occur, and includes both events that occur and events that do not occur. The terms "optionally substituted" or "substituted" mean that the mentioned group can be substituted by one or more additional groups, each and independently selected from alkyl, cycloalkyl, aryl, heteroaryl, heterocyclic, hydroxyl, alkoxy, cyano, halogen, amide, nitro, haloalkyl, amino, methanesulfonyl, alkylcarbonyl, alkoxycarbonyl, heteroarylalkyl, heterocycloalkylalkyl, aminoacyl, amino protecting group, etc. Preferably, the amino protecting group is selected from neopentanoyl, tert-butoxycarbonyl, benzyloxycarbonyl, 9-fluorenmethoxycarbonyl, benzyl, p-methoxybenzyl, allyloxycarbonyl, and trifluoroacetyl, etc.

As used herein, the "pharmaceutically acceptable form" of the disclosed compound includes, but is not limited to, pharmaceutically acceptable salts, hydrates, solvates, polymorphs, esters, acids, isomers, metabolites, prodrugs, and isotopically labeled derivatives of the disclosed compound.

The term "pharmaceutically acceptable salt" in this document refers to a salt that retains the desired biological activity of the subject compound and exhibits minimal undesirable toxicological effects. These pharmaceutically acceptable salts may be prepared in situ during the final isolation and purification of the compound, or by reacting the free acid or free base form of the purified compound separately with a suitable base or acid.

A solvate or solvate compound refers to a solvation form containing a stoichiometric or non-stoichiometric solvent. Some compounds tend to trap solvent molecules in a fixed molar ratio in a crystalline solid state, thus forming a solvate. If the solvent is water, the solvate formed is a hydrate; if the solvent is an alcohol, the solvate formed is an alcohol. A hydrate is formed by the binding of one or more water molecules to a molecule of the substance, wherein the water retains its molecular state as H₂O.

A "metabolite" of the compounds disclosed herein is a derivative of the compound formed when the compound is metabolized. The term "active metabolite" refers to a biologically active derivative of the compound formed when the compound is metabolized. As used herein, the term "metabolized" refers to the sum of processes by which a particular substance is altered by an organism (including, but not limited to, hydrolysis and enzyme-catalyzed reactions, such as oxidation). Thus, enzymes can produce specific structures that are transformed into compounds. For example, cytochrome P450 catalyzes various oxidation and reduction reactions, while glucuronyl diphosphate transferases catalyze the conversion of activated glucuronic acid molecules to aromatic alcohols, aliphatic alcohols, carboxylic acids, amines, and free sulfhydryl groups. Further information on metabolism can be obtained from "The Pharmacological Basis of Therapeutics", 9th edition, McGraw-Hill (1996). Metabolites of the compounds disclosed herein can be identified by administering the compound to a host and analyzing tissue samples from that host, or by incubating the compound with hepatocytes in vitro and analyzing the resulting compound. Both methods are known in the art. In some embodiments, the metabolites of the compound are formed via an oxidation process and correspond to the respective hydroxyl-containing compounds. In some embodiments, the compound is metabolized to a pharmaceutically active metabolite.

The term "modulation" as used herein refers to an interaction with a target, directly or indirectly, to alter the activity of the target, including, by way of example only, enhancing, inhibiting, limiting, or prolonging the activity of the target.

The term 'prodrug' refers to a derivative that may not have pharmacological activity, but in some cases can be orally or parenterally administered and subsequently metabolized in vivo to form a compound of the present invention with pharmacological activity. Non limiting examples of prodrugs include esters, carbonates, semi esters, phosphates, nitroesters, sulfates, sulfoxides, amides, amino formates, nitrogen-containing compounds, phosphoramides, glycosides, ethers, acetals, and ketals.

Effective dose "refers to the amount of a drug or pharmaceutical preparation that will trigger biological or medical reactions in tissues, systems, animals, or humans, such as those being studied by researchers or physicians. In addition, the term 'therapeutic effective dose' refers to any amount that improves the treatment, cure, prevention, or relief of diseases, disorders, or side effects, or reduces the rate of disease or disorder development, compared to the corresponding subjects who did not receive that dose. The term also includes quantities that effectively enhance normal physiological functions.

The term 'treatment' used in this article refers to the alleviation of at least one symptom of a disease, disorder, or illness. The term includes administering and/or applying one or more compounds described herein to a subject to provide management or treatment of a condition. The 'treatment' used for the purpose of this disclosure may, but does not necessarily, provide a cure; But rather, 'treatment' can be a form of disease management. When the compounds described in this article are used to treat harmful proliferating cells (including cancer), "treatment" includes partial or complete destruction of the harmful proliferating cells, but has minimal impact on normal cells. The required processing mechanism for rapidly proliferating pathogenic cells (including cancer cells) at the cellular level is apoptosis.

The term "prevention" as used herein includes the initiation of co-prevention or mitigation of the development of a clinically significant disease or the initiation of a preclinically significant disease phase in an individual at risk. This includes prophylactic treatment of individuals at risk of disease development.

The term 'subject' or 'patient' includes organisms capable of suffering from conditions or conditions associated with reduced or insufficient programmed cell death (apoptosis), or organisms that can benefit from the administration of compounds of the present invention in other ways, such as humans and non-human animals. Preferred humans include human patients who suffer from or have a tendency to suffer from diseases or related conditions as described in this article. The term 'non-human animals' includes vertebrates such as mammals such as non-human primates, sheep, cows, dogs, cats, and rodents such as mice, as well as non-mammals such as chickens, amphibians, reptiles, etc.

As used herein, GI₅₀ refers to the drug concentration required to inhibit 50% cell growth, i.e., the drug concentration at which the growth of 50% of cells (such as cancer cells) is inhibited or controlled.

As used herein, IC₅₀ refers to the amount, concentration, or dose of a specific test compound that achieves 50% inhibition of the maximum effect in an analysis measuring the effect.

As used herein, EC₅₀ refers to the dose/concentration that produces 50% of the maximal response in a given assay.

### PARP7 Inhibitor of the Invention

This invention relates to a PARP7 inhibitor, which is a compound of formula (I) or a pharmaceutically acceptable salt, solvate, ester, acid, metabolite or prodrug thereof. 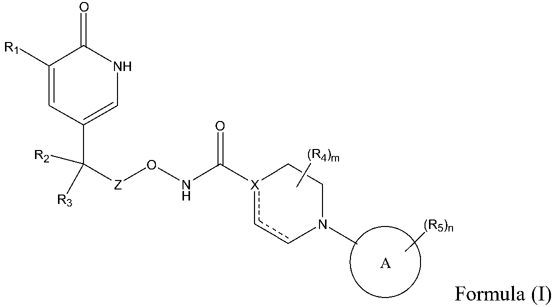 Wherein,
R₁ is selected from halogens, C1-6 haloalkyl groups, and C2-6 alkylyl groups;
R₂ is selected from hydrogen and deuterium;
R₃ is selected from hydrogen, C1-6 alkyl groups, C1-6 hydroxyalkyl groups, and C1-6 haloalkyl groups; or,
R₂ and R₃ together with adjacent carbon atoms form C3-6 cycloalkyl groups;
Z is selected from direct bonds or methylene groups;
X is selected from carbon and nitrogen, indicates a double bond or a single bond, provided that when X is nitrogen, the bond connected to X is a single bond, and when X is carbon, the bond connected to X is a double bond or a single bond, and at most one double bond is connected to any atom;
m is 0, 1, or 2, and when m is 1, R₄ is selected from C1-6 alkyl groups; when m is 2, the two R₄ are located on different ring atoms, and the two R₄ are connected to form an alkylene group containing 1-3 carbon atoms;
Ring A is a 5-6 member monocyclic heteroaryl or a 9-10 member bicyclic heteroaryl having 1-3 nitrogen atoms;
n is 0, 1, or 2, and each R₅ is independently selected from nitrile, halogen, C3-6 cycloalkyl, C1-6 alkyl, C2-6 alkenyl, C2-6 alkynyl, C1-6 haloalkyl, C2-6 alkylcarbonylamino, C1-4 alkylsulfinamide, C1-4 alkylsulfonamide, and phenyl optionally substituted with one or two halogens.
In another embodiment, ring A is selected from pyrimidinyl, pyridinyl, pyrrolopyridinyl, imidazopyridinyl, and quinazolinyl; ring A is more preferably selected from pyrimidin-2-yl, pyridin-2-yl, pyrrolo[2,3-c]pyridin-7-yl, imidazo[4,5-c]pyridin-4-yl, and quinazolin-2-yl; ring A is most preferably pyrimidin-2-yl or imidazo[4,5-c]pyridin-4-yl.
In a preferred aspect of the invention, Z is a direct bond.
In a preferred embodiment, n is 1.

In another preferred embodiment, R₅ is selected from halogen, C3-6 cycloalkyl, C1-3 haloalkyl, and phenyl optionally substituted with one halogen; and, more preferably, R₅ is selected from trifluoromethyl or cyclopropyl.

In yet another preferred embodiment, the substitution position of R₅ on the A ring is para to the nitrogen-containing six-membered heterocyclic group which is connected to the A ring.

In another embodiment, X is N, and - - - are all single bonds.

In this embodiment, preferably, m is 0, 1, or 2, more preferably 0 or 1. When m is 1, R₄ is a C1-3 alkyl group, more preferably methyl; optionally or preferably, R₄ is located ortho to the X atom of the nitrogen-containing six-membered heterocyclic group. When m is 2, the two R₄ group on different ring atoms together form a bridging bond selected from methylene or ethylene; optionally or preferably, the two R₄ groups are respectively located ortho to the X atom of the nitrogen-containing six-membered heterocyclic group or respectively located meta to the X atom of the nitrogen-containing six-membered heterocyclic group.

In one aspect, the present invention relates to a PARP7 inhibitor, which is a compound of formula (Ia), (Ib), or (Ic) or a pharmaceutically acceptable salt, solvate, ester, acid, metabolite, or prodrug thereof,

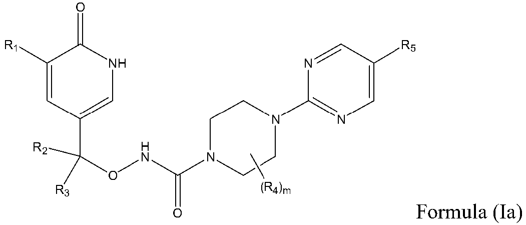

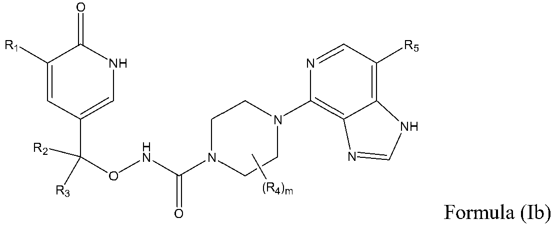

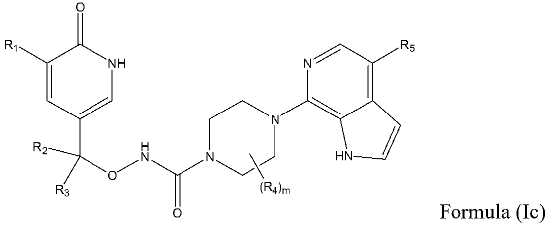

Wherein, R₁, R₂, R₃, R₄, R₅, and m are as defined above.

More preferred compounds of formulas (Ia) and (Ib), most preferred compound of formula (Ia).

In other embodiments, X is C or CH, and one of the two - is a double bond and the other is a single bond; more preferably, X is C, and one of the two - - - bonds to the X atom is a double bond and the other is a single bond.

In this embodiment, m is preferably 0 or 1, more preferably 0. When m is 1, R₄ is C1-3 alkyl, more preferably methyl.

In another aspect, the present invention relates to a PARP7 inhibitor, which is a compound of formula (Id), (Ie), (If), (Ig), or (Ih) or a pharmaceutically acceptable salt, solvate, ester, acid, metabolite, or prodrug thereof,

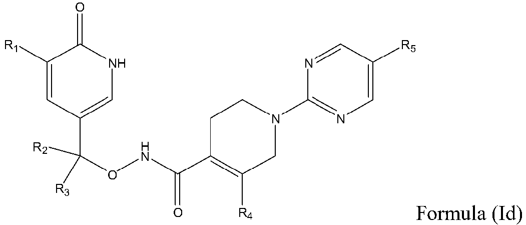

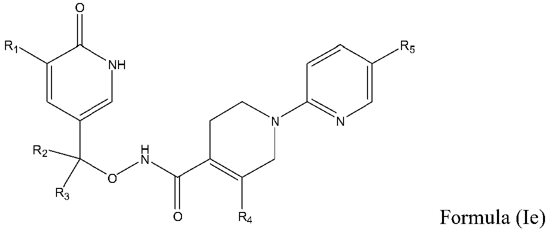

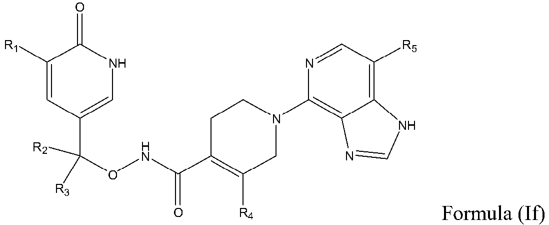

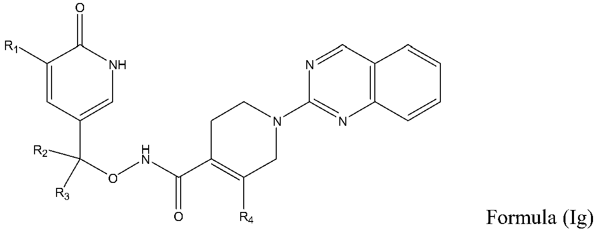

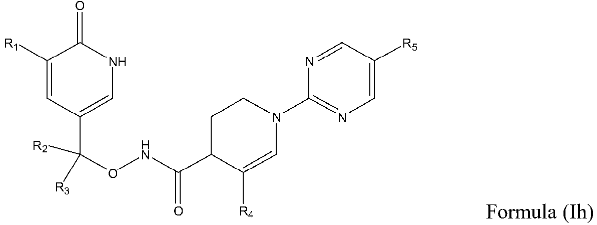

Wherein, R₁, R₂, R₃, R₄, and R₅ are as defined above.

More preferred compounds of formulas (Id) and (If), most preferred compound of formula (Id).

In another preferred embodiment, R₂ is hydrogen.

In yet another preferred embodiment, R₃ is selected from hydrogen, C1-3 alkyl, and C1-3 hydroxyalkyl; R₃ is more preferably methyl or ethyl.

In other preferred embodiments, R₁ is C1-3 haloalkyl, more preferably trifluoromethyl.

More preferably, the compound of formula (I) of this invention is selected from:

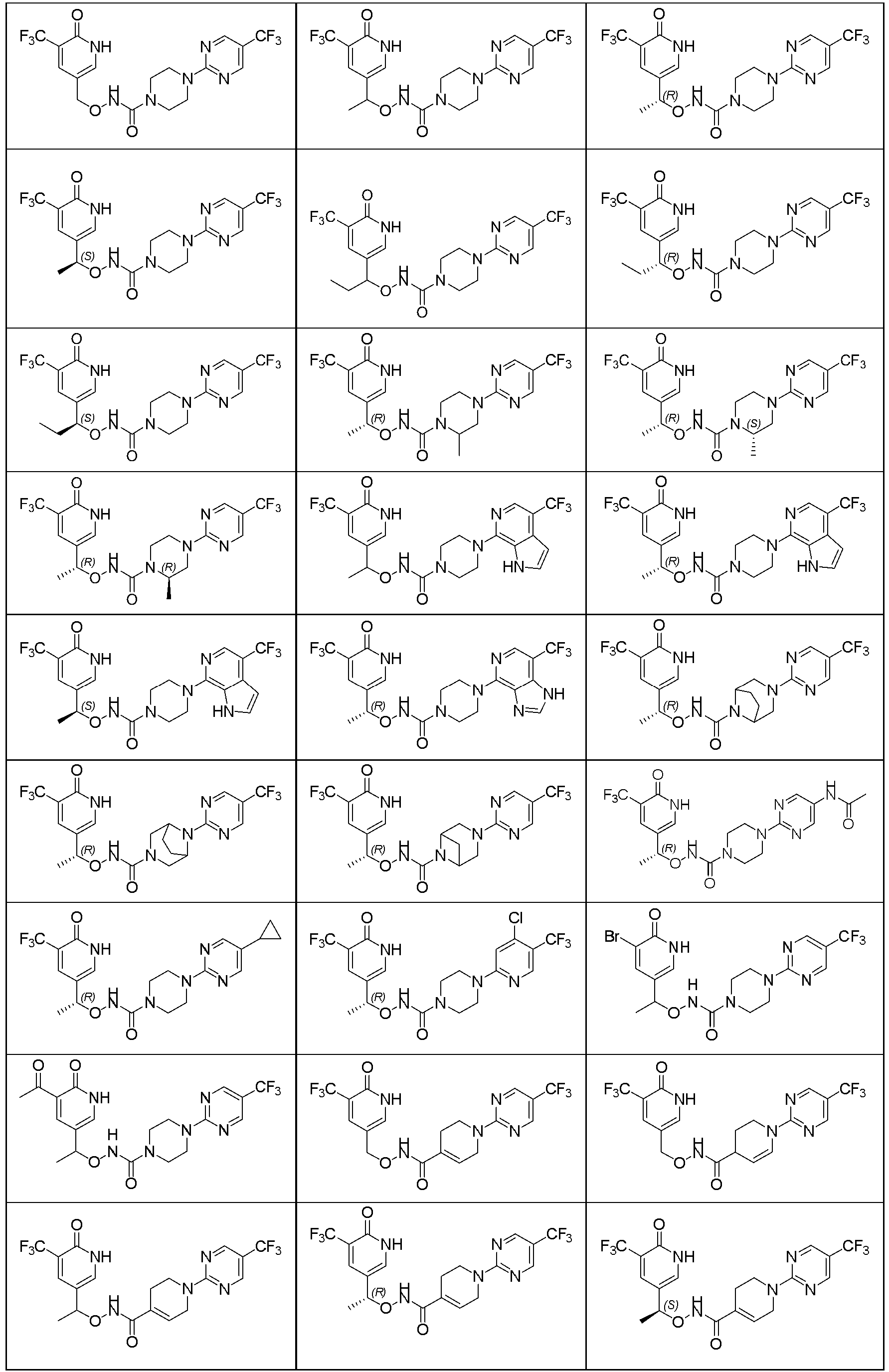

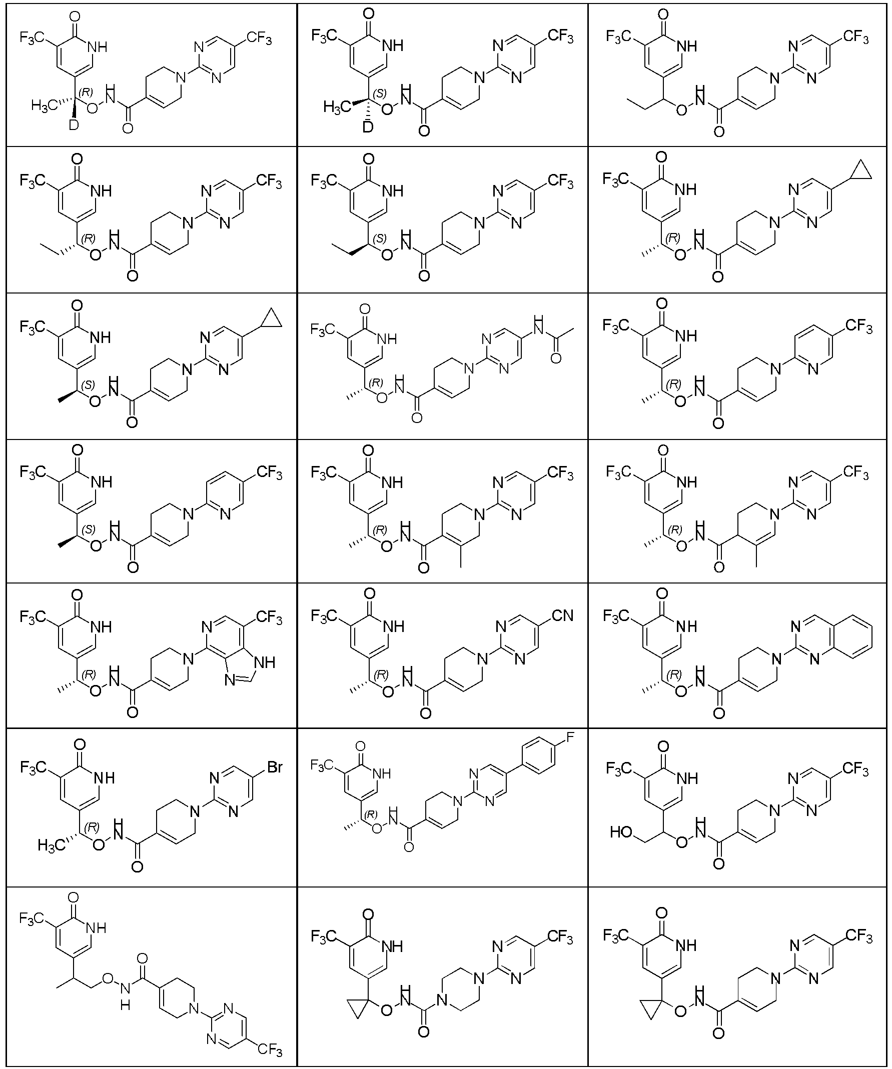

This document describes a novel PARP7 inhibitor. Pharmaceutically acceptable salts, solvates, esters, acids, metabolites, and prodrugs of this compound are also described herein.

The compounds of the present invention may exist in free form, such as free base or free acid or zwitterionic form, or may exist in salt form. The salt can be any salt, organic or inorganic addition salt, especially any physiologically acceptable organic or inorganic addition salt commonly used in pharmacy.

The preferred salt for the purpose of the present invention is the physiologically acceptable salt of the compound of the present invention. However, it also includes salts that are not suitable for pharmaceutical applications but can be used for isolating or purifying compounds of the present invention.

The term "pharmaceutically acceptable salt" refers to a relatively non-toxic inorganic or organic acid addition salt of the compound of the present invention, see, for example, SM Berge et al., "Pharmaceutical Salts, J. Pharm. Sci. 1977, 66, 1-19*".*

Pharmaceutically acceptable salts of the compounds of this invention include acid addition salts of inorganic acids, carboxylic acids, and sulfonic acids, such as salts of the following acids: hydrochloric acid, hydrobromic acid, hydroiodic acid, sulfuric acid, pyrosulfuric acid, phosphoric acid, nitric acid; or salts of organic acids, such as formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)-benzoic acid, camphoric acid, cinnamic acid, cyclopentanepropionic acid, digluconic acid, 3-hydroxy-2-naphthoic acid, gallic acid, nicotinic acid, pyruvic acid, etc. Salts of pectinic acid, persulfate, 3-phenylpropionic acid, picric acid, tert-valeric acid, 2-hydroxyethanesulfonic acid, itaconic acid, aminosulfonic acid, trifluoromethanesulfonic acid, dodecyl sulfate, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheponic acid, glycerophosphate, aspartic acid, sulfosalicylic acid, hemisulfonic acid, or thiocyanate.

Pharmaceutically acceptable salts of the compounds of the present invention also include salts of commonly used bases, such as and preferably alkali metal salts (e.g., sodium and potassium salts), alkaline earth metal salts (e.g., calcium and magnesium salts), and ammonium salts derived from ammonia or organic amines having 1 to 16 carbon atoms, said organic amines being, for example and preferably, ethylamine, diethylamine, triethylamine, ethyl diisopropylamine, monoethanolamine, diethanolamine, triethanolamine, dicyclohexylamine, dimethylaminoethanol, procaine, dibenzylamine, N-methylmorpholine, arginine, lysine, ethylenediamine, N-methylpiperidine, N-methylglucosamine, dimethylglucosamine, ethylglucosamine, 1,6-hexanediamine, glucosamine, sarcosine, serine, tris(hydroxymethyl)aminomethane, aminopropylene glycol, Sovak base, and 1-amino-2,3,4-butanetriol.

The present invention includes all possible salts of the compounds of the present invention, which may be a single salt or any mixture of the salts in any proportion.

The present invention includes all possible deuterated compounds of the present invention, where deuterium atoms can be arbitrarily substituted on carbon or nitrogen atoms.

Solvation is a term used for the purpose of the present invention for those forms of compounds of the present invention that form complexes with solvent molecules through coordination in solid or liquid state. Hydrate is a special solvate form that coordinates with water. Hydrates are preferred as solvates within the scope of the present invention.

In addition, the present invention also includes prodrugs of the compounds of the present invention. The term 'prodrug' includes compounds that can be biologically active or inert but are converted (e.g. through metabolism or hydrolysis) into compounds of the present invention during their retention time in the body.

In addition, the present invention includes all possible crystalline forms or polymorphs of the compounds of the present invention, either as individual polymorphs or as a mixture of more than one polymorph in any proportion.

In this specification, in some cases for convenience, the structural formula of a compound represents a specific isomer, but the present invention includes all isomers, such as geometric isomers, optical isomers based on asymmetric carbon atoms, stereoisomers, tautomers, etc.

The chiral compounds involved in the present invention can have any configuration or a mixture of racemates. When the compound used according to the present invention contains more than one chiral center, it may exist in a non-enantiomeric form. The non enantiomeric isomeric compounds can be separated by methods known to those skilled in the art (such as chromatography or crystallization), while individual enantiomers can be separated as described above. The present invention includes the application of various diastereomeric compounds and mixtures thereof used in accordance with the present invention. The compounds used in the present invention may exist in different tautomeric forms or in different geometric isomeric forms, and the present invention includes the application of various tautomers and/or geometric isomers of the compounds used in accordance with the present invention and their mixtures. The compounds used in the present invention can exist in the form of zwitterionic ions. The present invention includes the use of various zwitterionic forms and mixtures thereof of compounds used in accordance with the present invention.

Screening and characterization of pharmaceutically acceptable salts, polymorphs and/or solvates can be accomplished using a variety of techniques, including but not limited to thermal analysis, X-ray diffraction, spectroscopy, microscopy, and elemental analysis. Various spectroscopic techniques used include, but are not limited to, Raman, FTIR, UVIS, and NMR (liquid and solid states). Various microscopy techniques include, but are not limited to, IR microscopy and Raman microscopy.

Therefore, this invention includes all possible salts, polymorphs, metabolites, hydrates, deuterated compounds, solvates, or prodrugs (e.g., esters) of the compounds of this invention, as individual salts, polymorphs, metabolites, hydrates, deuterated compounds, solvates, or prodrugs, or as mixtures of more than one salt, polymorph, metabolite, hydrate, deuterated compound, solvate, or prodrug in any proportion.

### Pharmaceutical Uses of the Invention

The compounds of the present invention, or pharmaceutically acceptable salts, solvates, esters, acids, metabolites, or prodrugs thereof, are capable of selectively inhibiting the enzymatic activity of PARP7, and are therefore capable of being used to treat or prevent diseases, disorders, or conditions regulated by or affected by PARP7 activity, or involving PARP7 activity or overexpression.

In a preferred aspect, diseases, disorders, or conditions regulated by or affected by PARP7 activity, or involving PARP7 activity or overexpression, are hyperproliferative diseases, particularly cancers, including but not limited to squamous cell carcinoma of the lung, adenocarcinoma of the lung, large cell lung cancer, small cell lung cancer, head and neck squamous cell carcinoma, breast cancer, pancreatic cancer, pancreatic ductal adenocarcinoma, colorectal cancer, melanoma, ovarian cancer, esophageal squamous cell carcinoma, gastric cancer, liver cancer, oral cancer, urothelial carcinoma, prostate cancer, bladder cancer, renal cell carcinoma, gastrointestinal stromal tumor, cervical cancer, endometrial cancer, rhabdomyosarcoma, mesothelioma, neuroendocrine tumor, fibrosarcoma, brain cancer, and malignant glioma, etc.

In another preferred aspect, diseases, disorders or conditions regulated by or affected by PARP7 activity, or involving PARP7 activity or overexpression, are autoimmune or inflammatory diseases, including but not limited to ulcerative colitis, Crohn's disease, multiple sclerosis, autoimmune liver disease, type I diabetes, bronchial asthma, systemic lupus erythematosus, rheumatoid arthritis, ankylosing spondylitis, juvenile idiopathic arthritis, psoriasis, polymyositis and dermatomyositis, etc.

The compounds of the present invention can exert systemic and/or local effects. For this purpose, it can be administered in an appropriate manner, such as through oral route, parenteral route, pulmonary route, nasal route, sublingual route, lingual route, oral route, rectal route, dermal route, transdermal route, conjunctival route or ear route, or in the form of implants or stents.

Preferably, in the embodiments of the present invention, the drug comprising the compound of the present invention can be administered to the patient by at least one of injection, oral administration, inhalation, rectal administration, and transdermal administration.

Regardless of the chosen route of administration, the PARP7 inhibitors and/or pharmaceutical compositions of the present invention are formulated into pharmaceutically acceptable dosage forms using conventional methods known to those skilled in the art.

When treating a patient according to the present invention, the amount of a given drug depends on many factors, such as the specific dosing regimen, the type and severity of the disease or condition, and the unique characteristics of the patient or host requiring treatment (e.g., weight). However, depending on the specific surrounding circumstances, including, for example, the specific drug used, the route of administration, the condition being treated, and the patient or host being treated, the dosage can be conventionally determined by methods known in the art. Typically, for adult treatment, the dosage is typically in the range of 0.02-5000 mg/day, for example, about 1-1500 mg/day. This required dosage can be conveniently expressed as a single dose, or concurrent (or over a short period of time) or fractions at appropriate intervals, such as two, three, four or more doses per day. Those skilled in the art will understand that although the above dosage ranges are given, the specific effective amount can be appropriately adjusted according to the patient's condition and in conjunction with the physician's diagnosis.

The actual dosage level and time course of the compound of the present invention can be varied to obtain the amount of active ingredient that effectively achieves the desired therapeutic response for a specific patient and is non-toxic to the patient.

### Drug combination

Another aspect of the present invention relates to a pharmaceutical composition comprising a compound of the present invention, or a pharmaceutically acceptable salt, solvate, ester, acid, metabolite or prodrug thereof, and a pharmaceutically acceptable diluent, carrier or excipient, and optionally one or more other therapeutic agents.

The compounds of this invention can be administered as a single agent or in combination with one or more other therapeutic agents, wherein said combination does not cause unacceptable side effects. The pharmaceutical composition comprises a single-dose formulation for administering the compound of the present invention and one or more other therapeutic agents, as well as a single-dose formulation for administering the compound of the present invention and various other therapeutic agents. For example, the compound of formula (I) may be administered to a patient together with other therapeutic agents in the form of a single oral dose composition, such as tablets or capsules, or each agent may be administered in a single-dose formulation.

When using individual dosage formulations, the compounds of the present invention and one or more other therapeutic agents can be administered at substantially the same time (e.g. simultaneously) or at staggered times (e.g. sequentially).

Specifically, the compounds of the present invention can be used in fixed combinations or individual combinations with other anti-tumor agents, such as alkylating agents, anti-metabolic agents, plant derived anti-tumor agents, hormone therapy agents, topoisomerase inhibitors, camptothecin derivatives, kinase inhibitors, targeted drugs, antibodies, interferons and/or biological response modifiers, anti-angiogenic compounds, and other anti-tumor drugs.

The compounds of the present invention can also be used in conjunction with radiation therapy and/or surgical intervention for cancer treatment.

### Preparation of compounds

The compounds of the present invention can be synthesized using standard synthesis techniques known to those skilled in the art or by combining known methods in the art with the methods described in this article. In addition, the solvents, temperatures, and other reaction conditions provided in this article can be changed according to the technology in this field. As further guidance, the following synthesis methods can also be utilized.

The reactions can be used in sequence to provide the compounds described in this article; Or they can be used to synthesize fragments, which are subsequently added using the methods described in this article and/or methods known in the art.

The starting materials used for synthesizing the compounds described in this article can be synthesized or obtained from commercial sources. The compounds described in this article and other related compounds with different substituents can be synthesized using techniques and raw materials known to those skilled in the art. The general method for preparing the compounds disclosed in this article can come from reactions known in the art, and these reactions can be modified by appropriate reagents and conditions deemed suitable by those skilled in the art to introduce various parts of the molecules provided in this article.

If necessary, the reaction products can be separated and purified using conventional techniques, including but not limited to filtration, distillation, crystallization, chromatography, and other methods. These products can be characterized using conventional methods, including physical constants and spectral data.

Non-limiting examples of synthetic schemes for preparing compounds of formula (I) are described below.

### Embodiment

The following specific non limiting embodiments will be interpreted as merely illustrative and not limiting the present invention in any way. Although further detailed description is not necessary, it can be trusted that those skilled in the art can fully utilize this disclosure based on the description in this article.

The structure of the compound is determined by nuclear magnetic resonance (NMR) or mass spectrometry (MS). The solvents used for NMR determination were deuterated dimethyl sulfoxide (DMSO-d⁶), deuterated chloroform (CDCl₃, or deuterated methanol (CD₃OD).

Unless otherwise specified in the embodiments, the solution refers to an aqueous solution.

Unless otherwise specified in the embodiments, the reaction temperature is room temperature, such as 20 °C~30 °C.

The reaction process in the examples was monitored using thin-layer chromatography (TLC) and LCMS. The developing solvent used in the reaction, the eluent used for column chromatography to purify the compounds, and the developing solvent system for TLC included: A: dichloromethane/methanol system, B: n-hexane/ethyl acetate system, and C: petroleum ether/ethyl acetate system. The volume ratio of the solvent was adjusted according to the polarity of the compound, and small amounts of basic or acidic reagents such as triethylamine and acetic acid could also be added for adjustment.

### Common abbreviation meanings:

- CAN: Acetonitrile;
- CDI: N, N'-carbonyldiimidazole;
- DCM: Dichloromethane;
- DIAD: Diisopropyl azodicarboxylate;
- DIPEA: N, N-diisopropylethylamine;
- DMAP: 4-dimethylaminopyridine;
- DMF: N, N-dimethylformamide;
- DMT-MM: 4- (4,6-dimethoxytriazin-2-yl) -4-methylmorpholine hydrochloride;
- DPPA: Diphenyl azide phosphate;
- HATU: 2-(7-azabenzotriazole)-N, N, N, N'-tetramethylurea hexafluorophosphate;
- LDA: Lithium diisopropylamide;
- mCPBA: M-chloroperoxybenzoic acid;
- NBS: N-bromosuccinimide;
- NMM: N-methylmorpholine;
- NMP: N-methylpyrrolidone;
- PyBrOP: Tripyrrylphosphonium bromide hexafluorophosphate
- SEMCl: 2- (trimethylsilyl) ethoxymethyl chloride;
- SFC: Supercritical fluid chromatography;
- TBAF: Tetra-n-butylammonium fluoride;
- TBBSCl: Tert butyldimethylchlorosilane;
- TEA: Triethylamine;
- TFA: Trifluoroacetic acid;
- THF: Tetrahydrofuran;
- TMSI: Trimethyliodosilane;

(R)-RUCY XylBINAP

Chloro{(R)-(+)-2,2'-bis[bis(3,5-dimethylyl)phosphino]-1,1'-binaphthyl}[(2R)-(-)-1-(4-methoxyphen yl)-1-(4-methoxyphenyl-KC)-3-methyl-1,2-butanediamine]ruthenium(II).

### Example 1: Synthesis of Intermediates

### 1.1 Synthesis of Intermediate A

### 5-((aminooxy)methyl)-3-(trifluoromethyl)pyridin-2(1H)-one

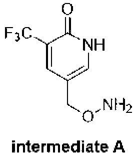

### Step A: Preparation of (6-methoxy-5-(trifluoromethyl)pyridin-3-yl)methanol

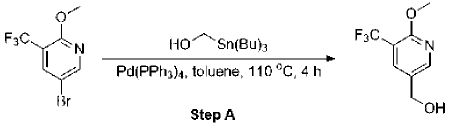

To a solution of 5-bromo-2-methoxy-3-(trifluoromethyl)pyridine (3.05 g, 11.91 mmol, 1.0 eq.) in toluene (80 mL), was added tetrakis(triphenylphosphine)palladium (277 mg, 0.24 mmol, 0.02 eq.) and (tributyltin)methanol (5.74 g, 17.87 mmol, 1.5 eq.) at r. t.. The reaction mixture was reacted at 110 °C for 4 h. The reaction mixture was cooled to room temperature and quenched with water (200 mL), and extracted with EtOAc (3 × 150 mL). The organic phases were combined, washed with saturated brine (500 mL), dried over anhydrous sodium sulfate, and concentrated. The crude product was purified by column chromatography (petroleum ether/EtOAc = 1/4; V/V) to give (6-methoxy-5-(trifluoromethyl)pyridin-3-yl)methanol (620 mg, white solid), yield: 25.1%.

LCMS: *(ESI)* [M+H]⁺ = 208.0.

¹H NMR: (400 MHz, CDCl₃) *δ* 8.30 - 8.25 (m, 1H), 7.90 (d, *J =* 1.6 Hz, 1H), 4.68 (d, *J =* 3.2 Hz, 2H), 4.03 (s, 3H), 1.97 - 1.89 (m, 1H).

### Step B: Preparation of 2-((6-methoxy-5-(trifluoromethyl)pyridin-3-yl)methoxy)isoindoline-1,3-dione

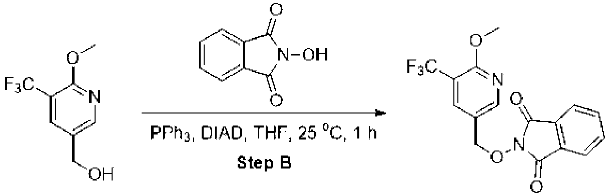

(6-methoxy-5-(trifluoromethyl)pyridin-3-yl)methanol (570 mg, 2.75 mmol, 1.0 eq.) was dissolved in THF (18 mL), N-hydroxyphthalimide (449 mg, 2.75 mmol, 1.0 eq.) and triphenylphosphine (795 mg, 3.03 mmol, 1.1 eq.) were added sequentially. Finally, diisopropyl azodicarbonate (613 mg, 3.03 mmol, 1.1 eq.) was added dropwise. The reaction mixture was reacted at 25 °C for 1 hour. The reaction mixture was quenched with water (100 mL) and extracted with EtOAc (3 × 80 mL). The organic phases were combined, washed with saturated brine (300 mL), dried over anhydrous sodium sulfate, and concentrated. The crude product was purified by column chromatography (petroleum ether / EtOAc = 2 / 1; V / V) to give 2-((6-methoxy-5-(trifluoromethyl)pyridin-3-yl)methoxy)isoindoline-1,3-dione (950 mg, white solid), yield: 98.0%.

LCMS: *(ESI)* [M+H]⁺ = 353.2.

¹H NMR: (400 MHz, CDCl₃) *δ* 8.33 (d, *J =* 2.0 Hz, 1H), 8.06 (d, *J =* 2.0 Hz, 1H), 7.80 - 7.74 (m, 2H), 7.72 - 7.66 (m, 2H), 5.11 (s, 2H), 3.98 (s, 3H).

### Step C: Preparation of 2-((6-oxo-5-(trifluoromethyl)-1,6-dihydropyridin-3-yl)methoxy)isoindoline-1,3-dione

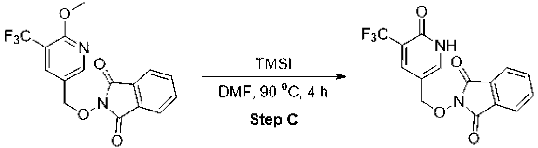

2-((6-methoxy-5-(trifluoromethyl)pyridin-3-yl)methoxy)isoindoline-1,3-dione (1.05 g, 2.98 mmol, 1.0 eq.) was dissolved in N, N-dimethylformamide (40 mL), and TMSI (543 mg, 3.87 mmol, 1.3 e q.) was added dropwise. The reaction mixture was reacted at 90 °C for 4 hours. The reaction m ixture was cooled to room temperature and quenched with water (200 mL), then extracted with EtOAc (3 × 150 mL). The combined organic phases were washed with saturated brine (500 mL), dried over anhydrous sodium sulfate, and concentrated. The crude product was purified by colu mn chromatography (petroleum ether / EtOAc = 1 / 2; V / V) to give 2-((6-oxo-5-(trifluorometh yl)-1,6-dihydropyridin-3-yl)methoxy)isoindoline-1,3-dione (420 mg, white solid), yield: 41.7%.

LCMS: *(ESI)* [M+H]⁺= 339.0.

¹H NMR: (400 MHz, DMSO-*d*₆) *δ* 12.37 (s, 1H), 8.16 - 8.10 (m, 1H), 7.92 - 7.88 (m, 1H), 7.87 - 7.83 (m, 4H), 4.99 (s, 2H).

### Step D: Preparation of 5-((aminooxy)methyl)-3-(trifluoromethyl)pyridin-2(1H)-one (Intermediate A)

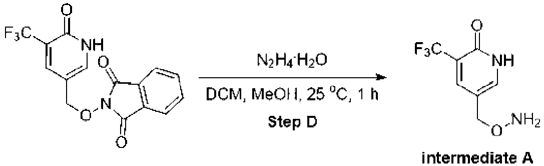

2-((6-oxo-5-(trifluoromethyl)-1,6-dihydropyridin-3-yl)methoxy)isoindoline-1,3-dione (310 mg, 0.92 mmol, 1.0 eq.) was dissolved in a mixture of dichloromethane (10 mL) and methanol (5 mL), and hydrazine hydrate (85%, 325 mg, 5.52 mmol, 6.0 eq.) was added. The reaction mixture was reacted at 25 °C for 1 hour. The reaction mixture was concentrated, and dichloromethane (20 mL) was added to the residue and the mixture was filtered. The filtrate was concentrated again. The crude product was purified by column chromatography (dichloromethane/methanol = 10/1; V/V) to obtain intermediate A, 5-((aminooxy)methyl)-3-(trifluoromethyl)pyridin-2(1H)-one (150 mg, white solid), yield: 78.6%.

LCMS: *(ESI)* [M+H]⁺ = 209.0.

¹H NMR: (400 MHz, DMSO-*d*₆) *δ* 12.22 (br s, 1H), 7.94 - 7.86 (m, 1H), 7.67 (d, *J =* 1.6 Hz, 1H), 6.00 (s, 2H), 4.34 (s, 2H).

### 1.2 Synthesis of Intermediate B

### 5-(1-(aminooxy)ethyl)-3-(trifluoromethyl)pyridin-2(1H)-one

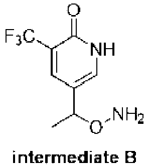

### Step A: Preparation of 1-(6-methoxy-5-(trifluoromethyl)pyridin-3-yl)acetone

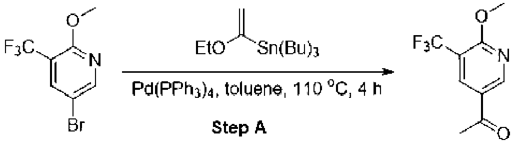

5-bromo-2-methoxy-3-(trifluoromethyl)pyridine (5.00 g, 19.53 mmol, 1.0 eq.) was dissolved in toluene (80 mL), and tetra(triphenylphosphine)palladium (451 mg, 0.39 mmol, 0.02 eq.) and tributyl(1-ethoxyethylene)tin (10.58 g, 29.30 mmol, 1.5 eq.) were added. Under nitrogen protection, the reaction mixture was reacted at 110 °C for 4 hours. The reaction mixture was cooled to room temperature and quenched with water (200 mL), and extracted with EtOAc (3 × 150 mL). The organic phases were combined, washed with saturated brine (500 mL), dried over anhydrous sodium sulfate, and concentrated. The crude product was purified by column chromatography (petroleum ether/EtOAc = 4/1; V/V) to give 1-(6-methoxy-5-(trifluoromethyl)pyridin-3-yl)acetone (4.75 g, white solid), yield: 100%.

LCMS: *(ESI)* [M+H]⁺ = 220.0.

¹H NMR: (400 MHz, DMSO-*d*₆) *δ* 9.06 (d, *J =* 2.0 Hz, 1H), 8.45 - 8.39 (m, 1H), 4.09 (s, 3H), 2.63 (s, 3H).

### Step B: Preparation of 1-(6-methoxy-5-(trifluoromethyl)pyridin-3-yl)ethanol

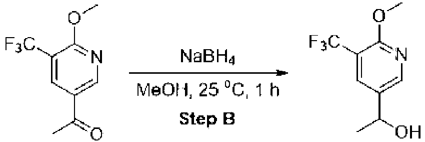

1-(6-methoxy-5-(trifluoromethyl)pyridin-3-yl)acetone (3.70 g, 16.88 mmol, 1.0 eq.) was dissolved in methanol (50 mL), and sodium borohydride (1.53 g, 40.51 mmol, 2.4 eq.) was added. The reaction mixture was reacted at 25 °C for 1 hour. The reaction mixture was quenched with water (200 mL) and adjusted to pH 7 with saturated sodium bicarbonate. Extraction was performed with dichloromethane (3 × 150 mL). The combined organic phases were dried over anhydrous sodium sulfate and concentrated. The crude product was purified by column chromatography (petroleum ether / EtOAc = 1/1; V/V) to give 1-(6-methoxy-5-(trifluoromethyl)pyridin-3-yl)ethanol (1.86 g, colorless oil), yield: 49.8%.

LCMS: *(ESI)* [M+H]⁺ = 222.0.

¹H NMR: (400 MHz, DMSO-*d*₆) *δ* 8.39 (d, *J =* 2.0 Hz, 1H), 8.02 (d, *J =* 2.4 Hz, 1H), 5.39 (d, *J =* 4.4 Hz, 1H), 4.90 - 4.75 (m, 1H), 3.97 (s, 3H), 1.36 (d, *J =* 6.8 Hz, 3H).

### Step C: Preparation of 2-(1-(6-methoxy-5-(trifluoromethyl)pyridin-3-yl)ethoxy)isoindoline-1,3-dione

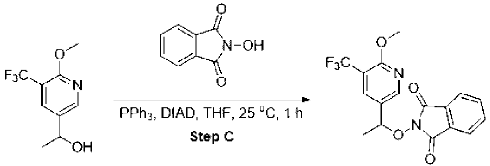

1-(6-methoxy-5-(trifluoromethyl)pyridin-3-yl)ethanol (2.72 g, 12.30 mmol, 1.0 eq.) was dissolved in THF (80 mL), followed by the addition of N-hydroxyphthalimide (2.00 g, 12.30 mmol, 1.0 eq.), triphenylphosphine (3.55 g, 13.53 mmol, 1.1 eq.), and then diisopropyl azodicarbonate (2.74 g, 13.53 mmol, 1.1 eq.) was added dropwise. The reaction mixture was stirred at 25 °C for 1 h. The reaction mixture was quenched with water (200 mL) and extracted with EtOAc (3 × 150 mL). The organic phases were combined, washed with saturated brine (500 mL), dried over anhydrous sodium sulfate, and concentrated. The crude product was purified by column chromatography (petroleum ether/EtOAc = 2/1;V/V) to give 2-(1-(6-methoxy-5-(trifluoromethyl)pyridin-3-yl)ethoxy)isoindoline-1,3-dione (4.50 g, white solid), yield: 99.9%.

LCMS: *(ESI)* [M+H]⁺= 367.0.

¹H NMR: (400 MHz, DMSO-*d*₆) *δ* 8.53 (d, *J =* 2.0 Hz, 1H), 8.30 (d, *J =* 2.0 Hz, 1H), 7.85 - 7.81 (m, 4H), 5.48 - 5.38 (m, 1H), 3.98 (s, 3H), 1.69 (d, *J =* 6.8 Hz, 3H).

### Step D: Preparation of 2-(1-(6-oxo-5-(trifluoromethyl)-1,6-dihydropyridin-3-yl)ethoxy)isoindoline-1,3-dione

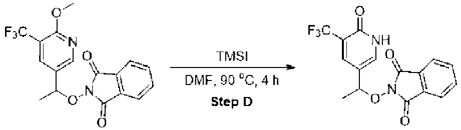

2-(1-(6-methoxy-5-(trifluoromethyl)pyridin-3-yl)ethoxy)isoindoline-1,3-dione (4.30 g, 11.74 mmol, 1. 0 eq.) was dissolved in N,N-dimethylformamide (50 mL), and TMSI (6.10 g, 30.52 mmol, 2.60 eq.) was added. The reaction mixture was reacted at 90 °C for 4 h. The reaction mixture was c ooled to room temperature and quenched with water (200 mL), and extracted with EtOAc (3 × 150 mL). The organic phases were combined, washed with saturated brine (500 mL), dried over anhydrous sodium sulfate, and concentrated. The crude product was purified by column chromato graphy (petroleum ether/EtOAc = 1/2; V/V) to give 2-(1-(6-oxo-5-(trifluoromethyl)-1,6-dihydropyri din-3-yl)ethoxy)isoindoline-1,3-dione (2.86 g, white solid), yield: 69.2%.

LCMS: *(ESI)* [M+H]⁺= 353.0.

¹H NMR: (400 MHz, DMSO-*d₆*) *δ* 12.32 (s, 1H), 8.15 (d, *J =* 2.0 Hz, 1H), 7.85 (s, 4H), 7.80 (d, *J =* 2.8 Hz, 1H), 5.17 (q, *J =* 6.8 Hz, 1H), 1.61 (d, *J =* 6.8 Hz, 3H).

### Step E: Preparation of 5-(1-(aminooxy)ethyl)-3-(trifluoromethyl)pyridin-2(1H)-one (intermediate B)

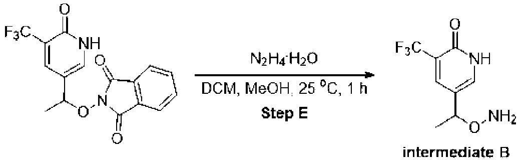

2-(1-(6-oxo-5-(trifluoromethyl)-1,6-dihydropyridin-3-yl)ethoxy)isoindoline-1,3-dione (2.80 g, 7.95 mmol, 1.0 eq.) was dissolved in a mixture of dichloromethane (40 mL) and methanol (20 mL), and hydrazine hydrate (85%, 2.81 g, 47.70 mmol, 6.0 eq.) was added. The reaction mixture was reacted at 25 °C for 1 hour. The reaction mixture was concentrated, and dichloromethane (20 mL) was added to the residue. The mixture was filtered to remove insoluble solid, and the filtrate was concentrated. The crude product was purified by column chromatography (dichloromethane/methanol = 10/1; V/V) to obtain intermediate B, 5-(1-(aminooxy)ethyl)-3-(trifluoromethyl)pyridine-2(1H)-one (940 mg, white solid), yield: 53.2%.

LCMS: (*ESI)* [M+H]⁺ = 223.2.

### 1.3 Synthesis of intermediate C

### O-(1-(6-methoxy-5-(trifluoromethyl)pyridin-3-yl)propyl)hydroxylamine

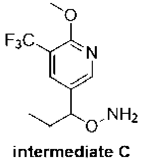

### Step A: Preparation of 6-methoxy-5-(trifluoromethyl)nicotinaldehyde

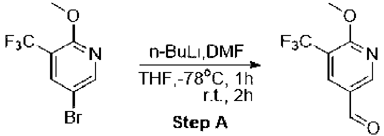

5-bromo-2-methoxy-3-(trifluoromethyl)pyridine (500 mg, 1.97 mmol, 1.0 eq.) was added dropwise to the solution of n-BuLi (2.5 M, 1.97 mL, 4.92 mmol, 2.5 eq.) in THF (5 mL) at -78 °C in a dry ice/ethanol bath under nitrogen protection. The reaction mixture was reacted at -78 °C for 1 hour. N, N-dimethylformamide (286 mg, 3.92 mmol, 2.0 eq.) was added to the reaction mixture. The reaction mixture was continued to react at room temperature for 2 hours. The reaction mixt ure was quenched with saturated ammonium chloride aqueous solution (35 mL) and extracted wit h EtOAc (2 × 20 mL). The organic phase was washed successively with saturated brine (30 mL), dried over anhydrous sodium sulfate, and concentrated by filtration. The crude product obtained was purified by column chromatography (petroleum ether/EtOAc = 85/15; V/V) to give 6-methox y-5-(trifluoromethyl)nicotinaldehyde (170 mg, yellow oil), yield: 42.1%.

LCMS: *(ESI)* [M+H]⁺ = 222.2.

¹H NMR (400 MHz, DMSO-*d*₆) *δ* 10.07 (s, 1H), 9.03 (s, 1H), 8.48 (s, 1H), 4.13 (s, 3H).

### Step B: Preparation of 1-(6-methoxy-5-(trifluoromethyl)pyridin-3-yl)propane-1-ol

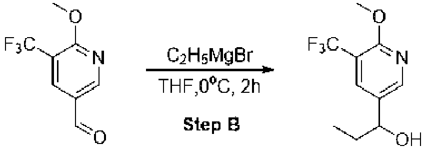

To a solution of 6-methoxy-5-(trifluoromethyl)nicotinaldehyde (170 mg, 0.83 mmol, 1.0 eq.) in THF (3 mL) was added ethyl magnesium bromide (1.25 mL, 1.25 mmol, 1.5 eq.) at 0 °C under N₂. The reaction mixture was reacted at 0 °C for 2 hours. The reaction mixture was quenched with 30 mL of saturated ammonium chloride aqueous solution and extracted with EtOAc (2 ×20 mL). The organic phases were combined, dried over anhydrous sodium sulfate, and concentrated. The crude product was purified by column chromatography (petroleum ether/EtOAc = 85/15; V/V) to give 1-(6-methoxy-5-(trifluoromethyl)pyridin-3-yl)propane-1-ol (80 mg, colorless oil), yield: 41.0%.

LCMS: *(ESI)* [M+H]⁺= 236.2.

¹H NMR (400 MHz, DMSO-*d*₆) *δ* 8.51 - 8.37 (m, 1H), 8.06 (d, *J =* 2.2 Hz, 1H), 5.43 (d, *J =* 4.7 Hz, 1H), 4.74 - 4.48 (m, 1H), 4.05 (s, 3H), 1.79 - 1.63 (m, 2H), 0.90 (t, *J =* 7.4 Hz, 3H).

### Step C: Preparation of 2-(1-(6-methoxy-5-(trifluoromethyl)pyridin-3-yl)propoxy)isedihydroindole-1,3-dione

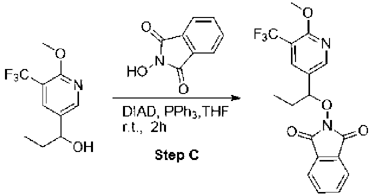

To a solution of 1-(6-methoxy-5-(trifluoromethyl)pyridin-3-yl)propane-1-ol (80 mg, 0.341 mmol, 1. 0 eq.) in THF (2 mL) was added sequentially N-hydroxyphthalimide (59 mg, 0.36 mmol, 1.05 e q.) and triphenylphosphine (100 mg, 0.38 mmol, 1.1 eq.). And finally, diisopropyl azodicarbonate (76 mg, 0.38 mmol, 1.1 eq.) was added dropwise to the reaction mixture at 0 °C. The reaction mixture was reacted at room temperature for 2 hours. The reaction mixture was quenched with water (20 mL) and extracted with EtOAc (2 × 15 mL). The organic phases were combined, drie d over anhydrous sodium sulfate, and concentrated. The crude product was purified by column c hromatography (petroleum ether/EtOAc = 9/1; V/V) to give 2-(1-(6-methoxy-5-(trifluoromethyl)pyri din-3-yl)propoxy)isodihydroindole-1,3-dione (30 mg, colorless oil), yield: 23.2%.

LCMS: (*ESI)* [M+H]⁺ = 381.2.

### Step D: Preparation of O-(1-(6-methoxy-5-(trifluoromethyl)pyridin-3-yl)propyl)hydroxylamine (inter mediate C)

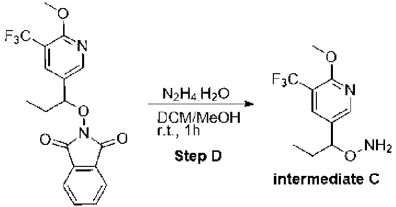

2-(1-(6-methoxy-5-(trifluoromethyl)pyridin-3-yl)propoxy)isodihydroindole-1,3-dione (250 mg, 0.66 mmol, 1.0 eq.) was dissolved in a mixture of dichloromethane (2 mL) and methanol (1 mL), and hydrazine hydrate (85%, 200 mg, 3.95 mmol, 6.0 eq.) was added. The reaction mixture was reacted at room temperature for 1 hour. The reaction mixture was concentrated and the residue was mixed with dichloromethane (10 mL), stirred and filtered. The filtrate was concentrated again. The crude product was purified by column chromatography (petroleum ether/EtOAc = 4/1; V/V) to give intermediate C, O-(1-(6-methoxy-5-(trifluoromethyl)pyridin-3-yl)propyl)hydroxylamine (80 mg, white solid), yield: 48.7%.

LCMS: (*ESI)* [M+H]⁺ = 251.2.

¹H NMR (400 MHz, DMSO-*d*₆) *δ* 8.35 (d, *J =* 2.0 Hz, 1H), 7.93 (d, *J =* 2.1 Hz, 1H), 5.96 (s, 2H), 4.43 (t, *J* = 6.7 Hz, 1H), 3.99 (s, 3H), 1.88 - 1.70 (m, 1H), 1.67 - 1.54 (m, 1H), 0.81 (t, *J =* 7.4 Hz, 3H).

### 1.4 Synthesis of intermediate D

### (R)-5-(1-(aminooxy)ethyl)-3-(trifluoromethyl)pyridin-2(1H)-one

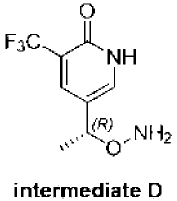

### Step A: Preparation of 1-(6-methoxy- 5-(trifluoromethyl)pyridin-3-yl)acetone

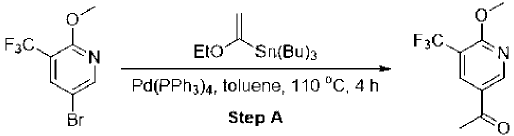

5-bromo-2-methoxy-3-(trifluoromethyl)pyridine (10.00 g, 39.06 mmol, 1.0 eq.) was dissolved in toluene (150 mL), and tetrakis(triphenylphosphine)palladium (1.80 g, 1.56 mmol, 0.04 eq.) and tributyl(1-ethoxyethylene)tin (21.16 g, 58.59 mmol, 1.5 eq.) were added. The reaction mixture was reacted at 110 °C for 4 h under nitrogen protection. The reaction mixture was cooled to room temperature and quenched with water (400 mL), then extracted with EtOAc (3 × 350 mL). The combined organic phases were washed with saturated brine (1.2 L), dried over anhydrous sodium sulfate, and concentrated. The crude product was purified by column chromatography (petroleum ether / EtOAc = 4 / 1; V / V) to give 1-(6-methoxy-5-(trifluoromethyl)pyridin-3-yl)acetone (6.28 g, white solid), yield: 73.4%.

LCMS: *(ESI)* [M+H]⁺= 220.0.

¹H NMR: (400 MHz, DMSO-*d*₆) *δ* 9.07 (d, *J =* 1.6 Hz, 1H), 8.43 (d, *J =* 1.6 Hz, 1H), 4.09 (s, 3H), 2.63 (s, 3H).

### Step B: Preparation of (S)-1-(6-methoxy-5-(trifluoromethyl)pyridin-3-yl)ethanol

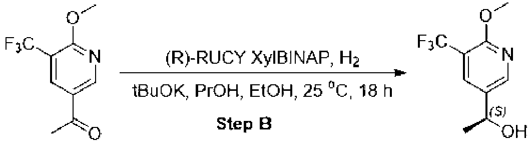

To a solution of 1-(6-methoxy-5-(trifluoromethyl)pyridin-3-yl)acetone (8.00 g, 36.36 mmol, 1.0 eq.) was dissolved in a mixture of ethanol (75 mL) and propanol (63 mL) was added potassium tert-butoxide (204 mg, 1.82 mmol, 0.05 eq.) and (R)-RUCY XylBINAP (213 mg, 0.18 mmol, 0.005 eq.). The reaction mixture was reacted at 25 °C for 18 hours under a hydrogen atmosphere. The reaction mixture was concentrated, and the residue was diluted with water (300 mL) and extracted with EtOAc (2 × 280 mL). The organic phases were combined, dried over anhydrous sodium sulfate, and concentrated. The crude product was purified by column chromatography (petroleum ether/EtOAc = 1/1; V/V) to obtain (S)-1-(6-methoxy-5-(trifluoromethyl)pyridin-3-yl)ethanol (6.85 g, colorless oil), yield: 85.2%.

LCMS: *(ESI)* [M+H]⁺ = 222.3.

¹H NMR: (400 MHz, DMSO-*d*₆) *δ* 8.33 (d, *J =* 2.4 Hz, 1H), 7.96 (d, *J =* 2.4 Hz, 1H), 5.34 (d, *J =* 4.4 Hz, 1H), 4.81 - 4.72 (m, 1H), 3.91 (s, 3H), 1.30 (d, *J =* 6.4 Hz, 3H).

### Step C: Preparation of (R)-2-(1-(6-methoxy-5-(trifluoromethyl)pyridin-3-yl)ethoxy)isoindoline-1,3-dione

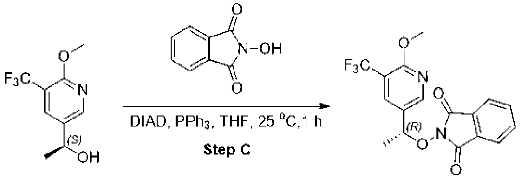

(S)-1-(6-methoxy-5-(trifluoromethyl)pyridin-3-yl)ethanol (11.00 g, 52.09 mmol, 1.0 eq.) was dissolved in THF (200 mL), followed by the addition of N-hydroxyphthalimide (8.50 g, 52.09 mmol, 1.0 eq.), triphenylphosphine (28.69 g, 109.39 mmol, 2.1 eq.), and then diisopropyl azodicarbonate (21.07 g, 104.18 mmol, 2.0 eq.). The reaction mixture was stirred at 25 °C for 1 h. The reaction mixture was quenched with water (400 mL) and extracted with EtOAc (3 × 350 mL). The organic phases were combined, washed with saturated brine (1.2 L), dried over anhydrous sodium sulfate, and concentrated. The crude product was purified by column chromatography (petroleum ether/EtOAc = 2/1; V/V) to give (R)-2-(1-(6-methoxy-5-(trifluoromethyl)pyridin-3-yl)ethoxy)isoindoline-1,3-dione (7.20 g, white solid), yield: 37.7%.

LCMS: *(ESI)* [M+H]⁺ = 367.0.

¹H NMR: (400 MHz, CDCl₃) *δ* 8.35 (d, *J =* 2.0 Hz, 1H), 8.17 (d, *J =* 2.0 Hz, 1H), 7.85 - 7.78 (m, 2H), 7.77 - 7.72 (m, 2H), 5.46 (q, *J =* 6.4 Hz, 1H), 4.04 (s, 3H), 1.75 (d, *J =* 6.8 Hz, 3H).

### Step D: Preparation of (R)-2-(1-(6-oxo-5-(trifluoromethyl)-1,6-dihydropyridin-3-yl)ethoxy)isoindoline-1,3-dione

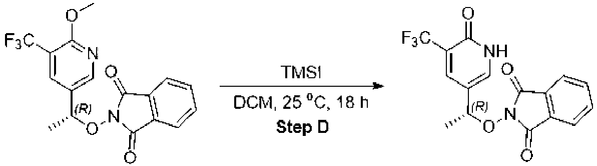

(R)-2-(1-(6-methoxy-5-(trifluoromethyl)pyridin-3-yl)ethoxy)isoindoline-1,3-dione (7.20 g, 19.66 mmol, 1.0 eq.) was dissolved in dichloromethane (100 mL), and TMSI (10.23 g, 51.12 mmol, 2.60 eq.) was added dropwise. The reaction mixture was reacted at 25 °C for 18 h. The reaction mixture was quenched with water (200 mL) and extracted with dichloromethane (3 × 150 mL). The co mbined organic phases were washed with saturated brine (500 mL), dried over anhydrous sodium sulfate, and concentrated. The crude product was purified by column chromatography (petroleum ether/EtOAc = 1/2; V/V) to give (R)-2-(1-(6-oxo-5-(trifluoromethyl)-1,6-dihydropyridin-3-yl)ethoxy) isoindoline-1,3-dione (5.60 g, white solid), yield: 80.8%.

LCMS: (*ESI)* [M+H]⁺ = 353.3.

¹H NMR: (400 MHz, DMSO-*d*₆) *δ* 12.37 (s, 1H), 8.21 (d, *J =* 2.0 Hz, 1H), 7.90 (s, 4H), 7.86 (d, *J =* 2.4 Hz, 1H), 5.23 (q, *J* = 6.4 Hz, 1H), 1.67 (d, *J =* 6.8 Hz, 3H).

### Step E: Preparation of (R)-5-(1-(aminooxy)ethyl)-3-(trifluoromethyl)pyridin-2(1H)-one (intermediate D)

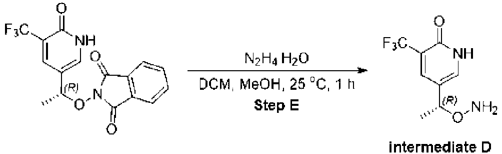

To a solution of (R)-2-(1-(6-oxo-5-(trifluoromethyl)-1,6-dihydropyridin-3-yl)ethoxy)isoindoline-1,3-di one (5.60 g, 15.89 mmol, 1.0 eq.) in a mixture of dichloromethane (100 mL) and methanol (50 mL) was added hydrazine hydrate (85%, 5.61 g, 95.34 mmol, 6.0 eq.). The reaction mixture was reacted at 25 °C for 1 hour. The reaction mixture was concentrated, and dichloromethane (20 m L) was added to the residue. The mixture was filtered to remove insoluble solid, and the filtrate was concentrated. The crude product was purified by column chromatography (dichloromethane/ methanol = 10/1; V/V) to obtain intermediate D,(R)-5-(1-(aminooxy)ethyl)-3-(trifluoromethyl)pyridi n-2(1H)-one (3.30 g, white solid), yield: 93.5%.

LCMS: (*ESI)* [M+H]⁺ = 223.2.

¹H NMR: (400 MHz, DMSO-*d*₆) *δ* 12.14 (s, 1H), 7.85 (d, *J =* 2.0 Hz, 1H), 7.59 (d, *J =* 2.4 Hz, 1H), 5.88 (s, 2H), 4.40 (q, *J =* 6.4 Hz, 1H), 1.28 (d, *J =* 6.4 Hz, 3H).

### Synthesis of 1.5 intermediate E

### 2-(piperazin-1-yl)-5-(trifluoromethyl)pyrimidine hydrochloride

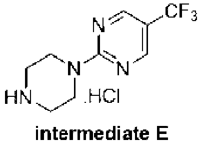

### Step A: Preparation of tert-butyl 4-(5-(trifluoromethyl)pyrimidin-2-yl)piperazine-1-carboxylate

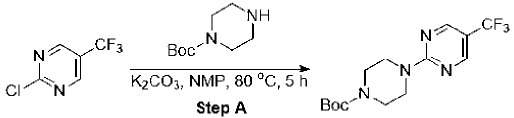

To a solution of 2-chloro-5-(trifluoromethyl)pyrimidine (4.00 g, 21.92 mmol, 1.05 g) in N-methyl pyrrolidone (40 mL) was added 4-(5-(trifluoromethyl)pyrimidin-2-yl)piperazine-1-carboxylic acid ter t-butyl ester (3.89 g, 20.87 mmol, 1.0 eq.) and potassium carbonate (3.46 g, 25.04 mmol, 1.2 e q.). The reaction mixture was heated to 80 °C and reacted for 5 hours. The reaction mixture wa s cooled to room temperature, and water (150 mL) was added. A precipitate was formed. The s olid was collected by filtration and the solid was dissolved in EtOAc (150 mL), dried over anhy drous sodium sulfate, and concentrated to give 6.4 g of 4-(5-(trifluoromethyl)pyrimidin-2-yl)pipera zine-1-carboxylic acid tert-butyl ester as a white solid, yield: 87.9%.

LCMS: (*ESI)* [M+H-56]⁺ = 276.8.

¹H NMR: (400 MHz, DMSO-*d*₆) *δ* 8.74 (s, 2H), 3.89 - 3.79 (m, 4H), 3.49 - 3.42 (m, 4H), 1.44 (s, 9H).

### Step B: Preparation of 2-(piperazin-1-yl)-5-(trifluoromethyl)pyrimidine hydrochloride (intermediate E)

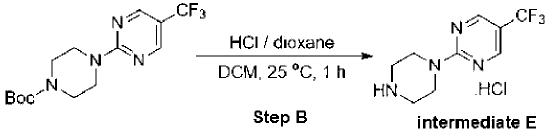

To a solution of 4-(5-(trifluoromethyl)pyrimidin-2-yl)piperazin-1-carboxylic acid tert-butyl ester (3.00 g, 9.03 mmol, 1.0 eq.) in dichloromethane (10 mL) was added dioxane hydrochloride solution (4 M, 10 mL). The reaction mixture was reacted at 25 °C for 1 hour. The reaction mixture was concentrated to give intermediate E (2-(piperazin-1-yl)-5-(trifluoromethyl)pyrimidine (2.90 g, yield: 100%) as a white solid.

LCMS: *(ESI)* [M+H]⁺ = 233.0.

### Synthesis of Intermediate F (1.6)

### 1-(5-(trifluoromethyl)pyrimidine-2-yl)-1,2,3,6-tetrahydropyridine-4-carboxylic acid

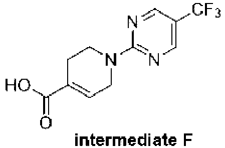

### Step A: Preparation of 1,2,3,6-tetrahydropyridine-4-carboxylate hydrochloride

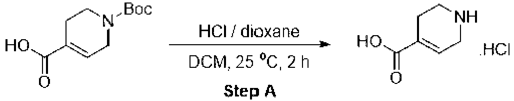

To a solution of 1-(tert-Butoxycarbonyl)-1,2,3,6-tetrahydropyridine-4-carboxylic acid (4.00 g, 17.60 mmol, 1.0 eq.) in dichloromethane (50 mL) was added dioxane hydrochloride solution (4 M, 20 mL). The reaction mixture was reacted at 25 °C for 2 h. The reaction mixture was concentrated to give 1,2,3,6-tetrahydropyridine-4-carboxylic acid hydrochloride (2.87 g, white solid), yield: 99.6%.

LCMS: *(ESI)* [M+H]⁺ = 128.4.

### Step B: Preparation of 1-(5-(trifluoromethyl)pyrimidin-2-yl)-1,2,3,6-tetrahydropyridine-4-carboxylic acid (intermediate F)

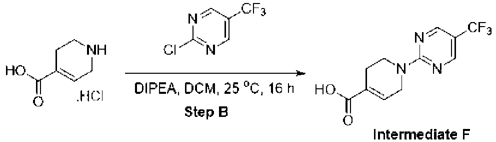

1,2,3,6-tetrahydropyridine-4-carboxylic acid hydrochloride (2.87 g, 17.54 mmol, 1.0 eq.) was dissolved in dichloromethane (50 mL), followed by the addition of DIPEA (10.20 g, 78.94 mmol, 4.5 eq.) and 2-chloro-5-trifluoromethylpyrimidinium (3.20 g, 17.54 mmol, 1.0 eq.). The reaction mixture was reacted at 25 °C for 16 h. The reaction mixture was diluted with water (80 mL), adjusted to pH 5 with dilute hydrochloric acid (1 M), and extracted with dichloromethane (2 × 100 mL). The organic phases were combined, dried over anhydrous sodium sulfate, and concentrated to give intermediate F, 1-(5-(trifluoromethyl)pyrimidin-2-yl)-1,2,3,6-tetrahydropyridine-4-carboxylic acid (4.20 g, yellow solid), yield: 87.7%.

LCMS: *(ESI)* [M+H]⁺= 274.2.

¹H NMR: (400 MHz, DMSO-*d*₆) δ 12.47 (s, 1H), 8.74 (s, 2H), 6.98 - 6.84 (m, 1H), 4.47 - 4.38 (m, 2H), 3.96 (t, *J* = 5.6 Hz, 2H), 2.42 - 2.32 (m, 2H).

### 1.7 Synthesis of intermediate G

### 7-Bromo-1-((2-(trimethylsilyl)ethoxy)methyl)-1H-imidazo[4.5-clpyridine 5-oxide

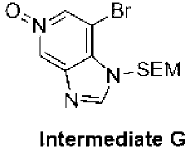

### Step A: Preparation of 7-bromo-1H-imidazo[4.5-c]pyridine

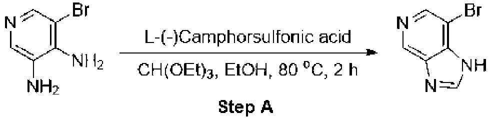

5-Bromo-3,4-diaminopyridine (8.80 g, 46.80 mmol, 1.0 eq.) was dissolved in ethanol (70 mL). triethyl orthoformate (110 mL) and L-camphor sulfonic acid (109 mg, 0.47 mmol, 0.01 eq.) were added. The reaction mixture was reacted at 80 °C for 2 h. The reaction mixture was concentrated to give 7-Bromo-1H-imidazo[4,5-c]pyridine (9.40 g, crude product, white solid).

LCMS: *(ESI)* [M+H]⁺ = 198.0.

¹H NMR: (400 MHz, DMSO-*d₆*) *δ* 8.92 (s, 1H), 8.55 (s, 1H), 8.46 (s, 1H).

### Step B: Preparation of 7-bromo-1-((2-(trimethylsilyl)ethoxy)methyl)-1H-imidazo[4,5-c]pyridine

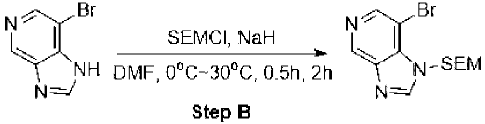

To a solution of 7-bromo-1H-imidazo[4.5-c]pyridine (9.90 g, 50.00 mmol, 1.0 eq.) in N, N-dimet hylformamide (150 mL) was added NaH (60%, 3.00 g, 75.00 mmol, 1.0 eq.) at 0 °C under nitr ogen protection. The reaction mixture was reacted at 0 °C for 0.5 h. 2-(trimethylsilyl)ethoxymeth yl chloride (8.34 g, 50.00 mmol, 1.0 eq.) was added dropwise to the reaction mixture. The reacti on mixture was further reacted at 30 °C for 2 h. The reaction mixture was cooled to 0 °C in a n ice bath, quenched with water (500 mL), and extracted with EtOAc (3 × 400 mL). The organi c phases were combined, washed with saturated brine (1000 mL), dried over anhydrous sodium s ulfate, and concentrated. The crude product was purified by column chromatography (petroleum e ther/EtOAc = 2/1; V/V) to give 7-bromo-1-((2-(trimethylsilyl)ethoxy)methyl)-1H-imidazo[4,5-c]pyri dine (9.60 g, colorless oil), yield: 58.5%.

LCMS: *(ESI)* [M+H]⁺ = 328.0.

¹H NMR: (400 MHz, CDCl₃) *δ* 9.02 (s, 1H), 8.53 (s, 1H), 8.06 (s, 1H), 5.83 (s, 2H), 3.62 - 3.56 (m, 2H), 0.95 - 0.88 (m, 2H), 0.05 (s, 6H).

### Step C: Preparation of 7-bromo-1-((2-(trimethylsilyl)ethoxy)methyl)-1H-imidazo[4,5-c]pyridine 5-oxide (intermediate G)

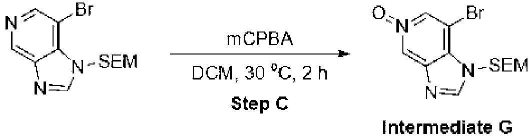

7-bromo-1-((2-(trimethylsilyl)ethoxy)methyl)-1H-imidazo[4,5-c]pyridine (3.28 g, 10.00 mmol, 1.0 e q.) was dissolved in dichloromethane (50 mL), and m-chloroperoxybenzoic acid (85%, 4.06 g, 20. 00 mmol, 2.0 eq.) was added. The reaction mixture was reacted at 30 °C for 2 h. The reaction mixture was quenched with an aqueous sodium thiosulfate solution (0.5 M, 80 mL) and extracted with dichloromethane (2 × 100 mL). The organic phases were combined, washed with saturated brine (200 mL), dried over anhydrous sodium sulfate, and concentrated. The crude product was purified by column chromatography (dichloromethane/methanol = 10/1; V/V) to give intermediate G,7-bromo-1-((2-(trimethylsilyl)ethoxy)methyl)-1H-imidazo[4,5-c]pyridine 5-oxide (3.40 g, yellow so lid), yield: 98.8%.

LCMS: *(ESI)* [M+H]⁺ = 344.2.

### 1.8 Synthesis of intermediate H

### 2-(1-(5-bromo-6-methoxypyridin-3-yl)ethoxy)isodihydroindole-1,3-dione

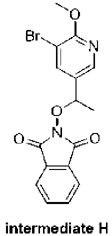

### Step A: Preparation of 5-bromo-6-methoxynicotinic acid

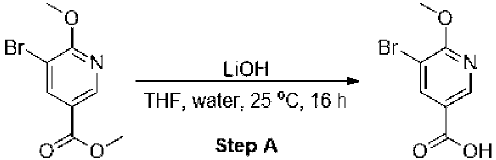

Methyl 5-bromo-6-methoxynicotinate (1.5 g, 6.09 mmol, 1.0 eq.) was dissolved in THF (15 mL), and water (10 mL). lithium hydroxide (292 mg, 12.19 mmol, 2.0 eq.) were added. The reaction mixture was reacted at 25 °C for 16 h. The reaction mixture was diluted with water (10 mL) and the pH was adjusted to 5 with dilute hydrochloric acid. Extraction was performed with EtOAc (3 × 15 mL). The combined organic phases were dried over anhydrous sodium sulfate and concentrated to give 5-bromo-6-methoxynicotinic acid (1.3 g, white solid), yield: 91.9%.

LCMS: *(ESI)* [M+H]⁺ = 232.0.

### Step B: Preparation of 5-bromo-N,6-dimethoxy-N-methylnicotinamide

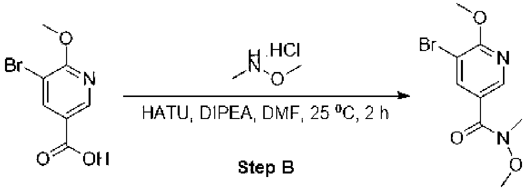

To a solution of 5-bromo-6-methoxynicotinic acid (1.3 g, 5.62 mmol, 1.0 eq.) in N,N-dimethylformamide (15 mL), was added DIPEA (2.2 g, 16.88 mmol, 3.0 eq.), 2-(7-azabenzotriazole)-N,N,N,N'-tetramethylurea hexafluorophosphate (2.5 g, 6.75 mmol, 1.2 eq.), and N,O-dimethylhydroxylamine hydrochloride (818 mg, 8.44 mmol, 1.5 eq.) . The reaction mixture was reacted at 25 °C for 2 h. The reaction mixture was diluted with water (20 mL) and extracted with EtOAc (3 × 30 mL). The organic phases were combined, dried over anhydrous sodium sulfate, and concentrated. The crude product was purified by column chromatography (petroleum ether/EtOAc = 3/1; V/V) to give 5-bromo-N,6-dimethoxy-N-methylnicotinamide (1.5 g, yellow solid), yield: 96.9%.

LCMS: *(ESI)* [M+H]⁺ = 275.0.

¹H NMR: (400 MHz, CDCl₃) *δ* 8.58 (d, *J =* 2.0 Hz, 1H), 8.26 (d, *J =* 2.0 Hz, 1H), 4.07 (s, 3H), 3.59 (s, 3H), 3.38 (s, 3H).

### Step C: Preparation of 1-(5-bromo-6-methoxypyridin-3-yl)ethane-1-one

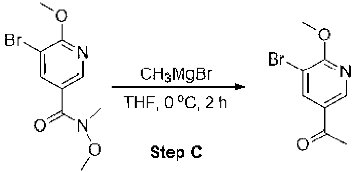

To a solution of 5-bromo-N,6-dimethoxy-N-methylnicotinamide (1.5 g, 5.45 mmol, 1.0 eq.) in THF (20 mL) was added methylmagnesium bromide (3 M, 2.7 mL, 8.18 mmol, 1.5 eq.) at 0 °C under nitrogen. The reaction mixture was reacted at 0 °C for 2 h. The reaction mixture was quenched with saturated ammonium chloride aqueous solution (30 mL), and extracted with EtOAc (2 ×20 mL). The organic phases were combined, dried over anhydrous sodium sulfate, and concentrated. The crude product was purified by column chromatography (petroleum ether/EtOAc = 9/1;V/V) to give 1-(5-bromo-6-methoxypyridin-3-yl)ethane-1-one (1 g, white solid), yield: 80.0%.

LCMS: *(ESI)* [M+H]⁺ = 230.0.

### Step D: Preparation of 1-(5-bromo-6-methoxypyridin-3-yl)ethane-1-ol

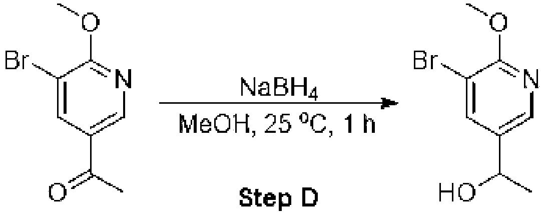

1-(5-bromo-6-methoxypyridin-3-yl)ethane-1-one (1 g, 4.36 mmol, 1.0 eq.) was dissolved in methanol (10 mL), and sodium borohydride (414 mg, 10.91 mmol, 1.5 eq.) was added. The reaction mixture was reacted at 25 °C for 1 h. The reaction mixture was quenched with water (20 mL) and extracted with dichloromethane (3 × 20 mL). The organic phases were combined, dried over anhydrous sodium sulfate, and concentrated. The crude product was purified by column chromatography (petroleum ether/EtOAc = 4/1; V/V) to give 1-(5-bromo-6-methoxypyridin-3-yl)ethane-1-ol (900 mg, colorless oil), yield: 36.5%.

LCMS: *(ESI)* [M+H]⁺ = 232.2.

¹H NMR: (400 MHz, CDCl₃) *δ* 8.00 - 7.96 (m, 1H), 7.84 - 7.79 (m, 1H), 4.81 (q, *J =* 6.5 Hz, 1H), 3.93 (s, 3H), 1.43 (d, *J =* 6.5 Hz, 3H).

### Step E: Preparation of 2-(1-(5-bromo-6-methoxypyridin-3-yl)ethoxy)isodihydroindole-1,3-dione (intermediate H)

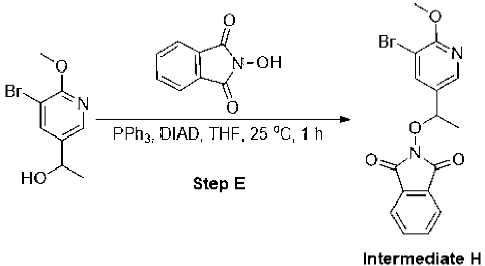

To a solution of 1-(5-bromo-6-methoxypyridin-3-yl)ethane-1-ol (900 mg, 3.87 mmol, 1.0 eq.) in THF (15 mL) was added N-hydroxyphthalimide (698 mg, 4.28 mmol, 1.3 eq.), triphenylphosphine (1.5 g, 5.84 mmol, 1.5 eq.), and diisopropyl azodicarbonate (1.5 g, 7.79 mmol, 2.0 eq.). The reaction mixture was reacted at 25 °C for 1 h. The reaction mixture was quenched with water (20 mL) and extracted with EtOAc (3 × 20 mL). The combined organic phases were washed with saturated brine (30 mL), dried over anhydrous sodium sulfate, and concentrated. The crude product was purified by column chromatography (petroleum ether / EtOAc = 2 / 1; V / V) to give intermediate H,2-(1-(5-bromo-6-methoxypyridin-3-yl)ethoxy)isodihydroindole-1,3-dione (1 g, white solid), yield: 68.4%.

### LCMS: (ESI) [M+H]⁺ = 377.0.Example 2: Synthesis of Compound 2

### N-((6-oxo-5-(trifluoromethyl)-1,6-dihydropyridin-3-yl)methoxy)-4-(5-(trifluoromethyl)pyrimidin-2-yl)pi perazine-1-carboxamide

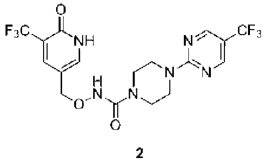

### Step A: Preparation of N-((6-oxo-5-(trifluoromethyl)-1,6-dihydropyridin-3-yl)methoxy)-4-(5-(trifluoro methyl)pyrimidin-2-yl)piperazin-1-carboxamide

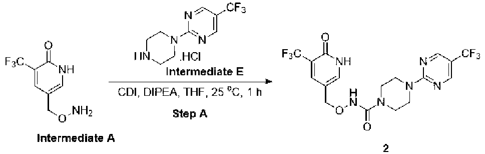

Dissolve 5-((aminooxy)methyl)-3-(trifluoromethyl)pyridin-2(1H)-one (100 mg, 0.48 mmol, 1.0 eq.) and 2-(piperazin-1-yl)-5-(trifluoromethyl)pyrimidin hydrochloride (129 mg, 0.48 mmol, 1.0 eq.) in THF (20 mL), and then DIPEA (248 mg, 1.92 mmol, 4.0 eq.) and N,N'-carbonyldiimidazole (94 mg, 0.58 mmol, 1.2 mL) was added sequentially. The reaction mixture was reacted at 25 °C for 1 hour. The reaction mixture was quenched with water (100 mL) and extracted with EtOAc (3 ×80 mL). The organic phases were combined, washed with saturated brine (300 mL), dried over anhydrous sodium sulfate, and concentrated. The crude product was purified by column chromat ography (dichloromethane/methanol = 10/1; V/V), and further purified by prep-HPLC to give com pound 2, N-((6-oxo-5-(trifluoromethyl)-1,6-dihydropyridin-3-yl)methoxy)-4-(5-(trifluoromethyl)pyrimi din-2-yl)piperazine-1-carboxamide (74.58 mg, white solid), yield: 33.3%.

LCMS: *(ESI)* [M+H]⁺= 467.2.

¹H NMR: (400 MHz, DMSO-*d₆*) *δ* 12.33 (s, 1H), 9.83 (s, 1H), 8.73 (s, 2H), 8.06 - 8.00 (m, 1H), 7.79 - 7.73 (m, 1H), 4.56 (s, 2H), 3.84 - 3.76 (m, 4H), 3.43 - 3.35 (m, 4H).

### Examples 3, 4, 5:

### Synthesis of Compound 3

### N-(1-(6-oxo-5-(trifluoromethyl)-1,6-dihydropyridin-3-yl)ethoxy)-4-(5-(trifluoromethyl)pyrimidin-2-yl)pi perazine-1-carboxamide

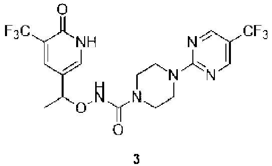

### Synthesis of Compound 4

### (R)-N-(1-(6-oxo-5-(trifluoromethyl)-1,6-dihydrpyridin-3-yl)ethoxy)-4-(5-(trifluoromethyl)pyrimidin-2-1)piperazine-1-carboxamide

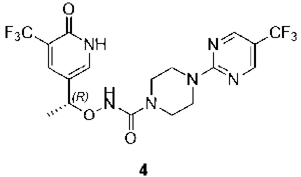

### Synthesis of Compound 5

### (S)-N-(1-(6-oxo-5-(trifluoromethyl)-1,6-dihydropyridin-3-yl)ethoxy)-4-(5-(trifluoromethylpyrimidin-2-y 1)piperazine-1-carboxamide

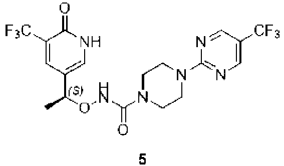

### Step A: Preparation of N-(1-(6-oxo-5-(trifluoromethyl)-1,6-dihydropyridin-3-yl)ethoxy)-4-(5-(trifluoro methyl)pyrimidin-2-yl)piperazin-1-carboxamide

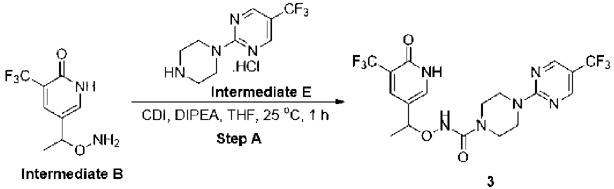

5-(1-(aminooxy)ethyl)-3-(trifluoromethyl)pyridin-2(1H)-one (660 mg, 2.97 mmol, 1.0 eq.) was dissolved in THF (30 mL), and 2-(piperazin-1-yl)-5-(trifluoromethyl)pyrimidin hydrochloride (400 mg, 1.49 mmol, 0.5 eq.), DIPEA (690 mg, 5.34 mmol, 2.0 eq.), N, N'-carbonyldiimidazole (289 mg, 1.78 eq.) were added sequentially. The reaction mixture was reacted at 25 °C for 1 h. The reaction mixture was quenched with water (100 mL) and extracted with EtOAc (3 × 80 mL). The organic phases were combined, washed with saturated brine (300 mL), dried over anhydrous sodium sulfate, and concentrated. The crude product was purified by column chromatography (dichloromethane/methanol = 10/1; V/V) to give a racemic crude product (410 mg, white solid), yield: 57.1%.

150 mg of the crude racemic product (compound 3) was further purified by prep-HPLC (formic acid) to afford N-(1-(6-oxo-5-(trifluoromethyl)-1,6-dihydropyridin-3-yl)ethoxy)-4-(5-(trifluoromethyl)p yrimidin-2-yl)piperazine-1-carboxamide (125 mg, white solid)

LCMS: *(ESI)* [M+H]⁺= 481.0.

¹H NMR: (400 MHz, DMSO-*d*₆) *δ* 12.30 (s, 1H), 9.69 (s, 1H), 8.72 (s, 2H), 8.03 (d, *J =* 2.0 Hz, 1H), 7.68 (d, *J* = 2.0 Hz, 1H), 4.66 (q, *J =* 6.4 Hz, 1H), 3.85 - 3.71 (m, 4H), 3.38 - 3.33 (m, 4H), 1.39 (d, *J =* 6.8 Hz, 3H).

### Step B: Preparation of isomers (R)-N-(1-(6-oxo-5-(trifluoromethyl)-1,6-dihydropyridin-3-yl)ethoxy)-4 -(5-(trifluoromethyl)pyrimidin-2-yl)piperazine-1-carboxamide and (S)-N-(1-(6-oxo-5-(trifluoromethyl)-1,6-dihydropyridin-3-yl)ethoxy)-4-(5-(trifluoromethyl)pyrimidin-2-yl)piperazine-1-carboxamide)

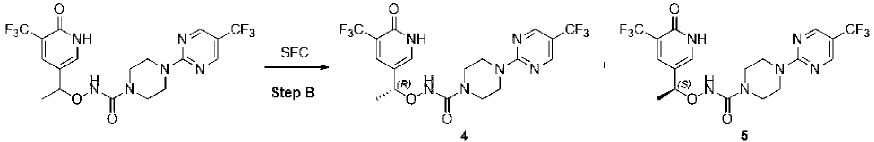

The obtained racemic product N-(1-(6-oxo-5-(trifluoromethyl)-1,6-dihydropyridin-3-yl)ethoxy)-4-(5-(t rifluoromethyl)pyrimidin-2-yl)piperazine-1-carboxamide (95.0 mg, 0.198 mmol) was purified by sup ercritical fluid chromatography (SFC) to give:
Compound 4, (R)-N-(1-(6-oxo-5-(trifluoromethyl)-1,6-dihydropyridin-3-yl)ethoxy)-4-(5-(trifluoromethy l)pyrimidin-2-yl)piperazine-1-carboxamide (36.59 mg, white solid), yield: 38.5%;
LCMS: *(ESI)* [M+H]⁺ = 481.2.

¹H NMR: (400 MHz, DMSO-*d*₆) *δ* 12.35 (s, 1H), 9.77 (s, 1H), 8.79 (s, 2H), 8.10 (d, *J =* 2.0 Hz, 1H), 7.75 (d, *J* = 2.0 Hz, 1H), 4.73 (q, *J* = 6.4 Hz, 1H), 3.93 - 3.79 (m, 4H), 3.48 - 3.41 (m, 4H), 1.46 (d, *J* = 6.4 Hz, 3H).

Compound 5, (S)-N-(1-(6-oxo-5-(trifluoromethyl)-1,6-dihydropyridin-3-yl)ethoxy)-4-(5-(trifluoromethy l)pyrimidin-2-yl)piperazine-1-carboxamide (25.55 mg, white solid), yield: 26.9%.

LCMS: *(ESI)* [M+H]⁺ = 481.2.

¹H NMR: (400 MHz, DMSO-*d₆*) *δ* 12.31 (s, 1H), 9.71 (s, 1H), 8.74 (s, 2H), 8.08 - 8.02 (m, 1H), 7.73 - 7.65 (m, 1H), 4.68 (q, *J =* 6.4 Hz, 1H), 3.86 - 3.74 (m, 4H), 3.43 - 3.35 (m, 4H), 1.41 (d, *J =* 6.4 Hz, 3H).

### Examples 6, 7, 8:

### Synthesis of Compound 6

### N-(1-(6-oxo-5-(trifluoromethyl)-1,6-dihydropyridin-3-ylpropoxy)-4-(5-(trifluoromethyl)pyrimidin-2-yl)p iperazine-1-carboxamide

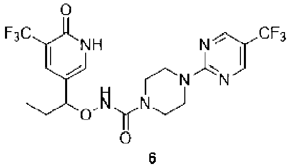

### Synthesis of Compound 7

### (R)-N-(1-(6-oxo-5-(trifluoromethyl)-1,6-dihydropyridin-3-yl)propoxy)-4-(5-(trifluoromethyl)pyrimidin-2 -yl)piperazine-1-carboxamide

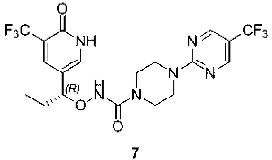

### Synthesis of Compound 8

### (S)-N-(1-(6-oxo-5-(trifluoromethyl)-1,6-dihydropyridin-3-yl)propoxy)-4-(5-(trifluoromethyl)pyrimidin-2-yl)piperazine-1-carboxamide

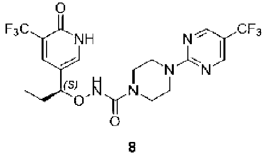

### Step A: Preparation of N-(1-(6-methoxy-5-(trifluoromethyl)pyridin-3-yl)propoxy)-4-(5-(trifluoromethy l)pyrimidin-2-yl)piperazine-1-carboxamide

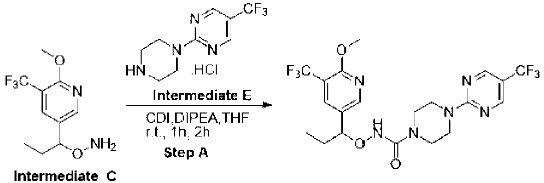

To a solution of O-(1-(6-methoxy-5-(trifluoromethyl)pyridin-3-yl)propyl)hydroxylamine (200 mg, 0.8 mmol, 1.0 eq.) in THF (7 mL) was added N,N'-carbonyldiimidazole (155 mg, 0.96 mmol, 1.2 eq.) and DIPEA (413 mg, 3.2 mmol, 4.0 eq.). The reaction mixture was reacted at 25 °C for 1 hour. Finally, 2-(piperazin-1-yl)-5-(trifluoromethyl)pyrimidine hydrochloride (215 mg, 0.8 mmol, 1.0 eq.) was added to the reaction mixture. The reaction mixture was reacted at room temperature for 2 hours. The reaction mixture was quenched with water (20 mL) and extracted with EtOAc (3 × 20 mL). The organic phases were combined, dried over anhydrous sodium sulfate, and concentrated. The crude product was purified by column chromatography (petroleum ether/EtOAc = 6/4; V/V) to give N-(1-(6-methoxy-5-(trifluoromethyl)pyridin-3-yl)propoxy)-4-(5-(trifluoromethyl)pyrimidin-2-yl)piperazi n-1-carboxamide (330 mg, colorless oil), yield: 73.3%.

LCMS: *(ESI)* [M+H]⁺ = 531.2.

¹H NMR (400 MHz, DMSO-*d*₆) *δ* 9.69 (s, 1H), 8.71 (d, *J =* 0.6 Hz, 2H), 8.41 (d, *J =* 1.9 Hz, 1H), 8.08 (d, *J* = 2.0 Hz, 1H), 4.70 (t, *J =* 6.8 Hz, 1H), 3.98 (s, 3H), 3.80 - 3.68 (m, 4H), 3.32 - 3.28 (m, 4H), 1.93 - 1.84 (m, 1H), 1.78 - 1.66 (m, 1H), 0.88 (t, *J =* 7.4 Hz, 3H).

### Step B: Preparation of N-(1-(6-oxo-5-(trifluoromethyl)-1,6-dihydropyridin-3-yl)propoxy)-4-(5-(trifluor omethyl)pyrimidin-2-yl)piperazin-1-carboxamide

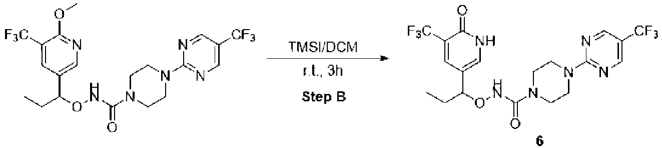

To a solution of N-(1-(6-methoxy-5-(trifluoromethyl)pyridin-3-yl)propoxy)-4-(5-(trifluoromethyl)pyri midin-2-yl)piperazin-1-carboxamide (330 mg, 0.65 mmol, 1.0 eq.) in dichloromethane (6 mL), was added TMSI (0.3 mL). The reaction mixture was reacted at room temperature for 3 hours. The reaction mixture was quenched with water (10 mL) and extracted with EtOAc (3 × 10 mL). The organic phases were combined, washed with saturated brine (2 × 10 mL), dried over anhydrous sodium sulfate, and concentrated. The crude product was purified by prep-HPLC to give racemi c N-(1-(6-oxo-5-(trifluoromethyl)-1,6-dihydropyridin-3-yl)propoxy)-4-(5-(trifluoromethyl)pyrimidin-2-y 1)piperazine-1-carboxamide (35 mg, white solid), yield: 10.9%.

LCMS: *(ESI)* [M+H]⁺ = 495.2.

¹H NMR (400 MHz, DMSO-*d*₆) *δ* 12.30 (s, 1H), 9.64 (s, 1H), 8.72 (d, *J =* 0.7 Hz, 2H), 7.92 (d, *J =* 2.0 Hz, 1H), 7.67 (d, *J =* 2.0 Hz, 1H), 4.44 (t, *J =* 6.9 Hz, 1H), 3.82 - 3.69 (m, 4H), 3.32 - 3.27 (m, 4H), 1.95 - 1.75 (m, 1H), 1.71 - 1.53 (m, 1H), 0.86 (t, *J =* 7.4 Hz, 3H).

### Step C: Preparation of (R)-N-(1-(6-oxo-5-(trifluoromethyl)-1,6-dihydropyridin-3-yl)propoxy)-4-(5-(trif luoromethyl)pyrimidin-2-yl)piperazine-1-carboxamide and (S)-N-(1-(6-oxo-5-(trifluoromethyl)-1,6-dihy dropyridin-3-yl)propoxy)-4-{5-(trifluoromethyl)pyrimidin-2-yl)piperazine-1-carboxamide)

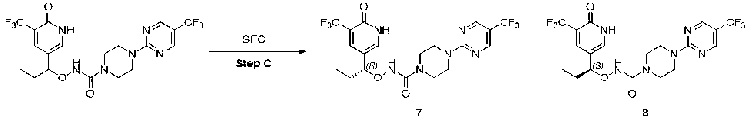

Racemic N-(1-(6-oxo-5-(trifluoromethyl)-1,6-dihydropyridin-3-yl)propoxy)-4-(5-(trifluoromethyl)pyrimi din-2-yl)piperazin-1-carboxamide (35 mg, 0.07 mmol, 1.0 eq.) was purified by supercritical fluid chromatography (SFC) to give:
Isomer 7, (R)-N-(1-(6-oxo-5-(trifluoromethyl)-1,6-dihydropyridin-3-yl)propoxy)-4-(5-(trifluoromethyl)p yrimidin-2-yl)piperazin-1-carboxamide (10.8 mg, white solid), yield: 30.8%;
LCMS: *(ESI)* [M+H]⁺ = 495.2.

¹H NMR (400 MHz, DMSO-*d*₆) *δ* 12.29 (s, 1H), 9.63 (s, 1H), 8.71 (s, 2H), 7.92 (s, 1H), 7.66 (s, 1H), 4.44 (t, 1H), 3.80 - 3.72 (m, 4H), 3.32 - 3.28 (m, 4H), 1.84 (dt, *J =* 14.2, 7.2 Hz, 1H), 1.63 (dt, *J =* 14.1, 7.2 Hz, 1H), 0.86 (t, *J =* 7.3 Hz, 3H).

Isomer 8, (S)-N-(1-(6-oxo-5-(trifluoromethyl)-1,6-dihydropyridin-3-yl)propoxy)-4-(5-(trifluoromethyl)p yrimidin-2-yl)piperazine-1-carboxamide (11.83 mg, white solid), yield: 33.7%.

LCMS: *(ESI)* [M+H]⁺ = 495.2.

¹H NMR (400 MHz, DMSO-*d*₆) *δ* 12.29 (s, 1H), 9.63 (s, 1H), 8.71 (s, 2H), 7.92 (s, 1H), 7.67 (s, 1H), 4.44 (t, *J* = 6.9 Hz, 1H), 3.81 - 3.70 (m, 4H), 3.31 - 3.28 (m, 4H), 1.89 - 1.77 (m, 1H), 1.69 - 1.58 (m, 1H), 0.86 (t, *J* = 7.4 Hz, 3H).

### Examples 9, 10, 11:

### Synthesis of Compound 9

### 2-Methyl-N-((R)-1-(6-oxo-5-(trifluoromethyl)-1,6-dihydropyridin-3-yl)ethoxy)-4-(5-(trifluoromethyl)pyri midin-2-yl)piperazine-1-carboxamide

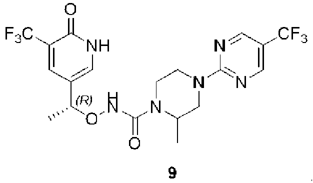

### Synthesis of Compound 10

### (S)-2-Methyl-N-((R)-1-(6-oxo-5-(trifluoromethyl)- 1,6-dihydropyridin-3-yl)ethoxy)-4-(5-(trifluoromethyl) pyrimidin-2-yl)piperazine-1-carboxamide

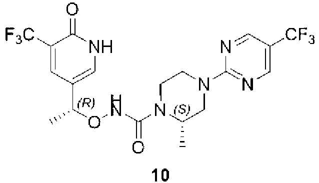

### Synthesis of Compound 11

### (R)-2-Methyl-N-((R)-1-(6-oxo-5-(trifluoromethyl)-1,6-dihydropyridin-3-yl)ethoxy)-4-(5-(trifluoromethyl )pyrimidin-2-yl)piperazine-1-carboxamide

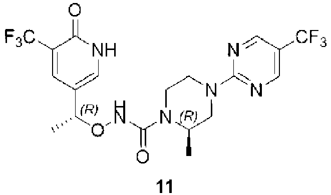

### Step A: Preparation of tert-butyl 2-methyl-4-(5-(trifluoromethyl)pyrimidin-2-yl)piperazine-1-carboxylic acid

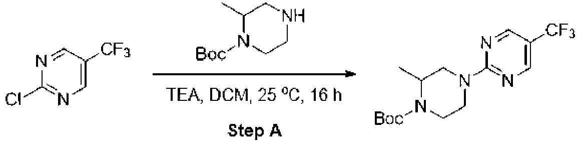

2-chloro-5-(trifluoromethyl)pyrimidinium (1 g, 5.49 mmol, 1.0 eq.) was dissolved in dichlorometha ne (15 mL), followed by the addition of TEA (1.1 g, 10.98 mmol, 4.0 eq.) and tert-butyl 2-meth ylpiperazine-1-carboxylic acid (1.2 g, 6.04 mmol, 1.1 eq.). The reaction mixture was reacted at 2 5 °C for 16 h. The reaction mixture was diluted with water (20 mL) and extracted with EtOAc (2 × 20 mL). The organic phases were combined, washed with saturated brine (20 mL), dried ov er anhydrous sodium sulfate, and concentrated. The crude product was purified by column chrom atography (petroleum ether/EtOAc = 3/1; V/V) to give tert-butyl 2-methyl-4-(5-(trifluoromethyl)pyr imidin-2-yl)piperazine-1-carboxylic acid (1.8 g, white solid), yield: 94.7%.

LCMS: *(ESI)* [M+H-100]⁺ = 247.4.

¹H NMR: (400 MHz, DMSO-*d₆*) *δ* 8.71 (s, 2H), 4.58 - 4.43 (m, 2H), 4.32 - 4.19 (m, 1H), 3.88 - 3.77 (m, 1H), 3.33 - 3.25 (m, 1H), 3.15 - 3.04 (m, 2H), 1.43 (s, 9H), 1.04 (d, *J =* 6.7 Hz, 3H).

### Step B: Preparation of 2-(3-methylpiperazin-1-yl)-5-(trifluoromethyl)pyrimidine hydrochloride

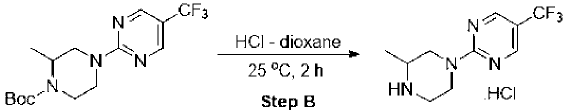

1.8 g (5.20 mmol, 1.0 eq.) of 2-methyl-4-(5-(trifluoromethyl)pyrimidin-2-yl)piperazin-1-carboxylic a cid tert-butyl ester was dissolved in dioxane hydrochloride solution (4 M, 15 mL). The reaction mixture was reacted at 25 °C for 2 h. The reaction mixture was concentrated to give 2-(3-methy lpiperazin-1-yl)-5-(trifluoromethyl)pyrimidine hydrochloride (1.6 g, crude product, white solid). LCMS: *(ESI)* [M+H]⁺ = 274.4.

### Step C: Preparation of 2-methyl-N-((R)-1-(6-oxo-5-(trifluoromethyl)-1,6-dihydropyridin-3-yl)ethoxy)-4-(5-(trifluoromethyl)pyrimidin-2-yl)piperazine-1-carboxamide

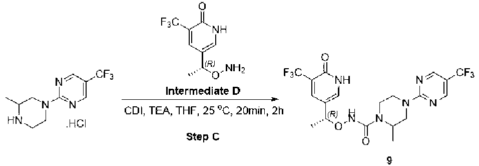

To a solution of (R)-5-(1-(aminooxy)ethyl)-3-(trifluoromethyl)pyridin-2(1H)-one (200 mg, 0.900 mmol, 1.0 eq.) in THF (5 mL) was added N,N'-carbonyldiimidazole (160 mg, 0.990 mmol, 1.1 eq.) and stirred at 25 °C for 20 min. DIPEA (464 mg, 3.603 mmol, 4.0 eq.) and 2-(3-methylpiperazin-1-yl)-5-(trifluoromethyl)pyrimidine hydrochloride (279 mg, 0.990 mmol, 1.0 eq.) were added to the reaction mixture. The reaction mixture was further reacted at 25 °C for 2 hours. The reaction mixture was quenched with water (10 mL) and extracted with EtOAc (2 × 10 mL). The organic phases were combined, washed with saturated brine (10 mL), dried over anhydrous sodium sulfate, and concentrated. The crude product was purified by prep-HPLC to give compound 9,2-methyl-N-((R)-1-(6-oxo-5-(trifluoromethyl)-1,6-dihydropyridin-3-yl)ethoxy)-4-(5-(trifluoromethyl)p yrimidin-2-yl)piperazine-1-carboxamide (210 mg, white solid), yield: 47.2%.

LCMS: *(ESI)* [M+H]⁺ = 495.2.

¹H NMR: (400 MHz, DMSO-*d₆*) *δ* 12.28 (s, 1H), 9.67 - 9.60 (m, 1H), 8.70 (s, 2H), 8.02 (s, 1H), 7.67 (d, *J* = 2.2 Hz, 1H), 4.69 - 4.62 (m, 1H), 4.57 - 4.49 (m, 1H), 4.48 - 4.41 (m, 1H), 4.22 - 4.13 (m, 1H), 3.72 - 3.63 (m, 1H), 3.25 - 3.18 (m, 1H), 3.08 - 2.95 (m, 2H), 1.39 (d, *J =* 6.6 Hz, 3H), 1.01 - 0.93 (m, 3H).

### Step D: Preparation of (S)-2-methyl-N-((R)-1-(6-oxo-5-(trifluoromethyl)-1,6-dihydropyridin-3-yl)etho xy)-4-(5-(trifluoromethyl)pyrimidin-2-yl)piperazine-1-carboxamide and (R)-2-methyl-N-((R)-1-(6-oxo-5-(trifluoromethyl)-1,6-dihydropyridin-3-yl)ethoxy)-4-(5-(trifluoromethyl)pyrimidin-2-yl)piperazine-1-ca rboxamide

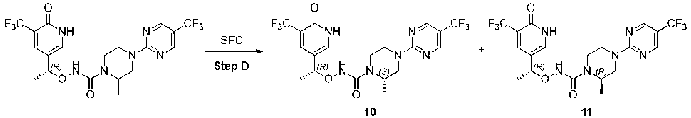

2-Methyl-N-((R)-1-(6-oxo-5-(trifluoromethyl)-1,6-dihydropyridin-3-yl)ethoxcy)-4-(5-(trifluoromethyl)pyri midin-2-yl)piperazine-1-carboxamide (120 mg, 0.242 mmol) Purified by supercritical fluid chromatography (SFC) to give:
Isomer 10, (S)-2-methyl-N-((R)-1-(6-oxo-5-(trifluoromethyl)-1,6-dihydropyridin-3-yl)ethoxy)-4-(5-(trifl uoromethyl)pyrimidin-2-yl)piperazin-1-carboxamide (25 mg, white solid), yield: 20.8%;
LCMS: *(ESI)* [M+H]⁺= 495.2.

¹H NMR: (400 MHz, DMSO-*d₆*) *δ* 12.28 (s, 1H), 9.65 (s, 1H), 8.70 (s, 2H), 8.02 (d, *J =* 2.0 Hz, 1H), 7.67 (d, *J =* 2.1 Hz, 1H), 4.70 - 4.62 (m, 1H), 4.52 (d, *J =* 9.9 Hz, 1H), 4.46 (d, *J =* 13.2 Hz, 1H), 4.23 - 4.11 (m, 1H), 3.73 - 3.59 (m, 1H), 3.21 (dd, *J* = 13.3, 3.7 Hz, 1H), 3.07 - 2.94 (m, 2H), 1.39 (d, 3H), 0.98 (d, *J* = 6.7 Hz, 3H).

Isomer 11, (R)-2-methyl-N-((R)-1-(6-oxo-5-(trifluoromethyl)-1,6-dihydropyridin-3-yl)ethoxy)-4-(5-(trif luoromethyl)pyrimidin-2-yl)piperazin-1-carboxamide (20 mg, white solid), yield: 16.6%.

LCMS: *(ESI)* [M+H]⁺= 495.2.

¹H NMR: (400 MHz, DMSO-*d*₆) *δ* 12.28 (s, 1H), 9.63 (s, 1H), 8.71 (s, 2H), 8.01 (d, *J =* 1.8 Hz, 1H), 7.67 (d, *J* = 2.0 Hz, 1H), 4.69 - 4.60 (m, 1H), 4.52 (d, *J* = 9.8 Hz, 1H), 4.45 (d, *J* = 13.2 Hz, 1H), 4.22 - 4.11 (m, 1H), 3.73 - 3.63 (m, 1H), 3.22 (dd, *J =* 13.3, 3.8 Hz, 1H), 3.09 - 2.95 (m, 2H), 1.39 (d, 3H), 0.96 (d, *J* = 6.6 Hz, 3H).

### Examples 12, 13, 14:

### Synthesis of Compound 12

### N-(1-(6-oxo-5-(trifluoromethyl)-1,6-dihydropyridin-3-yl)ethoxy)-4-(4-(trifluoromethyl)-1H-pyrrolor2,3-c ]pyridin-7-yl)piperazine-1-carboxamide

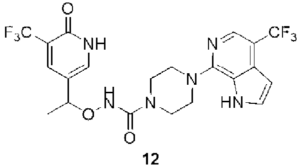

### Synthesis of Compound 13

### (R)-N-(1-(6-oxo-5-(trifluoromethyl)-1,6-dihydropyridin-3-yl)ethoxy)-4-(4-(trifluoromethyl)-1H-pyrrolo[2 ,3-c]pyridin-7-yl)piperazine-1-carboxamide

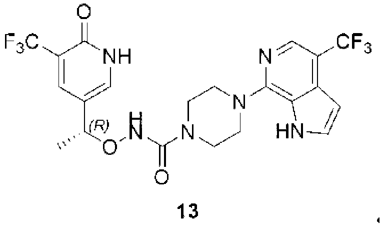

### Synthesis of Compound 14

### (S)-N-(1-(6-oxo-5-(trifluoromethyl)-1,6-dihydropyridin-3-yl)ethoxy)-4-(4-(trifluoromethyl)-1H-pyrrolo[2 ,3-c]pyridin-7-yl)piperazine-1-carboxylic acid tert-butyl ester

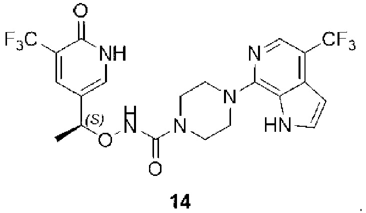

### Step A: Preparation of 7-chloro-4-(trifluoromethyl)-1H-pyrrolo[2,3-c]pyridine

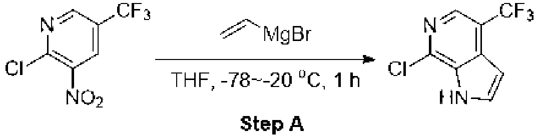

To a solution of 5-(trifluoromethyl)-3-nitro-2-chloropyridine (4.00 g, 17.66 mmol, 1.0 eq.) in THF (80 mL) was added dropwise vinyl magnesium bromide (1 M, 106 mL, 106.00 mmol, 6.0 eq.) under nitrogen protection at -78 °C. The reaction mixture was reacted at -20 °C for 1 h. The re action mixture was quenched with saturated ammonium chloride aqueous solution (100 mL) and extracted with EtOAc (2 × 100 mL). The combined organic phases were washed with saturated brine (100 mL), dried over anhydrous sodium sulfate, and concentrated. The crude product was p urified by column chromatography (petroleum ether/EtOAc = 4/1; V/V) to give 7-chloro-4-(trifluo romethyl)-1H-pyrrolo[2,3-c]pyridine (900 mg, yellow solid), yield: 23.1%.

LCMS: *(ESI)* [M+H]⁺= 221.0.

¹H NMR: (400 MHz, CDCl₃) *δ* 9.00 (s, 1H), 8.33 (d, *J =* 1.2 Hz, 1H), 7.58 - 7.56 (m, 1H), 6.84 - 6.81 (m, 1H).

### Step B: Preparation of 4-(4-(trifluoromethyl)-1H-pyrrolo[2,3-c]pyridin-7-yl)piperazine-1-carboxylic acid tert-butyl ester

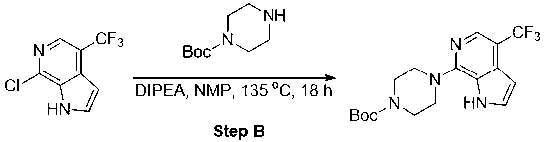

7-Chloro-4-(trifluoromethyl)-1H-pyrrolo[2,3-c]pyridine (300 mg, 1.36 mmol, 1.0 eq.) was dissolved in N-methylpyrrolidone (5 mL), followed by the addition of DIPEA (704 mg, 5.44 mmol, 4.0 eq.) and 1-Boc-piperazine (760 mg, 4.08 mmol, 3.0 eq.). The reaction mixture was reacted at 135 °C for 18 h. The reaction mixture was cooled to room temperature and diluted with water (80 mL), and extracted with EtOAc (2 × 80 mL). The organic phases were combined, washed with saturated brine (50 mL), dried over anhydrous sodium sulfate, and concentrated. The crude product was purified by column chromatography (petroleum ether/EtOAc = 1/1;V/V) to obtain tert-butyl 4-(4-(trifluoromethyl)-1H-pyrrolo[2,3-c]pyridin-7-yl)piperazine-1-carboxylic acid (500 mg, yellow solid), yield: 99.3%.

LCMS: *(ESI)* [M+H]⁺ = 371.0.

¹H NMR: (400 MHz, CDCl₃) *δ* 10.90 (s, 1H), 8.09 (d, *J =* 1.2 Hz, 1H), 7.32 (t, *J =* 2.8 Hz, 1H), 6.62 - 6.56 (m, 1H), 3.62 - 3.57 (m, 4H), 3.54 - 3.50 (m, 4H), 1.46 (s, 9H).

### Step C: Preparation of 7-(piperazine-1-yl)-4-(trifluoromethyl)-1H-pyrrolo[2,3-c]pyridine hydrochloride

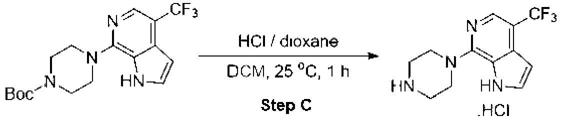

4-(4-(trifluoromethyl)-1H-pyrrolo[2,3-c]pyridin-7-yl)piperazine-1-carboxylic acid tert-butyl ester (300 mg, 0.81 mmol, 1.0 mg) was dissolved in dichloromethane (2 mL) and dioxane hydrochloride s olution (4 M, 4 mL) was added. The reaction mixture was reacted at 25 °C for 1 hour. The rea ction mixture was concentrated to give 7-(piperazin-1-yl)-4-(trifluoromethyl)-1H-pyrrolo[2,3-c]pyridi ne hydrochloride (300 mg, crude product, yellow solid).

LCMS: *(ESI)* [M+H]⁺ = 271.0.

### Step D: Preparation of N-(1-(6-oxo-5-(trifluoromethyl)-1,6-dihydropyridin-3-yl)ethoxy)-4-(4-(trifluoro methyl)-1H-pyrrolo[2,3-c]pyridin-7-yl)piperazine-1-carboxamide

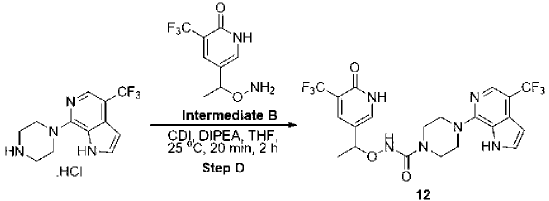

5-(1-(aminooxy)ethyl)-3-(trifluoromethyl)pyridin-2(1H)-one (71 mg, 0.32 mmol, 1.1 eq.) was dissol ved in THF (5 mL), and N,N'-carbonyldiimidazole (52 mg, 0.32 mmol, 1.1 eq.) was added. The reaction mixture was reacted at 25 °C for 20 min. DIPEA (150 mg, 1.16 mmol, 4.0 eq.) and 7-(piperazin-1-yl)-4-(trifluoromethyl)-1H-pyrrolo[2,3-c]pyridine hydrochloride (90 mg, 0.29 mmol, 1.0 eq.) were added to the reaction mixture. The reaction mixture was further reacted at 25 °C for 2 hours. The reaction mixture was quenched with water (50 mL) and extracted with EtOAc (2 × 50 mL). The organic phases were combined, washed with saturated brine (120 mL), dried over anhydrous sodium sulfate, and concentrated. The crude product was purified by prep-HPLC to ob tain racemic compound 12,N-(1-(6-oxo-5-(trifluoromethyl)-1,6-dihydropyridin-3-yl)ethoxy)-4-(4-(triflu oromethyl)-1H-pyrrolo[2,3-c]pyridin-7-yl)piperazine-1-carboxamide (47 mg, white solid), yield: 30. 9%.

LCMS: *(ESI)* [M+H]⁺ = 519.2.

¹H NMR: (400 MHz, DMSO-*d*₆) *δ* 12.30 (s, 1H), 11.83 (s, 1H), 9.70 (s, 1H), 8.09 - 7.99 (m, 2H), 7.71 - 7.62 (m, 2H), 6.52 (s, 1H), 4.67 (q, *J =* 6.4 Hz, 1H), 3.45 (s, 8H), 1.40 (d, *J =* 6.8 Hz, 3H).

### Step E: Preparation of (R)-N-(1-(6-oxo-5-(trifluoromethyl)-1,6-dihydropyridin-3-yl)ethoxy)-4-(4-(triflu oromethyl)-1H-pyrrolo[2,3-c]pyridin-7-yl)piperazine-1-carboxamide and (S)-N-(1-(6-oxo-5-(trifluorom ethyl)-1,6-dihydropyridin-3-yl)ethoxy)-4-(4-(trifluoromethyl)-1H-pyrrolo[2,3-c]pyridin-7-yl)piperazine-1 -carboxamide

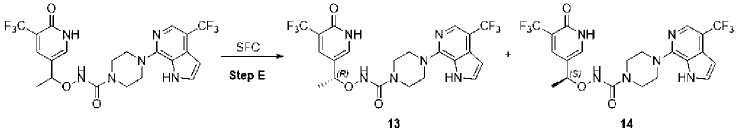

N-(1-(6-oxo-5-(trifluoromethyl)-1,6-dihydropyridin-3-yl)ethoxy)-4-(4-(trifluoromethyl)-1H-pyrrolo[2,3-c ]pyridin-7-yl)piperazine-1-carboxamide (40 mg, 0.08 mmol, 1.0 eq.) was purified by supercritical fluid chromatography (SFC) to give:
isomer 13, (R)-N-(1-(6-oxo-5-(trifluoromethyl)-1,6-dihydropyridin-3-yl)ethoxy)-4-(4-(trifluoromethyl)-1H-pyrrolo[2,3-c]pyridin-7-yl)piperazine-1-carboxamide (9.84 mg, white solid), yield: 24.6%;
LCMS: *(ESI)* [M+H]⁺ = 519.2.

¹H NMR: (400 MHz, DMSO-*d*₆) *δ* 12.30 (s, 1H), 11.82 (s, 1H), 9.69 (s, 1H), 8.08 - 8.00 (m, 2H), 7.69 - 7.62 (m, 2H), 6.52 (s, 1H), 4.67 (q, *J =* 6.4 Hz, 1H), 3.45 (s, 8H), 1.40 (d, *J =* 6.4 Hz, 3H).

and isomer 14,(S)-N-(1-(6-oxo-5-(trifluoromethyl)-1,6-dihydropyridin-3-yl)ethoxy)-4-(4-(trifluorometh yl)-1H-pyrrolo[2,3-c]pyridin-7-yl)piperazine-1-carboxamide (5.51 mg, white solid), yield: 13.8%. LCMS: *(ESI)* [M+H]⁺ = 519.2.

¹H NMR: (400 MHz, DMSO-*d*₆) *δ* 12.30 (s, 1H), 11.83 (s, 1H), 9.70 (s, 1H), 8.07 - 8.02 (m, 2H), 7.69 (s, 1H), 7.65 (t, *J =* 2.8 Hz, 1H), 6.56 - 6.50 (m, 1H), 4.68 (q, *J =* 6.4 Hz, 1H), 3.46 (s, 8H), 1.40 (d, *J =* 6.8 Hz, 3H).

### Example 15: Synthesis of Compound 15

### (R)-N-(1-(6-oxo-5-(trifluoromethyl)-1,6-dihydropyridin-3-yl)ethoxy)-4-(7-(trifluoromethyl)-1H-imidazo[ 4,5-c]pyridin-4-yl)piperazine-1-carboxamide

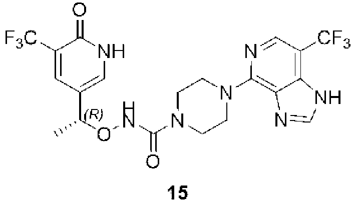

### Step A: Preparation of tert-butyl 4-(7-bromo-1-((2-(trimethylsilyl)ethoxy)methyl)-1H-imidazo[4,5-c]p yridin-4-yl)piperazine-1-carboxylic acid

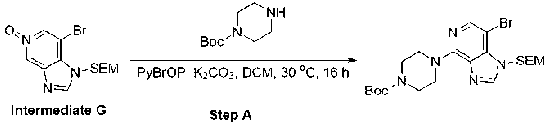

7-Bromo-1-((2-(trimethylsilyl)ethoxy)methyl)-1H-imidazo[4,5-c]pyridine 5-oxide (3.00 g, 8.71 mmol, 1.0 eq.) was dissolved in dichloromethane (30 mL), followed by the addition of 1-Boc piperazine (16.23 g, 87.14 mmol, 10.0 eq.), PyBrOP (5.28 g, 11.32 mmol, 1.3 eq.), and potassium carbonate (3.61 g, 26.13 mmol, 3.0 eq.). The reaction mixture was reacted at 30 °C for 16 h. The reaction mixture was quenched with water (100 mL) and extracted with EtOAc (2 × 100 mL). The organic phases were combined, washed with saturated brine (200 mL), dried over anhydrous sodium sulfate, and concentrated. The crude product was purified by column chromatography (petroleum ether/EtOAc = 4/1; V/V) to obtain 4-(7-bromo-1-((2-(trimethylsilyl)ethoxy)methyl)-1H-imidazo[4,5-c]pyridin-4-yl)piperazine-1-carboxylic acid tert-butyl ester (4.10 g, yellow solid), yield: 91.8%.

LCMS: *(ESI)* [M+H]⁺ = 512.2.

¹H NMR: (400 MHz, CDCl₃) *δ* 8.03 (s, 1H), 7.86 (s, 1H), 5.83 (s, 2H), 4.16 - 4.05 (m, 4H), 3.65 - 3.56 (m, 6H), 1.52 (s, 9H), 1.00 - 0.91 (m, 2H), 0.00 (s, 9H).

### Step B: Preparation of tert-butyl 4-(7-(trifluoromethyl)-1-((2-(trimethylsilyl)ethoxy)methyl)-1H-imida zo[4,5-c]pyridin-4-yl)piperazine-1-carboxylic acid

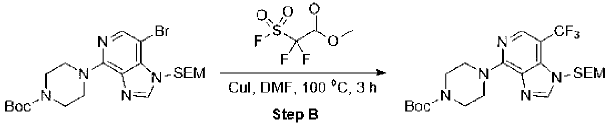

To a solution of 4-(7-bromo-1-((2-(trimethylsilyl)ethoxy)methyl)-1H-imidazo[4,5-c]pyridin-4-yl)piper azine-1-carboxylic acid tert-butyl ester (1.60 g, 3.12 mmol, 1.0 eq.) in N,N-dimethylformamide (2 0 mL) was added,cuprous iodide (297 mg, 1.56 mmol, 0.5 eq.) and methyl fluorosulfonyl difluor oacetate (2.40 g, 12.48 mmol, 4.0 eq.). The reaction mixture was reacted at 100 °C for 3 h und er nitrogen protection. The reaction mixture was cooled to room temperature and diluted with wa ter (80 mL), then extracted with EtOAc (2 × 100 mL). The organic phases were combined, was hed with saturated brine (300 mL), dried over anhydrous sodium sulfate, and concentrated. The c rude product was purified by column chromatography (petroleum ether/EtOAc = 4/1; V/V) to giv e 4-(7-(trifluoromethyl)-1-((2-(trimethylsilyl)ethoxy)methyl)-1H-imidazo[4,5-c]pyridin-4-yl)piperazine-1-carboxylic acid tert-butyl ester (400 mg, yellow solid), yield: 25.5%.

LCMS: *(ESI)* [M+H]⁺ = 502.2.

¹H NMR: (400 MHz, DMSO-*d*₆) *δ* 8.55 (s, 1H), 8.28 (s, 1H), 5.67 (s, 2H), 4.40 - 4.22 (m, 4H), 3.64 - 3.57 (m, 2H), 3.56 - 3.48 (m, 4H), 1.50 (s, 9H), 0.92 - 0.87 (m, 2H), 0.00 (s, 9H).

### Step C: Preparation of 4-(piperazine-1-yl)-7-(trifluoromethyl)-1H-imidazo[4,5-c]pyridine hydrochloride

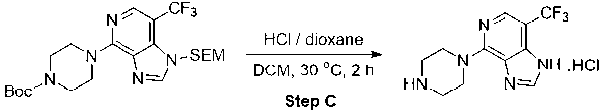

4-(7-(trifluoromethyl)-1-((2-(trimethylsilyl)ethoxy)methyl)-1H-imidazo[4,5-c]pyridin-4-yl)piperazine-1-c arboxylic acid tert-butyl ester (400 mg, 0.80 mmol, 1.0 eq.) was dissolved in dichloromethane (5 mL), and dioxane hydrochloride solution (4 M, 20 mL) was added. The reaction mixture was re acted at 30 °C for 2 hours. The reaction mixture was concentrated to give 4-(piperazine-1-yl)-7-(t rifluoromethyl)-1H-imidazo[4,5-c]pyridine hydrochloride (280 mg, crude product, white solid).

LCMS: *(ESI)* [M+H]⁺ = 272.4.

### Step D: Preparation of (R)-N-(1-(6-oxo-5-(trifluoromethyl)-1,6-dihydropyridin-3-yl)ethoxy)-4-(7-(trifl uoromethyl)-1H-imidazo[4,5-c]pyridin-4-yl)piperazine-1-carboxamide

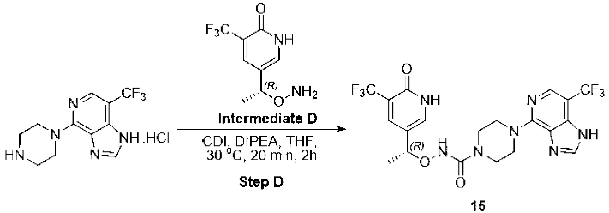

To a solution of (R)-5-(1-(aminooxy)ethyl)-3-(trifluoromethyl)pyridin-2(1H)-one (70 mg, 0.32 mmol, 1.0 eq.) in THF (10 mL) was added N, N'-carbonyldiimidazole (57 mg, 0.35 mmol, 1.1 eq.). The reaction mixture was reacted at 30 °C for 20 min. DIPEA (165 mg, 1.28 mmol, 4.0 eq.) and 4-(piperazin-1-yl)-7-(trifluoromethyl)-1H-imidazo[4,5-c]pyridine hydrochloride (98 mg, 0.32 mmol, 1.0 eq.) were added to the reaction mixture. The reaction mixture was reacted at 30 °C for 2 h. The reaction mixture was quenched with water (50 mL) and extracted with EtOAc (2 × 50 mL). The organic phases were combined, washed with saturated brine (120 mL), dried over anhydrous sodium sulfate, and concentrated. The crude product was purified by prep-HPLC to give compound 15, (R)-N-(1-(6-oxo-5-(trifluoromethyl)-1,6-dihydropyridin-3-yl)ethoxy)-4-(7-(trifluoromethyl)-1H-imidazo[ 4,5-c]pyridin-4-yl)piperazine-1-carboxamide (49.22 mg, white solid), yield: 33.1%.

LCMS: *(ESI)* [M+H]⁺ = 520.2.

¹H NMR: (400 MHz, DMSO-*d₆*) *δ* 13.21 (s, 1H), 12.30 (s, 1H), 9.68 (s, 1H), 8.26 (s, 1H), 8.12 (d, *J* = 1.2 Hz, 1H), 8.04 (d, *J* = 2.0 Hz, 1H), 7.72 - 7.66 (m, 1H), 4.67 (q, *J =* 6.8 Hz, 1H), 4.32 - 4.12 (m, 4H), 3.41 - 3.35 (m, 4H), 1.40 (d, *J =* 6.8 Hz, 3H).

### Example 16: Synthesis of Compound 16

### N-((R)-1-(6-oxo-5-(trifluoromethyl)-1,6-dihydropyridin-3-yl)ethoxy)-3-(5-(trifluoromethyl)pyrimidin-2-y 1)-3,8-diazabicyclo[3.2.1]octane-8-carboxamide

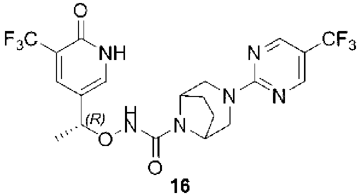

### Step A: Preparation of tert-butyl 3-(5-(trifluoromethyl)pyrimidin-2-yl)-3,8-diazabicyclo[3.2.1]octane-8 -carboxylic acid

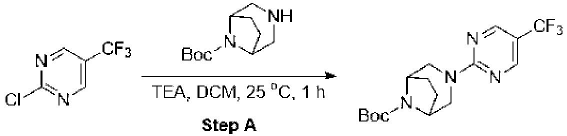

2-chloro-5-(trifluoromethyl)pyrimidin (819 mg, 4.49 mmol, 1.0 eq.) was dissolved in dichlorometh ane (10 mL), followed by the addition of TEA (908 mg, 8.97 mmol, 2.0 eq.) and 8-BOC-3,8-dia zabicyclo[3.2.1]octane (1.00 g, 4.71 mmol, 1.05 eq.). The reaction mixture was reacted at 25 °C for 1 h. The reaction mixture was diluted with saturated ammonium chloride aqueous solution (5 0 mL) and extracted with dichloromethane (3 × 30 mL). The organic phases were combined, dri ed over anhydrous sodium sulfate, and concentrated. The crude product was purified by column chromatography (petroleum ether/EtOAc = 5/1; V/V) to obtain tert-butyl 3-(5-(trifluoromethyl)pyri midin-2-yl)-3,8-diazabicyclo[3.2.1]octane-8-carboxylate (1.45 g, white solid), yield: 90.2%.

LCMS: *(ESI)* [M+H-56]⁺ = 303.2.

¹H NMR: (400 MHz, CDCl₃) *δ* 8.48 (s, 2H), 4.53 - 4.45 (m, 2H), 4.45 - 4.22 (m, 2H), 3.26 - 3.11 (m, 2H), 1.99 - 1.87 (m, 2H), 1.69 - 1.62 (m, 2H), 1.49 (s, 9H).

### Step B: Preparation of 3-(5-(trifluoromethyl)pyrimidin-2-yl)-3,8-diazabicyclo[3.2.1]octane hydrochloride

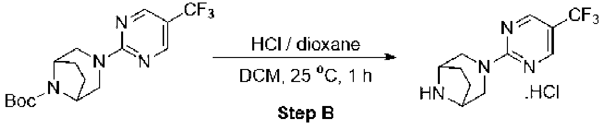

Tert-butyl 3-(5-(trifluoromethyl)pyrimidin-2-yl)-3,8-diazabicyclo[3.2.1]octane-8-carboxylic acid (700 mg, 1.95 mmol, 1.0 eq.) was dissolved in dichloromethane (10 mL), and dioxane hydrochloride s olution (4 M, 5 mL) was added. The reaction mixture was reacted at 25 °C for 1 hour. A solid precipitated from the reaction mixture. The reaction mixture was filtered, and the solid was drie d under vacuum to obtain 3-(5-(trifluoromethyl)pyrimidin-2-yl)-3,8-diazabicyclo[3.2.1]octane hydroc hloride (500 mg, white solid), yield: 86.9%.

LCMS: *(ESI)* [M+H]⁺= 259.2.

### Step C: Preparation of N-((R)-1-(6-oxo-5-(trifluoromethyl)-1,6-dihydropyridin-3-yl)ethoxy)-3-(5-(trifl uoromethyl)pyrimidin-2-yl)-3,8-diazabicyclo[3.2.1]octane-8-carboxamide

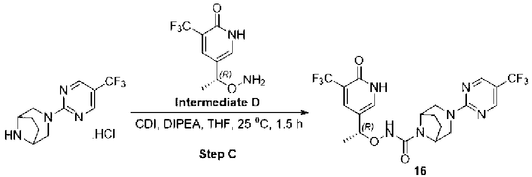

(R)-5-(1-(aminooxy)ethyl)-3-(trifluoromethyl)pyridin-2(1H)-one (50 mg, 0.22 mmol, 1.1 eq.) was di ssolved in THF (0.5 mL), and N, N'-carbonyldiimidazole (36 mg, 0.22 mmol, 1.1 eq.) was added. The reaction mixture was reacted at 25 °C for 0.5 h. A solution of 3-(5-(trifluoromethyl)pyrimid in-2-yl)-3,8-diazabicyclo[3.2.1]octane hydrochloride (60 mg, 0.20 mmol, 1.0 eq.) and DIPEA (105 mg, 0.81 mmol, 4.0 eq.) in THF (0.5 mL) was added to the reaction mixture. The reaction mix ture was further reacted at 25 °C for 1 hour. The reaction mixture was quenched with water (50 mL) and extracted with EtOAc (3 × 30 mL). The organic phases were combined, washed with saturated brine (50 mL), dried over anhydrous sodium sulfate, and concentrated. The crude produ ct was purified by prep-HPLC to give compound 16, N-((R)-1-(6-oxo-5-(trifluoromethyl)-1,6-dihyd ropyridin-3-yl)ethoxy)-3-(5-(trifluoromethyl)pyrimidin-2-yl)-3,8-diazabicyclo[3.2.1]octane-8-carboxamid e (5.31 mg, white solid), yield: 5.2%.

LCMS: *(ESI)* [M+H]⁺ = 507.0.

¹H NMR: (400 MHz, DMSO-*d*₆) *δ* 12.27 (s, 1H), 9.81 (s, 1H), 8.70 (d, *J =* 0.4 Hz, 2H), 8.00 (d, *J =* 2.0 Hz, 1H), 7.68 (d, *J* = 2.0 Hz, 1H), 4.69 (q, *J =* 6.4 Hz, 1H), 4.39 - 4.31 (m, 2H), 4.27 - 4.21 (m, 2H), 3.03 (dd, *J* = 12.0, 8.8 Hz, 2H), 1.77 - 1.70 (m, 2H), 1.54 - 1.47 (m, 2H), 1.39 (d, *J =* 6.4 Hz, 3H).

Following the same method used to synthesize compound 16, compounds 17 and 18 were prepared using (R)-5-(1-(aminooxy)ethyl)-3-(trifluoromethyl)pyridin-2(1H)-one (intermediate D) and suitable amine compounds.

### Example 17: Compound 17

### N-((R)-1-(6-oxo-5-(trifluoromethyl)-1,6-dihydropyridin-3-yl)ethoxy)-8-(5-(trifluoromethyl)pyrimidin-2-y 1)-3,8-diazabicyclo[3.2.1]octane-3-carboxamide

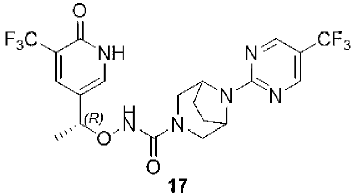

LCMS: *(ESI)* [M+H]⁺ = 507.2.

¹H NMR: (400 MHz, DMSO-*d*₆) *δ* 12.30 (s, 1H), 9.55 (s, 1H), 8.72 (d, *J =* 0.8 Hz, 2H), 8.01 (d, *J=* 2.0 Hz, 1H), 7.67 (d, *J =* 2.0 Hz, 1H), 4.73 (s, 2H), 4.65 (q, *J =* 6.4 Hz, 1H), 3.65 (dd, *J =* 24.4, 12.0 Hz, 2H), 2.92 (t*, J* = 12.0 Hz, 2H), 1.93 - 1.80 (m, 2H), 1.71 - 1.60 (m, 2H), 1.38 (d, *J =* 6.8 Hz, 3H).

### Example 18: Compound 18

### N-((R)-1-(6-oxo-5-(trifluoromethyl)-1,6-dihydropyridin-3-yl)ethoxy)-3-(5-(trifluoromethyl)pyrimidin-2-y l)-3,6-diazabicyclo[3.1.1]heptane-6-carboxamide

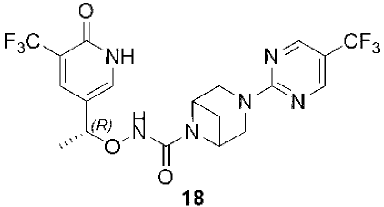

LCMS: *(ESI)* [M+H]⁺ = 493.2.

¹H NMR: (400 MHz, DMSO-*d*₆) *δ* 12.18 (s, 1H), 9.75 (s, 1H), 8.74 (s, 2H), 7.90 (d, *J =* 2.4 Hz, 1H), 7.54 (d, *J* = 2.0 Hz, 1H), 4.56 (q, *J =* 6.4 Hz, 1H), 4.32 - 4.21 (m, 2H), 4.01 (t, *J =* 12.0 Hz, 2H), 3.55 (dd, *J =* 12.4, 8.4 Hz, 2H), 2.62 - 2.54 (m, 1H), 1.49 (d, *J =* 8.8 Hz, 1H), 1.30 (d, *J =* 6.8 Hz, 3H).

### Example 19: Synthesis of Compound 19

### (R)-4-(5-acetamidopyrimidin-2-yl)-N-(1-(6-oxo-5-(trifluoromethyl)-1,6-dihydropyridin-3-yl)ethoxy)piper azine-1-carboxamide

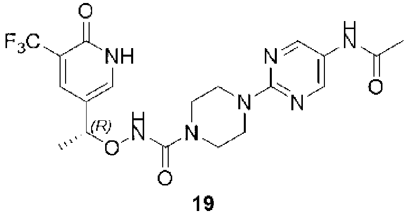

### Step A: Preparation of tert-butyl 4-(5-nitropyrimidin-2-yl)piperazine-1-carboxylate

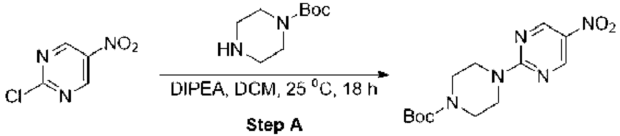

1-Boc-piperazine (583 mg, 3.31 mmol, 1.0 eq.) was dissolved in dichloromethane (10 mL). DIPEA (1.21 g, 9.39 mmol, 3.0 eq.) and 2-chloro-5-nitropyrimidine (500 mg, 3.31 mmol, 1.0 eq.) were added sequentially. The reaction mixture was reacted at 25 °C for 18 h. The reaction mixture was quenched with water (80 mL), adjusted to pH 6 with dilute hydrochloric acid (1 M), and extracted with EtOAc (2 × 70 mL). The organic phases were combined, dried over anhydrous sodium sulfate, and concentrated to give 4-(5-nitropyrimidin-2-yl)piperazine-1-carboxylic acid tert-butyl ester (960 mg, pale yellow solid), yield: 93.8%.

LCMS: *(ESI)* [M+H-56]⁺ = 254.0.

¹H NMR: (400 MHz, DMSO-*d*₆) *δ* 9.13 (s, 2H), 3.94 - 3.90 (m, 4H), 3.48 - 3.44 (m, 4H), 1.43 (s, 9H).

### Step B: Preparation of 4-(5-aminopyrimidin-2-yl)piperazine-1-carboxylic acid tert-butyl ester

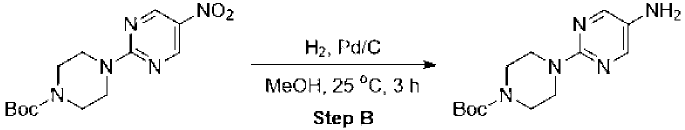

4-(5-nitropyrimidin-2-yl)piperazine-1-carboxylic acid tert-butyl ester (460 mg, 1.49 mmol, 1.0 eq.) was dissolved in methanol (10 mL), and palladium on carbon (46 mg) was added under nitrogen protection. The reaction mixture was degassed with a hydrogen balloon for 3 times and the mix ture was reacted at 25 °C for 3 h under a hydrogen atmosphere. The reaction mixture was filter ed to remove palladium on carbon, and the filtrate was concentrated to give 4-(5-aminopyrimidin-2-yl)piperazine-1-carboxylic acid tert-butyl ester (400 mg, yellow solid), yield: 96.2%.

LCMS: *(ESI)* [M+H]⁺ = 280.2.

¹H NMR: (400 MHz, CDCl₃) *δ* 8.00 (s, 2H), 3.70 - 3.65 (m, 4H), 3.55 - 3.46 (m, 4H), 3.20 (s, 2H), 1.50 (s, 9H).

### Step C: Preparation of tert-butyl 4-(5-acetaminopyrimidin-2-yl)piperazine-1-carboxylate

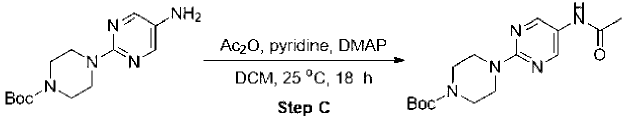

To a solution of 4-(5-aminopyrimidin-2-yl)piperazine-1-carboxylate (tert-butyl) (370 mg, 1.32 mmo I, 1.0 eq.) in dichloromethane (20 mL) was added 4-dimethylaminopyridine (9 mg, 0.07 mmol, 0. 05 eq.), pyridine (157 mg, 1.98 mmol, 1.5 eq.), and acetic anhydride (202 mg, 1.98 mmol, 1.5 e q.). The reaction mixture was reacted at 25 °C for 18 h. The reaction mixture was quenched wit h water (50 mL) and extracted with dichloromethane (2 × 45 mL). The organic phases were co mbined, dried over anhydrous sodium sulfate, and concentrated to give tert-butyl 4-(5-acetaminopy rimidin-2-yl)piperazine-1-carboxylate (370 mg, white solid), yield: 87.3%.

LCMS: *(ESI)* [M+H]⁺ = 322.2.

¹H NMR: (400 MHz, CDCl₃) *δ* 8.42 (s, 2H), 7.12 (s, 1H), 3.80 - 3.73 (m, 4H), 3.51 - 3.44 (m, 4H), 2.17 (s, 3H), 1.48 (s, 9H).

### Step D: Preparation of N-(2-(piperazine-1-yl)pyrimidin-5-yl)acetamide hydrochloride

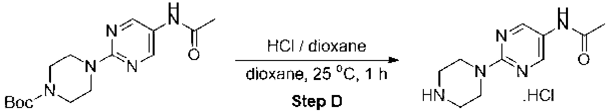

tert-butyl 4-(5-acetaminopyrimidin-2-yl)piperazine-1-carboxylate (370 mg, 1.15 mmol, 1.0 eq.) was dissolved in dioxane (2 mL), and dioxane hydrochloride solution (4 M, 6 mL) was added. The reaction mixture was reacted at 25 °C for 1 h. The reaction mixture was concentrated to give N-(2-(piperazine-1-yl)pyrimidin-5-yl)acetamide hydrochloride (330 mg, crude product, white solid).

LCMS: *(ESI)* [M+H]⁺ = 222.4.

### Step E: Preparation of (R)-4-(5-acetamidopyrimidin-2-yl)-N-(1-(6-oxo-5-(trifluoromethyl)-1,6-dihydro pyridin-3-yl)ethoxy)piperazine-1-carboxamide

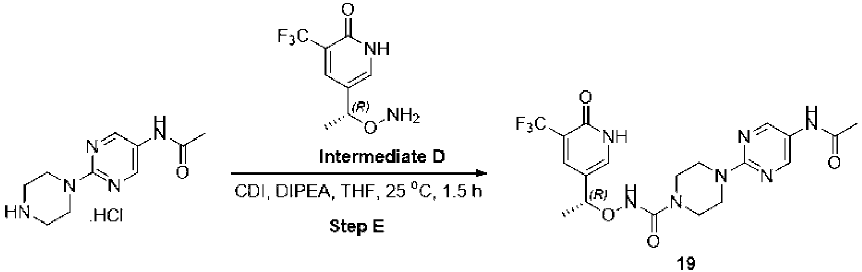

To a solution of (R)-5-(1-(aminooxy)ethyl)-3-(trifluoromethyl)pyridin-2(1H)-one (389 mg, 1.75 mm ol, 1.5 eq.) in THF (10 mL) was added N,N'-carbonyldiimidazole (227 mg, 1.40 mmol, 1.2 eq.). The reaction mixture was reacted at 25 °C for 0.5 h. N-(2-(piperazin-1-yl)pyrimidin-5-yl)acetami de hydrochloride (300 mg, 1.17 mmol, 1.0 eq.) and DIPEA (605 mg, 4.68 mmol, 4.0 eq.) were added to the reaction mixture. The reaction mixture was further reacted at 25 °C for 1 h. The r eaction mixture was quenched with water (60 mL) and extracted with EtOAc (2 × 50 mL). The organic phases were combined, washed with saturated brine (2 × 100 mL), dried over anhydrou s sodium sulfate, and concentrated. The crude product was purified by prep-HPLC to give compo und 19, (R)-4-(5-acetamidopyrimidin-2-yl)-N-(1-(6-oxo-5-(trifluoromethyl)-1,6-dihydropyridin-3-yl)eth oxy)piperazin-1-carboxamide (11.49 mg, white solid), yield: 2.1%.

LCMS: *(ESI)* [M+H]⁺ = 470.2.

¹H NMR: (400 MHz, DMSO-*d*₆) *δ* 9.87 (s, 1H), 9.65 (s, 1H), 8.51 (s, 2H), 8.01 - 7.94 (m, 1H), 7.72 - 7.65 (m, 1H), 4.64 (q, *J =* 6.4 Hz, 1H), 3.66 - 3.58 (m, 4H), 3.31 - 3.28 (m, 4H), 2.01 (s, 3H), 1.38 (d, *J =* 6.8 Hz, 3H).

### Example 20: Synthesis of Compound 20

### (R)-4-(5-cyclopropylpyrimidin-2-yl)-N-(1-(6-oxo-5-(trifluoromethyl)-1,6-dihydropyridin-3-yl)ethoxy)pip erazine-1-carboxamide

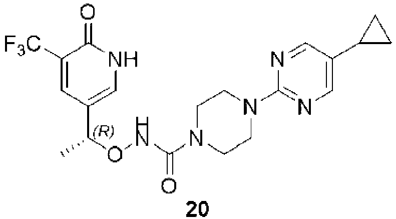

### Step A: Preparation of tert-butyl 4-(5-cyclopropylpyrimidin-2-yl)piperazine-1-carboxylate

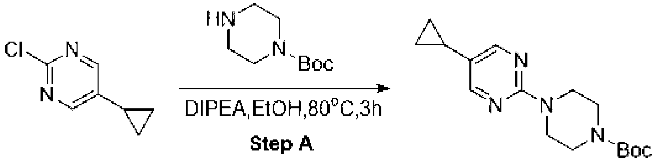

To a solution of 2-Chloro-5-cyclopropylpyrimidine (75 mg, 0.488 mmol, 1.0 eq.) in ethanol (3 m L) was added DIPEA (190 mg, 1.47 mmol, 3.0 eq.) and piperazine-1-carboxylic acid tert-butyl es ter (100 mg, 0.54 mmol, 1.1 eq.) sequentially. The reaction mixture was reacted at 80 °C for 3 h. The reaction mixture was diluted with water (20 mL) and extracted with EtOAc (2 × 20 mL). The organic phases were combined, dried over anhydrous sodium sulfate, and concentrated. The crude product was purified by column chromatography (petroleum ether/EtOAc = 88/12; V/V) to give 4-(5-cyclopropylpyrimidine-2-yl)piperazine-1-carboxylic acid tert-butyl ester (100 mg, white s olid), yield: 68.3%.

LCMS: *(ESI)* [M+H]⁺ = 249.2.

### Step B: Preparation of 5-cyclopropyl-2-(piperazine-1-yl)pyrimidine hydrochloride

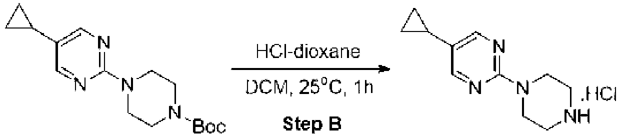

4-(5-cyclopropylpyrimidin-2-yl)piperazine-1-carboxylic acid tert-butyl ester (100 mg, 0.329 mmol, 1.0 eq.) was dissolved in dichloromethane and 2 mL of dioxane hydrochloride solution was added. The reaction mixture was reacted at 25 °C for 1 h. The reaction mixture was concentrated to give 5-cyclopropyl-2-(piperazine-1-yl)pyrimidine hydrochloride (90 mg, crude product, white solid).

LCMS: *(ESI)* [M+H]⁺ = 205.3.

### Step C: Preparation of (R)-4-(5-cyclopropylpyrimidin-2-yl)-N-(1-(6-oxo-5-(trifluoromethyl)-1,6-dihydr opyridin-3-yl)ethoxy)piperazine-1-carboxamide

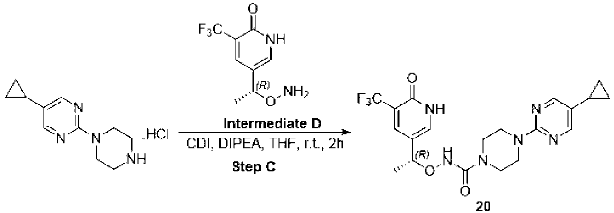

To a solution of (R)-5-(1-(aminooxy)ethyl)-3-(trifluoromethyl)pyridin-2(1H)-one (108 mg, 0.49 mmol, 1.1 eq.) in THF (3 mL)was added N,N'-carbonyldiimidazole (80 mg, 0.49 mmol, 1.1 eq.). The reaction mixture was reacted at 25 °C for 20 min. DIPEA (232 mg, 1.8 mmol, 4.0 eq.) and 5-cyclopropyl-2-(piperazin-1-yl)pyrimidine hydrochloride (90 mg, 0.45 mmol, 1.0 eq.) were added to the reaction mixture. The reaction mixture was continued to react at room temperature for 2 hours. The reaction mixture was quenched with water (30 mL) and extracted with EtOAc (2 × 30 mL). The organic phases were combined, washed with saturated brine (40 mL), dried over anhydrous sodium sulfate, and concentrated. The crude product was purified by prep-HPLC to give compound 20,(R)-4-(5-cyclopropylpyrimidin-2-yl)-N-(1-(6-oxo-5-(trifluoromethyl)-1,6-dihydropyridin-3-yl)ethoxy) piperazin-1-carboxamide (78.58 mg, white solid), yield: 87.3%.

LCMS: *(ESI)* [M+H]⁺= 453.2.

¹H NMR (400 MHz, DMSO-*d*₆) *δ* 12.29 (s, 1H), 9.65 (s, 1H), 8.18 (s, 2H), 8.03 (d, *J* = 2.1 Hz, 1H), 7.68 (s, 1H), 4.66 (q, *J* = 6.5 Hz, 1H), 3.69 - 3.56 (m, 4H), 3.31 - 3.24 (m, 4H), 1.83 - 1.69 (m, 1H), 1.39 (d, *J* = 6.6 Hz, 3H), 0.95 - 0.81 (m, 2H), 0.70 - 0.51 (m, 2H).

### Example 21: Synthesis of Compound 21

### (R)-4-(4-chloro-5-(trifluoromethyl)pyridin-2-yl)-N-(1-(6-oxo-5-(trifluoromethyl)-1,6-dihydropyridin-3-yl )ethoxy)piperazine-1-carboxamide

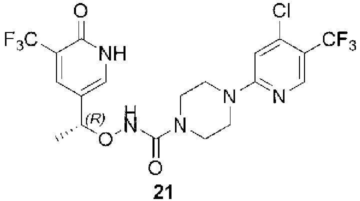

### Step A: Preparation of 2-(4-(tert-butyloxycarbonyl)piperazin-1-yl)-5-(trifluoromethyl)isonicotinic acid

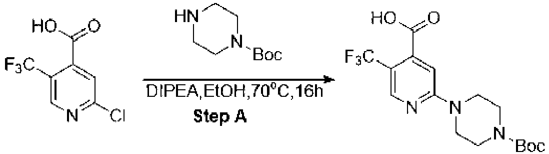

To a solution of 2-chloro-5-(trifluoromethyl)isonicotinic acid (2.4 g, 10.6 mmol, 1.0 eq.) in ethanol (25 mL) was added piperazine-1-carboxylic acid tert-butyl ester (2.97 g, 16.0 mmol, 1.5 eq.) and DIPEA (4.1 g, 31.8 mmol, 3.0 eq.). The reaction mixture was reacted at 70 °C for 16 h. The reaction mixture was concentrated, and the crude product was purified by column chromatography (dichloromethane/methanol = 9/1; V/V) to obtain 2-(4-(tert-butoxycarbonyl)piperazin-1-yl)-5-(trifluoromethyl)isonicotinic acid (3.2 g, white solid), yield: 80.0%.

LCMS: *(ESI)* [M+H]⁺ = 320.2.

### Step B: Preparation of tert-butyl 4-(4-((methoxycarbonyl)amino)-5-(trifluoromethyl)pyridin-2-yl)piper azine-1-carboxylic acid

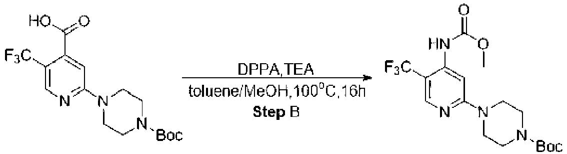

To a solution of 2-(4-(tert-butoxycarbonyl)piperazin-1-yl)-5-(trifluoromethyl)isonicotinic acid (3.2 g, 8.54 mmol, 1.0 eq.) in toluene (40 mL) was added TEA (1.72 g, 17.07 mmol, 2.0 eq.) and Diphenyl azidophosphate (9.3 g, 34.16 mmol, 4.0 eq.) at 0 °C, and the reaction mixture was reacted at 100 °C for 30 min. The reaction mixture was cooled to room temperature and methanol (10 mL) was added. The reaction mixture was then reacted at 100 °C for 16 hours. The reaction mixture was cooled to room temperature and concentrated. The crude product obtained was purified by column chromatography (petroleum ether/EtOAc = 75/25; V/V) to give tert-butyl 4-(4-((methoxycarbonyl)amino)-5-(trifluoromethyl)pyridin-2-yl)piperazine-1-carboxylate (1.5 g, yellow oil), yield: 44.1%.

LCMS: *(ESI)* [M+H]⁺ = 405.4.

### Step C: Preparation of tert-butyl 4-(4-amino-5-(trifluoromethyl)pyridin-2-yl)piperazine-1-carboxylic acid

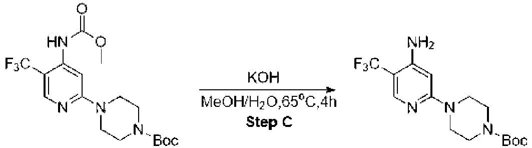

To a solution of tert-butyl 4-(4-((methoxycarbonyl)amino)-5-(trifluoromethyl)pyridin-2-yl)piperazine-1-carboxylate (1.3 g, 3.22 mmol, 1.0 eq.) in a mixed solvent of methanol (10 mL) and water (1 0 mL) was added potassium hydroxide (720 mg, 12.8 mmol, 4.0 eq.). The reaction mixture was reacted at 65 °C for 4 hours. The reaction mixture was quenched with water (40 mL) and extra cted with EtOAc (2 × 35 mL). The organic phases were combined, dried over anhydrous sodium sulfate, and concentrated. The crude product was purified by column chromatography (petroleum ether/EtOAc = 75/25; V/V) to give 4-(4-amino-5-(trifluoromethyl)pyridin-2-yl)piperazine-1-carboxy lic acid tert-butyl ester (430 mg, colorless oil), yield: 39%.

LCMS: (*ESI)* [M+H]⁺ = 347.2.

¹H NMR (400 MHz, DMSO-*d₆*) *δ* 7.97 (s, 1H), 6.07 (s, 2H), 5.99 (s, 1H), 3.46 - 3.36 (m, 8H), 1.42 (s, 9H).

### Step D: Preparation of tert-butyl 4-(4-chloro-5-(trifluoromethyl)pyridin-2-yl)piperazine-1-carboxylic acid

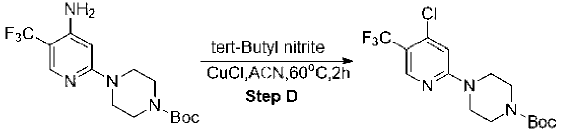

To a solution of 4-(4-amino-5-(trifluoromethyl)pyridin-2-yl)piperazine-1-carboxylic acid tert-butyl ester (215 mg, 0.63 mmol, 1.0 eq.) in acetonitrile (3 mL) was added tert-butyl nitrite (128 mg, 1.24 mmol, 2.0 eq.) and cuprous chloride (123 mg, 1.24 mmol, 2.0 eq.) at r.t... The reaction mixture was reacted at 60 °C for 2 h. The reaction mixture was quenched with water (30 mL) and extracted with EtOAc (3 × 25 mL). The organic phases were combined, dried over anhydrous sodium sulfate, and concentrated. The crude product was purified by column chromatography (petroleum ether/EtOAc = 4/1; V/V) to give tert-butyl 4-(4-chloro-5-(trifluoromethyl)pyridin-2-yl)piperazine-1-carboxylic acid (110 mg, colorless oil), yield: 48.6%.

LCMS: (*ESI)* [M+H-56]⁺ = 310.2.

¹H NMR (400 MHz, DMSO-*d₆*) *δ* 8.42 (s, 1H), 7.15 (s, 1H), 3.71 - 3.62 (m, 4H), 3.47 - 3.36 (m, 4H), 1.42 (s, 9H).

### Step E: Preparation of 1-(4-chloro-5-(trifluoromethyl)pyridin-2-yl)piperazine trifluoroacetate

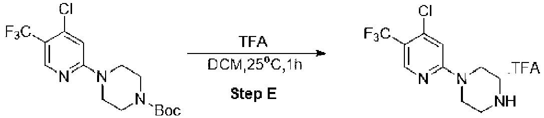

4-(4-chloro-5-(trifluoromethyl)pyridin-2-yl)piperazine-1-carboxylic acid tert-butyl ester (220 mg, 0.61 mmol, 1.0 eq.) was dissolved in dichloromethane (1 mL), and trifluoroacetic acid (1 mL) was added. The reaction mixture was reacted at 25 °C for 1 h. The reaction mixture was concentrated to give 1-(4-chloro-5-(trifluoromethyl)pyridin-2-yl)piperazine trifluoroacetate (160 mg, white solid), yield: 100%.

LCMS: (*ESI)* [M+H]⁺ = 266.2.

### Step F: Preparation of (R)-4-(4-chloro-5-(trifluoromethyl)pyridin-2-yl)-N-(1-(6-oxo-5-(trifluoromethyl) -1,6-dihydropyridin-3-yl)ethoxy)piperazine-1-carboxamide

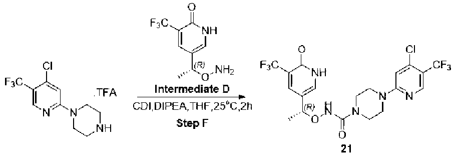

(R)-5-(1-(aminooxy)ethyl)-3-(trifluoromethyl)pyridin-2(1H)-one (110 mg, 0.49 mmol, 1.1 eq.) and N,N'-carbonyldiimidazole (80 mg, 0.49 mmol, 1.1 eq.) were dissolved in THF (3 mL), and the r eaction mixture was reacted at 25 °C for 20 min. Then, DIPEA (232 mg, 1.8 mmol, 4.0 eq.) an d 1-(4-chloro-5-(trifluoromethyl)pyridin-2-yl)piperazine trifluoroacetate (120 mg, 0.45 mmol, 1.0 e q.) were added to the reaction mixture. The reaction mixture was reacted at 25 °C for 2 h. The reaction mixture was quenched with water (30 mL) and extracted with EtOAc (2 × 30 mL). Th e organic phases were combined, dried over anhydrous sodium sulfate, and concentrated. The cru de product was purified by prep-HPLC to give compound 21,(R)-4-(4-chloro-5-(trifluoromethyl)pyr idin-2-yl)-N-(1-(6-oxo-5-(trifluoromethyl)-1,6-dihydropyridin-3-yl)ethoxy)piperazine-1-carboxamide (8 0 mg, colorless oil), yield: 34.3%.

LCMS: *(ESI)* [M+H]⁺ = 514.0.

¹H NMR (400 MHz, DMSO-*d*₆) *δ* 12.30 (s, 1H), 9.72 - 9.67 (m, 1H), 8.43 (s, 1H), 8.03 (d, *J =* 2.0 Hz, 1H), 7.68 (s, 1H), 7.16 (s, 1H), 4.66 (q, *J =* 6.5 Hz, 1H), 3.69 - 3.57 (m, 4H), 3.37 - 3.33 (m, 4H), 1.39 (d, *J* = 6.6 Hz, 3H).

### Example 22: Synthesis of Compound 22

### N-(1-(5-bromo-6-oxo-1,6-dihydropyridin-3-yl)ethoxy)-4-(5-(trifluoromethyl)pyrimidin-2-yl)piperazine-1 -carboxamide

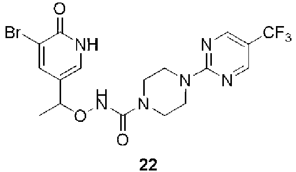

### Step A: Preparation of 2-(1-(5-bromo-6-oxo-1,6-dihydropyridin-3-yl)ethoxy)isodihydroindole-1,3-dione

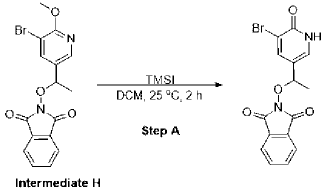

To a solution of 2-(1-(5-bromo-6-methoxypyridin-3-yl)ethoxy)isodihydroindole-1,3-dione (1 g, 2.65 mmol, 1.0 eq.) in dichloromethane (10 mL) was added TMSI (1 mL). The reaction mixture was reacted at 25 °C for 2 h. The reaction mixture was quenched with water (10 mL) and extracted with EtOAc (3 × 15 mL). The organic phases were combined, washed with saturated brine (50 mL), dried over anhydrous sodium sulfate, and concentrated. The crude product was purified by column chromatography (petroleum ether / EtOAc = 3 / 1; V / V) to give 2-(1-(5-bromo-6-oxo-1,6-dihydropyridin-3-yl)ethoxy)isodihydroindole-1,3-dione (700 mg, yellow solid), yield: 72.7%.

LCMS: *(ESI)* [M+H]⁺ = 363.0.

### Step B: Preparation of 5-(1-(aminooxy)ethyl)-3-bromopyridin-2(1H)-one

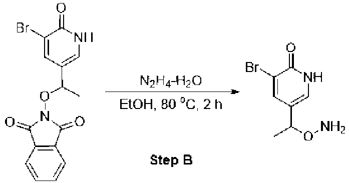

To a solution of 2-(1-(5-bromo-6-oxo-1,6-dihydropyridin-3-yl)ethoxy)isodihydroindole-1,3-dione (700 mg, 1.92 mmol, 1.0 eq.) in ethanol (10 mL) was added hydrazine hydrate (85%, 576 mg, 11.52 mmol, 6.0 eq.). The reaction mixture was reacted at 80 °C for 2 hours. The reaction mixture w as concentrated, and dichloromethane (10 mL) was added to the residue. The insoluble solid was removed by filtration, and the filtrate was concentrated. The crude product obtained was purified by column chromatography (petroleum ether/EtOAc = 1/1; V/V) to give 5-(1-(aminooxy)ethyl)-3-bromopyridin-2(1H)-one (250 mg, white solid), yield: 55.8%.

LCMS: *(ESI)* [M+H]⁺ = 233.2.

¹H NMR: (400 MHz, DMSO-*d*₆) *δ* 12.00 (s, 1H), 7.87 (d, *J =* 2.3 Hz, 1H), 7.33 (d, *J =* 2.2 Hz, 1H), 7.26 - 7.10 (m, 2H), 4.34 (q, *J =* 6.5 Hz, 1H), 1.26 (d, *J =* 6.5 Hz, 3H).

### Step C: Preparation of N-(1-(5-bromo-6-oxo-1,6-dihydropyridin-3-yl)ethoxy)-4-(5-(trifluoromethyl)pyr imidin-2-yl)piperazine-1-carboxamide

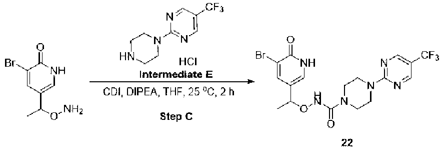

To a solution of 5-(1-(aminooxy)ethyl)-3-bromopyridin-2(1H)-one (150 mg, 0.64 mmol, 1.0 eq.) in THF (4 mL) was added N, N'-carbonyldiimidazole (124 mg, 0.76 mmol, 1.2 eq.). The reaction mixture was stirred at 25 °C for 0.5 h. 2-(piperazin-1-yl)-5-(trifluoromethyl)pyrimidine hydrochlori de (188 mg, 0.70 mmol, 1.1 eq.) and DIPEA (330 mg, 2.56 mmol, 4.0 eq.) was added to the re action mixture. The reaction mixture was stirred at 25 °C for 2 h. Quench the reaction mixture with water (10 mL) and extract with EtOAc (3 × 10 mL). Combine the organic phases, wash wi th saturated brine (20 mL), dry to anhydrous sodium sulfate, and concentrate. The crude product was purified by prep-HPLC to give compound 22, N-(1-(5-bromo-6-oxo-1,6-dihydropyridin-3-yl)eth oxy)-4-(5-(trifluoromethyl)pyrimidin-2-yl)piperazine-1-carboxamide (13.44 mg, white solid), with a recovery of 4.2%.

LCMS: *(ESI)* [M+H]⁺ = 491.2.

¹H NMR: (400 MHz, DMSO-*d*₆) *δ* 12.05 (s, 1H), 9.66 (s, 1H), 8.72 (s, 2H), 8.03 (d, *J =* 2.3 Hz, 1H), 7.41 (*d, J* = 2.2 Hz, 1H), 4.59 (q, *J =* 6.5 Hz, 1H), 3.83 - 3.72 (m, 4H), 3.40 - 3.33 (m, 4H), 1.37 (d, *J=* 6.5 Hz, 3H).

Example 23: Synthesis of Compound 23

### N-(1-(5-acetyl-6-oxo-1,6-dihydropyridin-3-yl)ethoxy)-4-(5-(trifluoromethyl)pyrimidin-2-yl)piperazine-1-carboxamide

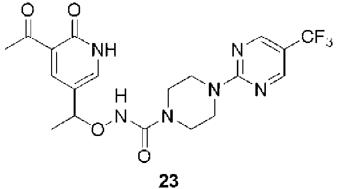

### Step A: Preparation of 2-(1-(5-acetyl-6-methoxypyridin-3-yl)ethoxy)isodihydroindole-1,3-dione

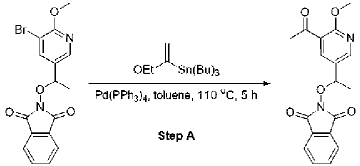

To a solution of 2-(1-(5-bromo-6-methoxypyridin-3-yl)ethoxy)isodihydroindole-1,3-dione (intermedia te H, 3 g, 7.97 mmol, 1.0 eq.) in toluene (30 mL) was added tributyl(1-ethoxyvinyl)stanane (4 g, 11.17 mmol, 1.4 eq.) and tetrakis(triphenylphosphine)palladium (460 mg, 0.38 mmol, 0.05 eq.). The reaction mixture was reacted at 110 °C for 5 h. A potassium fluoride aqueous solution (30 mL) was added to the reaction mixture and stirred for 1 h. The reaction mixture was diluted wit h water (20 mL) and extracted with EtOAc (3 × 30 mL). The organic phases were combined, dr ied over anhydrous sodium sulfate, and concentrated. The crude product was purified by column chromatography (petroleum ether / EtOAc = 3 / 1; V / V) to give 2-(1-(5-acetyl-6-methoxypyridi n-3-yl)ethoxy)isodihydroindole-1,3-dione (1.5 g, white solid), yield: 55.3%.

LCMS: *(ESI)* [M+H]⁺ = 341.2, t_{R} = 0.731.

¹H NMR: (400 MHz, DMSO-*d*₆) *δ* 8.47 (d, *J* = 2.5 Hz, 1H), 8.22 (d, *J =* 2.5 Hz, 1H), 7.87 - 7.78 (m, 4H), 5.40 (q, J=6.5 Hz, 1H), 3.98 (s, 3H), 2.57 (s, 3H), 1.66 (d, *J =* 6.6 Hz, 3H).

### Step B: Preparation of 2-(1-(5-acetyl-6-oxo-1,6-dihydropyridin-3-yl)ethox)isodihydroindole-1,3-dione

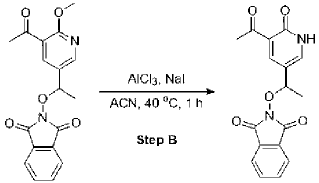

To a solution of 2-(1-(5-acetyl-6-methoxypyridin-3-yl)ethoxy)isodihydroindole-1,3-dione (1 g, 2.94 mmol, 1.0 eq.) in acetonitrile (15 mL) was added aluminum trichloride (977 mg, 7.35 mmol, 2.5 eq.) and sodium iodide (882 mg, 5.88 mmol, 2.0 eq.). The reaction mixture was reacted at 40 °C for 1 hour. The reaction mixture was quenched with water (20 mL) and extracted with EtOAc (3 × 30 mL). The organic phases were combined, dried over anhydrous sodium sulfate, and concentrated. The crude product was purified by column chromatography (petroleum ether / EtOAc = 1/1; V/V) to give 2-(1-(5-acetyl-6-oxo-1,6-dihydropyridin-3-yl)ethoxy)isodihydroindole-1,3-dione (900 mg, yellow solid), yield: 93.9%.

LCMS: *(ESI)* [M+H]⁺ = 327.2.

### Step C: Preparation of 3-acetyl-5-(1-(aminooxy)ethyl)pyridin-2(1H)-one hydrochloride

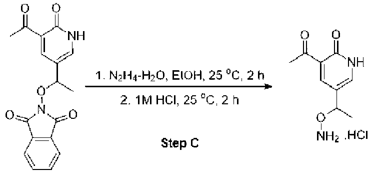

To a solution of 2-(1-(5-acetyl-6-oxo-1,6-dihydropyridin-3-yl)ethoxy)isodihydroindole-1,3-dione (500 mg, 1.53 mmol, 1.0 eq.) in ethanol (8 mL) was added hydrazine hydrate (85%, 230 mg, 4.60 mmol, 3.0 eq.). The reaction mixture was stirred at 25 °C for 2 hours. The reaction mixture was filtered, and the filtrate was concentrated. Dilute hydrochloric acid (1 M, 10 mL) was added, and the mixture was stirred at 25 °C for another 2 hours. The reaction mixture was concentrated, and the crude product was purified by reversed-phase column chromatography (acetonitrile/water = 2/1; V/V) to give 3-acetyl-5-(1-(aminooxy)ethyl)pyridin-2(1H)-one hydrochloride (50 mg, white solid), yield: 16.6%. LCMS: *(ESI)* [M+H]⁺ = 197.0.

### Step D: Preparation of N-(1-(5-acetyl-6-oxo-1,6-dihydropyridin-3-yl)ethoxy)-4-(5-(trifluoromethyl)pyr imidin-2-yl)piperazine-1-carboxamide

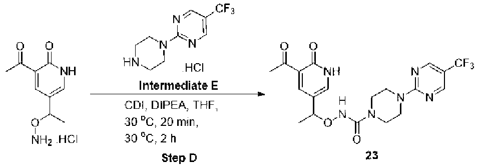

To a solution of 3-Acetyl-5-(1-(aminooxy)ethyl)pyridin-2(1H)-one (35 mg, 0.17 mmol, 1.0 eq.) in THF (3 mL) was added N,N'-carbonyldiimidazole (31 mg, 0.19 mmol, 1.1 eq.). The reaction mi xture was reacted at 30 °C for 20 min. DIPEA (98 mg, 0.76 mmol, 4.0 eq.) and 2-(piperazin-1-yl)-5-(trifluoromethyl)pyrimidine hydrochloride (51 mg, 0.19 mmol, 1.1 eq.) were added to the re action mixture. The reaction mixture was further reacted at 30 °C for 2 h. The reaction mixture was quenched with water (10 mL) and extracted with EtOAc (3 × 10 mL). The organic phases were combined, washed with saturated brine (10 mL), dried over anhydrous sodium sulfate, and concentrated. The crude product was purified by prep-HPLC to give compound 23, N-(1-(5-acetyl -6-oxo-1,6-dihydropyridin-3-yl)ethoxy)-4-(5-(trifluoromethyl)pyrimidin-2-yl)piperazine-1-carboxamide (13.38 mg, white solid), yield: 16.8%.

LCMS: *(ESI)* [M+H]⁺ = 455.2.

¹H NMR: (400 MHz, DMSO-*d₆*) *δ* 11.84 (s, 1H), 8.77 - 8.72 (s, 2H), 7.61 (d, *J =* 2.6 Hz, 1H), 7.40 (d, *J* = 2.4 Hz, 1H), 5.16 (d, *J =* 4.3 Hz, 1H), 4.60 - 4.51 (m, 1H), 3.98 - 3.87 (m, 4H), 3.66 - 3.47 (m, 4H), 2.27 (s, 3H), 1.28 (d, *J =* 6.4 Hz, 3H).

### Examples 24, 25:

### Synthesis of Compound 24

### N-((6-oxo-5-(trifluoromethyl)-1,6-dihydropyridin-3-yl)methoxy)-1-(5-(trifluoromethyl)pyrimidin-2-yl)-1, 2,3,6-tetrahydropyridin-4-carboxamide

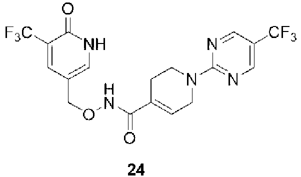

### Synthesis of Compound 25

### N-((6-oxo-5-(trifluoromethyl)-1,6-dihydropyridin-3-yl)methoxy)-1-(5-(trifluoromethyl)pyrimidin-2-yl)-1, 2,3,4-tetrahydropyridin-4-carboxamide

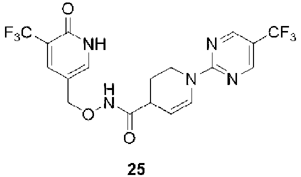

### Step A: Preparation of N-((6-oxo-5-(trifluoromethyl)-1,6-dihydropyridin-3-yl)methoxy)-1-(5-(trifluoro methyl)pyrimidin-2-yl)-1,2,3,6-tetrahydropyridin-4-carboxamide and N-((6-oxo-5-(trifluoromethyl)-1,6-dihydropyridin-3-yl)methoxy)-1-(5-(trifluoromethyl)pyrimidin-2-yl)-1,2,3,4-tetrahydropyridin-4-carboxa mide)

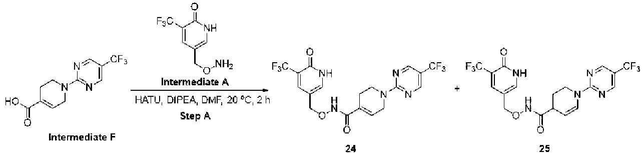

Dissolve 1-(5-(trifluoromethyl)pyrimidin-2-yl)-1,2,3,6-tetrahydropyridin-4-carboxylic acid (79 mg, 0. 29 mmol, 1.2 eq.) in N,N-dimethylformamide (5 mL), 5-((aminooxy)methyl)-3-(trifluoromethyl)pyri din-2(1H)-one (50 mg, 0.24 mmol, 1.0 eq.), HATU (119 mg, 0.31 mmol, 1.3 eq.), and DIPEA (93 mg, 0.72 mmol, 3.0 eq.) were added sequentially. The reaction mixture was reacted at 20 °C for 2 h. The reaction mixture was quenched with water (40 mL) and extracted with EtOAc (2 × 50 mL). The combined organic phases were washed with saturated brine (2 × 60 mL), dried o ver anhydrous sodium sulfate, and concentrated. The crude product was purified by prep-HPLC t o give compound 24, N-((6-oxo-5-(trifluoromethyl)-1,6-dihydropyridin-3-yl)methoxy)-1-(5-(trifluorom ethyl)pyrimidin-2-yl)-1,2,3,6-tetrahydropyridin-4-carboxamide (2.10 mg, white solid), yield: 1.9%;

LCMS: *(ESI)* [M+H]⁺ = 464.0.

¹H NMR: (400 MHz, DMSO-*d₆*) *δ* 12.32 (br s, 1H), 11.17 (s, 1H), 8.74 (s, 2H), 8.04 - 8.00 (m, 1H), 7.79 - 7.74 (m, 1H), 6.56 - 6.49 (m, 1H), 4.64 (s, 2H), 4.42 - 4.33 (m, 2H), 3.95 (t, *J =* 5.6 Hz, 2H), 2.35 - 2.31 (m, 2H).
and compound 25, N-((6-oxo-5-(trifluoromethyl)-1,6-dihydropyridin-3-yl)methoxy)-1-(5-(trifluorometh yl)pyrimidin-2-yl)-1,2,3,4-tetrahydropyridin-4-carboxamide (7.47 mg, white solid), yield: 6.7%. LCMS: *(ESI)* [M+H]⁺ = 464.0.

¹H NMR: (400 MHz, DMSO-*d₆*) *δ* 12.36 (br s, 1H), 11.05 (s, 1H), 8.85 (s, 2H), 8.01 - 7.96 (m, 1H), 7.79 - 7.73 (m, 1H), 7.56 (dd, *J* = 8.4, 1.6 Hz, 1H), 4.99 (dd, *J* = 8.4, 3.6 Hz, 1H), 4.61 (s, 2H), 3.95 (t, *J* = 5.6 Hz, 2H), 3.00 - 2.92 (m, 1H), 2.07 - 1.84 (m, 2H).

### Examples 26, 27, 28:

### Synthesis of Compound 26

### N-(1-(6-oxo-5-(trifluoromethyl)-1,6-dihydropyridin-3-ylethoxy)-1-(5-(trifluoromethyl)pyrimidin-2-y)-1, 2,3,6-tetrahydropyridin-4-carboxamide

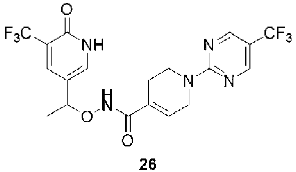

### Synthesis of Compound 27

### (R)-N-(1-(6-oxo-5-(trifluoromethyl)-1,6-dihydropyridin-3-yl)ethoxy)-1-(5-(trifluoromethyl)pyrimidin-2-y 1)-1,2,3,6-tetrahydropyridin-4-carboxamide

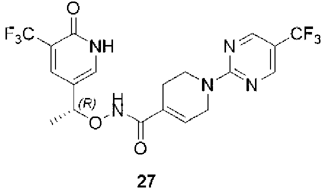

### Synthesis of Compound 28

### (S)-N-(1-(6-oxo-5-(trifluoromethyl)-1,6-dihydropyridin-3-yl)ethoxy)-1-(5-(trifluoromethyl)pyrimidin-2-y 1)-1,2,3,6-tetrahydropyridin-4-carboxamide

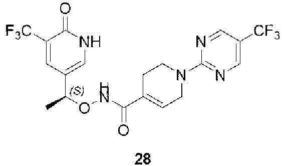

### Step A: Preparation of N-(1-(6-oxo-5-(trifluoromethyl)-1,6-dihydropyridin-3-yl)ethoxy)-1-(5-(trifluoro methyl)pyrimidin-2-yl)-1,2,3,6-tetrahydropyridin-4-carboxamide

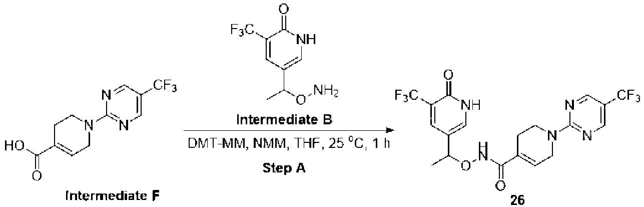

Dissolve 1-(5-(trifluoromethyl)pyrimidin-2-yl)-1,2,3,6-tetrahydropyridine-4-carboxylic acid (55 mg, 0. 20 mmol, 1.0 eq.) in THF (3.5 mL), cool to 0 °C in an ice bath, and was added 4-(4,6-dimetho xytriazine-2-yl)-4-methylmorpholine hydrochloride (77 mg, 0.26 mmol, 1.3 eq.). The solution was stirred at 0 °C for 5 min. N-methylmorpholine (61 mg, 0.60 mmol, 3.0 eq.) and 5-(1-(aminooxy) ethyl)-3-(trifluoromethyl)pyridine-2(1H)-one (667 mg, 0.30 mmol, 1.5 eq.) was added. The solutio n was stirred at 25 °C for 1 h. The reaction mixture was quenched with saturated ammonium ch loride (50 mL) and extracted with EtOAc (2 × 50 mL). The organic phases were combined, drie d over anhydrous sodium sulfate, and concentrated. The crude product was purified by prep-HPL C (formic acid) to give racemic compound 26, N-(1-(6-oxo-5-(trifluoromethyl)-1,6-dihydropyridin-3 -yl)ethoxy)-1-(5-(trifluoromethyl)pyrimidin-2-yl)-1,2,3,6-tetrahydropyridin-4-carboxamide (42 mg, whi te solid), yield: 44.0%.

LCMS: *(ESI)* [M+H]⁺ = 478.0.

¹H NMR: (400 MHz, DMSO-*d₆*) *δ* 12.30 (s, 1H), 11.01 (s, 1H), 8.73 (d, *J =* 0.8 Hz, 2H), 8.01 (d, *J =* 2.0 Hz, 1H), 7.68 (d, *J* = 2.0 Hz, 1H), 6.51 - 6.42 (m, 1H), 4.77 (q, *J =* 6.4 Hz, 1H), 4.39 - 4.29 (m, 2H), 3.93 (t, *J* = 5.6 Hz, 2H), 2.35 - 2.26 (m, 2H), 1.42 (d, *J =* 6.8 Hz, 3H).

### Step B: Preparation of (R)-N-(1-(6-oxo-5-(trifluoromethyl)-1,6-dihydropyridin-3-yl)ethoxy)-1-(5-(trifl uoromethyl)pyrimidin-2-yl)-1,2,3,6-tetrahydropyridin-4-carboxamide and (S)-N-(1-(6-oxo-5-(trifluorom ethyl)-1,6-dihydropyridin-3-yl)ethoxy)-1-(5-(trifluoromethyl)pyrimidin-2-yl)-1,2,3,6-tetrahydropyridin-4-carboxamide

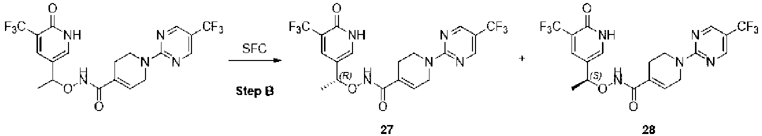

Racemic compound 26, N-(1-(6-oxo-5-(trifluoromethyl)-1,6-dihydropyridin-3-yl)ethoxy)-1-(5-(trifluor omethyl)pyrimidin-2-yl)-1,2,3,6-tetrahydropyridin-4-carboxamide (85 mg, 0.18 mmol, 1.0 eq.) in m ethanol (4 mL) and purified by supercritical fluid chromatography (SFC) to give:
Isomer 27, (R)-N-(1-(6-oxo-5-(trifluoromethyl)-1,6-dihydropyridin-3-yl)ethoxy)-1-(5-(trifluoromethyl)p yrimidin-2-yl)-1,2,3,6-tetrahydropyridin-4-carboxamide (19.06 mg, white solid), yield: 22.4%;
LCMS: *(ESI)* [M+H]⁺ = 478.0.

¹H NMR: (400 MHz, DMSO-*d₆*) *δ* 12.30 (s, 1H), 11.02 (s, 1H), 8.73 (d, *J =* 0.4 Hz, 2H), 8.01 (d, *J =* 2.0 Hz, 1H), 7.68 (d, *J =* 1.6 Hz, 1H), 6.50 - 6.43 (m, 1H), 4.77 (q, *J =* 6.4 Hz, 1H), 4.39 - 4.29 (m, 2H), 3.93 (t, *J* = 5.6 Hz, 2H), 2.36 - 2.26 (m, 2H), 1.42 (d, *J =* 6.8 Hz, 3H).

Isomer 28, (S)-N-(1-(6-oxo-5-(trifluoromethyl)-1,6-dihydropyridin-3-yl)ethoxy)-1-(5-(trifluoromethyl)p yrimidin-2-yl)-1,2,3,6-tetrahydropyridin-4-carboxamide (20.27 mg, white solid), yield: 23.8%. LCMS: *(ESI)* [M+H]⁺ = 478.2.

¹H NMR: (400 MHz, DMSO-*d₆*) *δ* 12.29 (s, 1H), 11.02 (s, 1H), 8.73 (s, 2H), 8.01 (d, *J =* 2.0 Hz, 1H), 7.68 (d, *J =* 2.0 Hz, 1H), 6.51 - 6.42 (m, 1H), 4.77 (q, *J =* 6.4 Hz, 1H), 4.39 - 4.30 (m, 2H), 3.93 (t, *J =* 5.6 Hz, 2H), 2.36 - 2.26 (m, 2H), 1.43 (d, *J =* 6.4 Hz, 3H).

### Examples 29, 30:

### Synthesis of Compound 29

### (R)-N-(1-deuter-1-(6-oxo-5-(trifluoromethyl)-1,6-dihydropyridin-3-yl)ethoxy)-1-(5-(trifluoromethyl)pyri midin-2-yl)-1,2,3,6-tetrahydropyridin-4-carboxamide

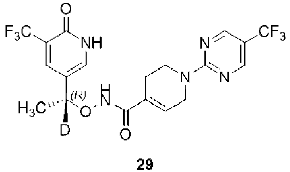

### Synthesis of Compound 30

### (S)-N-(1-deuter-1-(6-oxo-5-(trifluoromethyl)-1,6-dihydropyridin-3-yl)ethoxy)-1-(5-(trifluoromethyl)pyri midin-2-yl)-1,2,3,6-tetrahydropyridin-4-carboxamide

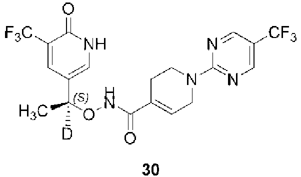

### Step A: Preparation of 1-Deuterium-1-(6-methoxy-5-(trifluoromethyl)pyridin-3-yl)ethanol

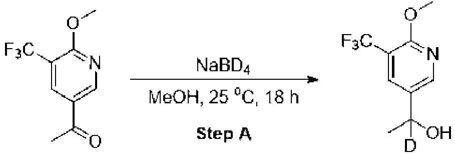

To a solution of 1-(6-methoxy-5-(trifluoromethyl)pyridin-3-yl)acetone (2.09 g, 9.54 mmol, 1.0 eq.) in methanol (50 mL) was added sodium borodeuteride (959 mg, 22.90 mmol, 2.4 eq.) at 0 °C in an ice bath. The reaction mixture was reacted at 25 °C for 18 h. The reaction mixture was quenched with water (200 mL) and extracted with EtOAc (2 × 180 mL). The organic phases were combined, washed with saturated brine (400 mL), dried over anhydrous sodium sulfate, and concentrated. The crude product was purified by column chromatography (petroleum ether/EtOAc = 1/1; V/V) to obtain 1-deuterium-1-(6-methoxy-5-(trifluoromethyl)pyridin-3-yl)ethanol (1.90 g, yellow oil), yield: 89.7%.

LCMS: *(ESI)* [M+H]⁺ = 223.4.

¹H NMR: (400 MHz, DMSO-*d₆*) *δ* 8.40 (d, *J =* 1.6 Hz, 1H), 8.03 (d, *J =* 2.0 Hz, 1H), 5.38 (s, 1H), 3.98 (s, 3H), 1.36 (s, 3H).

### Step B: Preparation of 2-(1-deuter-1-(6-methoxy-5-(trifluoromethyl)pvridin-3-yl)ethoxy)isoindoline-1, 3-dione

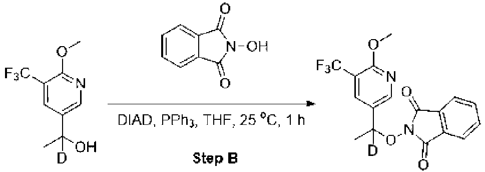

To a solution of 1-Deuterium-1-(6-methoxy-5-(trifluoromethyl)pyridin-3-yl)ethanol (1.80 g, 8.10 m mol, 1.0 eq.) in THF (40 mL) was added N-hydroxyphthalimide (1.32 g, 8.10 mmol, 1.0 eq.), tri phenylphosphine (4.46 g, 17.01 mmol, 2.1 eq.) sequentially, followed by the dropwise addition of diisopropyl azodicarbonate (3.28 g, 16.20 mmol, 2.0 eq.) at 0 °C in an ice bath,. The reaction mixture was reacted at 25 °C for 1 h. The reaction mixture was quenched with water (300 mL) and extracted with EtOAc (3 × 280 mL). The organic phases were combined, washed with satu rated brine (800 mL), dried over anhydrous sodium sulfate, and concentrated. The crude product was purified by column chromatography (petroleum ether/EtOAc = 2/1; V/V) to give 2-(1-deuteri um-1-(6-methoxy-5-(trifluoromethyl)pyridin-3-yl)ethoxy)isoindoline-1,3-dione (2.20 g, white solid), y ield: 73.9%.

LCMS: *(ESI)* [M+H]⁺ = 368.2.

¹H NMR: (400 MHz, CDCl₃) *δ* 8.21 (d, *J =* 2.0 Hz, 1H), 8.02 (dd, *J =* 2.4, 0.4 Hz, 1H), 7.68 - 7.64 (m, 2H), 7.63 - 7.58 (m, 2H), 3.90 (s, 3H), 1.60 (s, 3H).

### Step C: Preparation of 2-(1-deuter-1-(6-oxo-5-(trifluoromethyl)-1,6-dihydropyridin-3-yl)ethoxy)isoind oline-1,3-dione

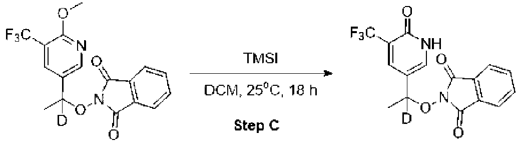

To a solution of 2-(1-deuterium-1-(6-methoxy-5-(trifluoromethyl)pyridin-3-yl)ethoxy)isoindoline-1,3-d ione (1.00 g, 2.72 mmol, 1.0 eq.) in dichloromethane (20 mL) was added TMSI (1 M, 2 mL, 5. 44 mmol, 2.0 eq.). The reaction mixture was reacted at 25 °C for 18 h. The reaction mixture w as quenched with water (100 mL) and extracted with dichloromethane (2 × 80 mL). The organic phases were combined, washed with saturated brine (200 mL), dried over anhydrous sodium sulf ate, and concentrated. The crude product was purified by column chromatography (petroleum ethe r/EtOAc = 1/2; V/V) to give 2-(1-deuterium-1-(6-oxo-5-(trifluoromethyl)-1,6-dihydropyridin-3-yl)eth oxy)isoindoline-1,3-dione (810 mg, yellow solid), yield: 84.2%.

LCMS: *(ESI)* [M+H]⁺ = 354.0.

¹H NMR: (400 MHz, CDCl₃) *δ* 8.12 (d, *J =* 2.0 Hz, 1H), 7.79 - 7.71 (m, 2H), 7.71 - 7.66 (m, 2H), 7.57 (d, *J* = 2.4 Hz, 1H), 1.61 (s, 3H).

### Step D: Preparation of 5-(1-faminooxy)-1-deuterethyl)-3-(trifluoromethyl)pyridin-2(1H)-one

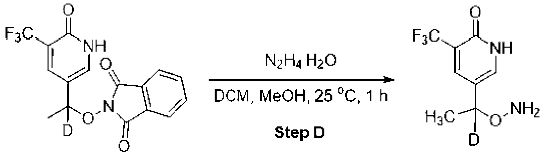

To a solution of 2-(1-deuterium-1-(6-oxo-5-(trifluoromethyl)-1,6-dihydropyridin-3-yl)ethoxy)isoindoli ne-1,3-dione (760 mg, 2.15 mmol, 1.0 eq.) in dichloromethane (6 mL) and methanol (3 mL) was added hydrazine hydrate (80%, 2 mL). The reaction mixture was reacted at 25 °C for 1 h. The reaction mixture was filtered, and the filtrate was concentrated. The residue was diluted with di chloromethane (20 mL), and the insoluble solid was filtered off. The filtrate was concentrated. T he crude product obtained was purified by column chromatography (dichloromethane/methanol = 10/1; V/V) to give 5-(1-(aminooxy)-1-deuteriumethyl)-3-(trifluoromethyl)pyridin-2(1H)-one (360 mg, white solid), yield: 75.0%.

LCMS: *(ESI)* [M+H]⁺ = 224.0.

¹H NMR: (400 MHz, DMSO-*d₆*) *δ* 12.26 (s, 1H), 7.84 (d, *J =* 2.0 Hz, 1H), 7.58 (d, *J =* 2.0 Hz, 1H), 5.86 (s, 2H), 1.27 (s, 3H).

### Step E: Preparation of N-(1-deuter-1-(6-oxo-5-(trifluoromethyl)-1,6-dihydropyridin-3-yl)ethoxy)-1-(5-(trifluoromethyl)pyrimidin-2-yl)-1,2,3,6-tetrahydropyridin-4-carboxamide

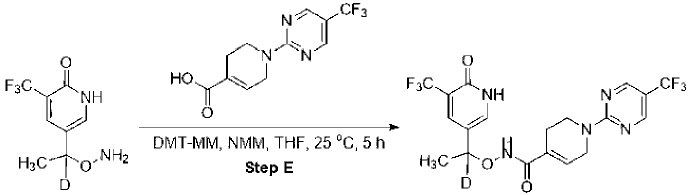

To a solution of 1-(5-(trifluoromethyl)pyrimidin-2-yl)-1,2,3,6-tetrahydropyridine-4-carboxylic acid (intermediate F, 330 mg, 1.21 mmol, 1.0 eq.) in THF (10 mL) was added 4-(4,6-dimethoxytriazine-2-yl)-4-methylmorpholine hydrochloride (435 mg, 1.57 mmol, 1.3 eq.) at 0 °C in an ice bath, . The reaction mixture was reacted at 0 °C for 20 min. N-methylmorpholine (367 mg, 3.63 mmol, 3.0 eq.) and 5-(1-(aminooxy)-1-deuterylethyl)-3-(trifluoromethyl)pyridine-2(1H)-one (270 mg, 1.21 mmol, 1.0 eq.) were added to the reaction mixture. The reaction mixture was reacted at 25 °C for 5 hours. The reaction mixture was quenched with water (100 mL) and extracted with EtOAc (2 × 80 mL). The combined organic phases were washed with saturated brine (200 mL), dried over anhydrous sodium sulfate, and concentrated. The crude product was purified by column chromatography (petroleum ether/EtOAc = 2/1; V/V) to give the crude racemic compound (N-(1-deuterium-1-(6-oxo-5-(trifluoromethyl)-1,6-dihydropyridin-3-yl)ethoxy)-1-(5-(trifluoromethyl)pyri midin-2-yl)-1,2,3,6-tetrahydropyridin-4-carboxamide (578 mg, white solid).

### Step F: Preparation of (R)-N-(1-deuterium-1-(6-oxo-5-(trifluoromethyl)-1,6-dihydropyridin-3-yl)ethox y)-1-(5-(trifluoromethyl)pyrimidin-2-yl)-1,2,3,6-tetrahydropyrimidin-4-carboxamide and (S)-N-(1-deute rium-1-(6-oxo-5-(trifluoromethyl)-1,6-dihydropyridin-3-yl)ethoxy)-1-(5-(trifluoromethyl)pyrimidin-2-yl) -1,2,3 ,6-tetrahydropyrimidin-4-carboxamide)

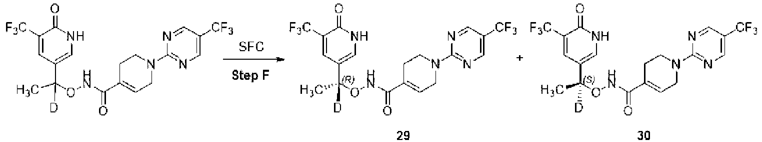

The racemic compound N-(1-deuterium-1-(6-oxo-5-(trifluoromethyl)-1,6-dihydropyridin-3-yl)ethoxy)-1-(5-(trifluoromethyl)pyrimidin-2-yl)-1,2,3,6-tetrahydropyridin-4-carboxamide (578 mg, white solid) was purified by supercritical fluid chromatography (SFC) to give:
Isomer 29, (R)-N-(1-deuterium-1-(6-oxo-5-(trifluoromethyl)-1,6-dihydropyridin-3-yl)ethoxy)-1-(5-(trifl uoromethyl)pyrimidin-2-yl)-1,2,3,6-tetrahydropyridin-4-carboxamide (61.20 mg, white solid), yield: 10.6%;
LCMS: *(ESI)* [M+H]⁺ = 479.0.

¹H NMR: (400 MHz, DMSO-*d₆*) *δ* 12.29 (s, 1H), 11.01 (s, 1H), 8.74 (s, 2H), 8.01 (d, J = 2.0 Hz, 1H), 7.69 (d, J = 2.0 Hz, 1H), 6.51 - 6.45 (m, 1H), 4.40 - 4.32 (m, 2H), 3.94 (t, J = 5.6 Hz, 2H), 2.34 - 2.28 (s, 2H), 1.42 (s, 3H).

And isomer 30, (S)-N-(1-deuter-1-(6-oxo-5-(trifluoromethyl)-1,6-dihydropyridin-3-yl)ethoxy)-1-(5-(trif luoromethyl)pyrimidin-2-yl)-1,2,3,6-tetrahydropyridin-4-carboxamide (61.94 mg, white solid), yield 10.7%.

LCMS: [M+H]⁺ = 479.2.

¹H NMR: (400 MHz, DMSO-*d₆*) *δ* 12.31 (s, 1H), 11.01 (s, 1H), 8.74 (s, 2H), 8.01 (d, *J =* 2.0 Hz, 1H), 7.69 (d, *J* = 2.0 Hz, 1H), 6.51 - 6.45 (m, 1H), 4.39 - 4.32 (m, 2H), 3.94 (t, *J =* 5.6 Hz, 2H), 2.36 - 2.27 (d, *J* = 1.7 Hz, 2H), 1.42 (s, 3H).

### Examples 31, 32, 33:

### Synthesis of Compound 31

### N-(1-(6-oxo-5-(trifluoromethyl)-1,6-dihydropyridin-3-yl)propoxy)-1-(5-(trifluoromethyl)pyrimidin-2-yl)-1,2,3,6-tetrahydropyridin-4-carboxamide

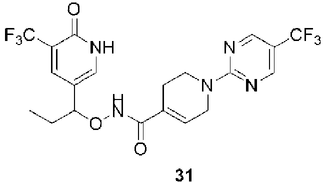

### Synthesis of Compound 32

### (R)-N-(1-(6-oxo-5-(trifluoromethyl)-1,6-dihydropyridin-3-yl)propoxy)-1-(5-(trifluoromethyl)pyrimidin-2 -yl)-1,2,3,6-tetrahydropyridin-4-carboxamide

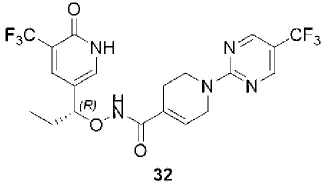

### Synthesis of Compound 33

### (S)-N-(1-(6-oxo-5-(trifluoromethyl)-1,6-dihydropyridin-3-yl)propoxy)-1-(5-(trifluoromethyl)pyrimidin-2-yl)-1,2,3 ,6-tetrahydropyridin-4-carboxamide

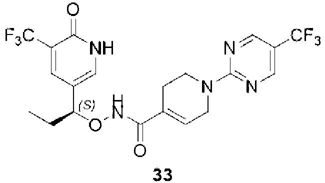

### Step A: Preparation of N-(1-(6-methoxy-5-(trifluoromethyl)pyridin-3-yl)propoxy)-1-(5-(trifluoromethy l)pyrimidin-2-yl)-1,2,3,6-tetrahydropyridin-4-carboxamide)

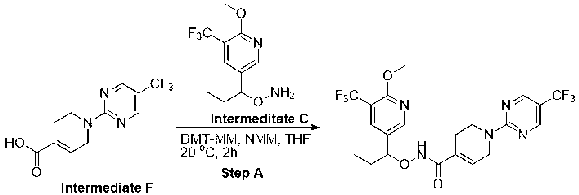

To a solution of 1-(5-(trifluoromethyl)pyrimidin-2-yl)-1,2,3,6-tetrahydropyridin-4-carboxylic acid (23 0 mg) 0.84 mmol (1.0 eq.) in THF (5 mL) was added O-(1-(6-methoxy-5-(trifluoromethyl)pyridin-3-yl)propyl)hydroxylamine (210 mg, 0.84 mmol, 1.0 eq.), 4-(4,6-dimethoxytriazine-2-yl)-4-methylm orpholine hydrochloride (320 mg, 0.93 mmol, 1.1 eq.), and N-methylmorpholine (255 mg, 2.52 m mol, 3.0 eq.) sequentially. The reaction mixture was reacted at 20 °C for 2 h. The reaction mixt ure was quenched with water (30 mL) and extracted with EtOAc (2 × 20 mL). The organic pha ses were combined, washed with saturated brine (2 × 30 mL), dried over anhydrous sodium sulf ate, and concentrated. The crude product was washed with column chromatography (petroleum et her / EtOAc = 7/3; V/V) was purified to obtain N-(1-(6-methoxy-5-(trifluoromethyl)pyridin-3-yl)pr opoxy)-1-(5-(trifluoromethyl)pyrimidin-2-yl)-1,2,3,6-tetrahydropyridine-4-carboxamide (260 mg, color less oil), yield: 67.1%.

LCMS: *(ESI)* [M+H]⁺ = 506.2.

¹H NMR (400 MHz, DMSO-*d₆*) *δ* 11.01 (s, 1H), 8.72 (s, 2H), 8.41 (d, *J =* 1.9 Hz, 1H), 8.09 (d, *J =* 2.0 Hz, 1H), 6.41 (s, 1H), 4.81 (t, *J =* 6.9 Hz, 1H), 4.32 (d, *J =* 2.8 Hz, 2H), 3.98 (s, 3H), 3.92 (t, *J =* 5.7 Hz, 2H), 2.33 - 2.21 (m, 2H), 2.04 - 1.87 (m, 1H), 1.85 - 1.66 (m, 1H), 0.88 (t, *J* = 7.4 Hz, 3H).

### Step B: Preparation of N-(1-(6-oxo-5-(trifluoromethyl)-1,6-dihydropyridin-3-yl)propoxy)-1-(5-(trifluor omethyl)pyrimidin-2-yl)-1,2,3,6-tetrahydropyridin-4-carboxamide)

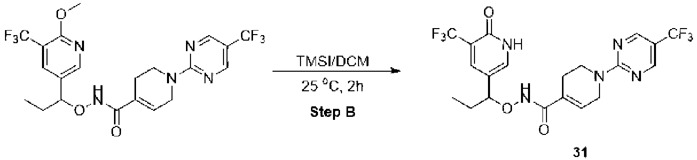

To a solution of N-(1-(6-methoxy-5-(trifluoromethyl)pyridin-3-yl)propoxy)-1-(5-(trifluoromethyl)pyri midin-2-yl)-1,2,3,6-tetrahydropyridin-4-carboxamide (260 mg, 0.52 mmol, 1.0 eq.) in dichlorometha ne (3 mL) was added TMSI (0.3 mL). The reaction mixture was reacted at 25 °C for 2 h. The reaction mixture was quenched with water (10 mL) and extracted with EtOAc (3 × 10 mL). The organic phases were combined, washed with saturated brine (2 × 10 mL), dried over anhydrous sodium sulfate, and concentrated. The crude product was purified by prep-HPLC to give racemi c compound 31, N-(1-(6-oxo-5-(trifluoromethyl)-1,6-dihydropyridin-3-yl)propoxy)-1-(5-(trifluorometh yl)pyrimidin-2-yl)-1,2,3,6-tetrahydropyridin-4-carboxamide (21.68 mg, white solid), yield: 8.3%.

LCMS: *(ESI)* [M+H]⁺ = 492.2.

¹H NMR (400 MHz, DMSO-*d₆*) *δ* 12.32 (s, 1H), 10.97 (s, 1H), 8.75 (s, 2H), 7.94 (s, 1H), 7.69 (s, 1H), 6.45 (s, 1H), 4.56 (t, *J* = 7.0 Hz, 1H), 4.38 - 4.31 (m, 2H), 3.94 (t, *J =* 5.7 Hz, 2H), 2.31 (s, 2H), 1.98 - 1.81 (m, 1H), 1.77 - 1.58 (m, 1H), 0.88 (t, *J =* 7.4 Hz, 3H).

### Step C: Preparation of (R)-N-(1-(6-oxo-5-(trifluoromethyl)-1,6-dihydropyridin-3-yl)propoxy)-1-(5-(trif luoromethyl)pyrimidin-2-yl)-1,2,3,6-tetrahydropyrimidin-4-carboxamide and (S)-N-(1-(6-oxo-5-(trifluor omethyl)-1,6-dihydropyridin-3-yl)propoxy)-1-(5-(trifluoromethyl)pyrimidin-2-y1)-1,2,3,6-tetrahydropyri midin-4-carboxamide)

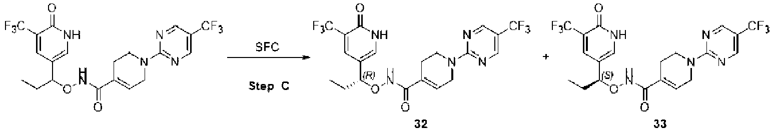

Racemic compound 31, N-(1-(6-oxo-5-(trifluoromethyl)-1,6-dihydropyridin-3-yl)propoxy)-1-(5-(trifluo romethyl)pyrimidin-2-yl)-1,2,3,6-tetrahydropyridin-4-carboxamide (95 mg, 0.193 mmol, 1.0 eq.) was purified by supercritical fluid chromatography (SFC) to give:
Isomer 32, (R)-N-(1-(6-oxo-5-(trifluoromethyl)-1,6-dihydropyridin-3-yl)propoxy)-1-(5-(trifluoromethyl) pyrimidin-2-yl)-1,2,3,6-tetrahydropyridin-4-carboxamide (30.34 mg, white solid), yield: 31.93%.

LCMS: *(ESI)* [M+H]⁺ = 492.2.

¹H NMR (400 MHz, DMSO-*d₆*) *δ* 12.31 (s, 1H), 10.96 (s, 1H), 8.74 (d, *J =* 0.6 Hz, 2H), 7.93 (d, *J =* 1.9 Hz, 1H), 7.68 (s, 1H), 6.45 (s, 1H), 4.56 (t, *J =* 7.0 Hz, 1H), 4.42 - 4.30 (m, 2H), 3.94 (t, *J =* 5.8 Hz, 2H), 2.34 - 2.25 (m, 2H), 1.95 - 1.83 (m, 1H), 1.78 - 1.60 (m, 1H), 0.88 (t, *J=* 7.4 Hz, 3H).

and isomer 33, (S)-N-(1-(6-oxo-5-(trifluoromethyl)-1,6-dihydropyridin-3-yl)propoxy)-1-(5-(trifluorome thyl)pyrimidin-2-yl)-1,2,3,6-tetrahydropyridin-4-carboxamide (34.96 mg, white solid), yield: 36.8%. LCMS: *(ESI)* [M+H]⁺ = 492.2.

¹H NMR (400 MHz, DMSO-*d₆*) *δ* 12.30 (s, 1H), 10.95 (s, 1H), 8.73 (s, 2H), 7.92 (s, 1H), 7.67 (s, 1H), 6.43 (s, 1H), 4.54 (t, *J =* 7.0 Hz, 1H), 4.43 - 4.26 (m, 2H), 3.92 (t, *J =* 5.8 Hz, 2H), 2.32 - 2.24 (m, 2H), 1.93 - 1.81 (m, 1H), 1.74 - 1.59 (m, 1H), 0.86 (t, *J =* 7.4 Hz, 3H).

### Examples 34, 35:

### Synthesis of Compound 34

### (R)-1-(5-cyclopropylpyrimidin-2-yl)-N-(1-(6-oxo-5-(trifluoromethyl)-1,6-dihydropyridin-3-yl)ethoxy)-1, 2,3,6-tetrahydropyridin-4-carboxamide

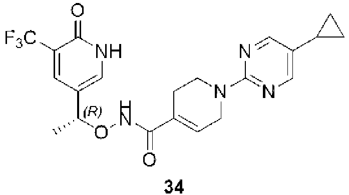

### Synthesis of Compound 35

### (S)-1-(5-cyclopropylpyrimidin-2-yl)-N-(1-(6-oxo-5-(trifluoromethyl)-1,6-dihydropyridin-3-yl)ethoxy)-1, 2,3,6-tetrahydropyridin-4-carboxamide

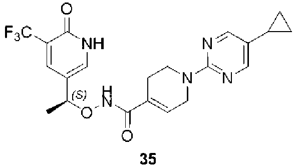

### Step A: Preparation of 1-(5-cyclopropylpyrimidin-2-yl)-1.2,3,6-tetrahydropyridine-4-carboxylic acid

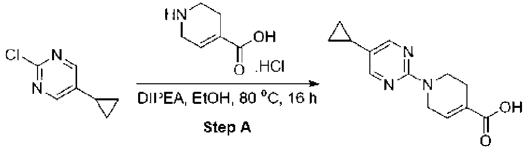

Dissolve 1,2,3,6-tetrahydropyridin-4-carboxylic acid hydrochloride (100 mg, 0.614 mmol, 1.0 eq.) in a mixed solvent of ethanol (6 mL) and water (1 mL), and then was added DIPEA (314 mg, 0.614 mmol, 1.0 eq.) and 2-chloro-5-cyclopropylpyrimidine (95 mg, 0.614 mmol, 1.0 eq.) sequentially. The reaction mixture was reacted at 80 °C for 16 h. The reaction mixture was diluted with water (30 mL), adjusted to pH 6 with dilute hydrochloric acid (1 M), and extracted with EtOAc (2 × 25 mL). The organic phases were combined, dried over anhydrous sodium sulfate, and concentrated to give 1-(5-cyclopropylpyrimidin-2-yl)-1,2,3,6-tetrahydropyridine-4-carboxylic acid (110 mg, white solid), yield: 57.3%.

LCMS: *(ESI)* [M+H]⁺ = 246.0.

### Step B: Preparation of 1-(5-cyclopropylpyrimidin-2-yl)-N-(1-(6-oxo-5-(trifluoromethyl)-1,6-dihydropy ridin-3-yl)ethoxy)-1,2,3,6-tetrahydropyridine-4-carboxamide

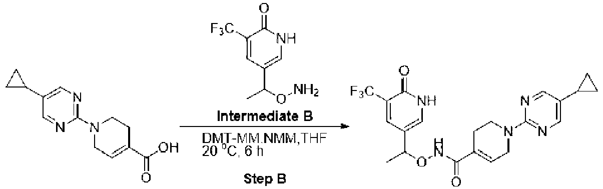

To a solution of 1-(5-cyclopropylpyrimidin-2-yl)-1,2,3,6-tetrahydropyridine-4-carboxylic acid (100 mg, 0.41 mmol, 1.0 eq.) in THF (3 mL) was added 5-(1-(aminooxy)ethyl)-3-(trifluoromethyl)pyrid in-2(1H)-one (108 mg, 0.49 mmol, 1.2 eq.), 4-(4,6-dimethoxytriazine-2-yl)-4-methylmorpholine hyd rochloride (156 mg, 0.54 eq.) and N-methylmorpholine (124 mg, 1.23 mmol, 3.0 eq.) sequentially. The reaction mixture was reacted at 20 °C for 6 h. The reaction mixture was quenched with w ater (30 mL) and extracted with EtOAc (2 × 20 mL). The organic phases were combined, washe d with saturated brine (2 × 30 mL), dried over anhydrous sodium sulfate, and concentrated. The crude product was purified by prep-HPLC to give the racemic compound 1-(5-cyclopropylpyrimi din-2-yl)-N-(1-(6-oxo-5-(trifluoromethyl)-1,6-dihydropyridin-3-yl)ethoxy)-1,2,3,6-tetrahydropyridine-4-c arboxamide (40 mg, white solid), yield: 21.7%.

### Step C: (R)-1-(5-cyclopropylpyrimidin-2-yl)-N-(1-(6-oxo-5-(trifluoromethyl)-1,6-dihydropyridin-3-yl)et hoxy)-1,2,3,6-tetrahydropyridin-4-carboxamide and (S)-1-(5-cyclopropylpyrimidin-2-yl)-N-(1-(6-oxo-5-(trifluoromethyl)-1,6-dihydropyridin-3-yl)ethoxy)-1,2,3,6-tetrahydropyridin-4-carboxamide

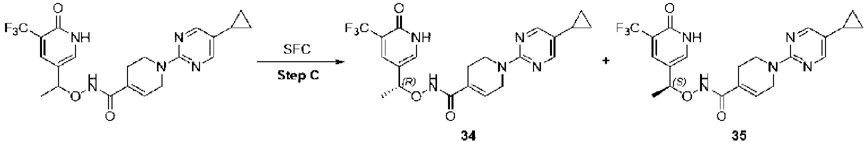

The racemic compound 1-(5-cyclopropylpyrimidin-2-yl)-N-(1-(6-oxo-5-(trifluoromethyl)-1,6-dihydrop yridin-3-yl)ethoxy)-1,2,3,6-tetrahydropyridin-4-carboxamide (40 The following is a white solid (0.0 9 mmol, mg) was purified by SFC to obtain:
Isomer 34,(R)-1-(5-cyclopropylpyrimidin-2-yl)-N-(1-(6-oxo-5-(trifluoromethyl)-1,6-dihydropyridin-3-yl) ethoxy)-1,2,3,6-tetrahydropyridin-4-carboxamide (17.65 mg, white solid), yield: 44.2%;
LCMS: *(ESI)* [M+H]⁺ = 450.2.

¹H NMR (400 MHz, DMSO-*d₆*) *δ* 12.29 (s, 1H), 10.97 (s, 1H), 8.19 (s, 2H), 8.00 (s, 1H), 7.68 (s, 1H), 6.45 (s, 1H), 4.76 (q, *J* = 6.4 Hz, 1H), 4.20 (d, *J* = 2.8 Hz, 2H), 3.79 (t, *J* = 5.6 Hz, 2H), 2.25 (t, 2H), 1.81 - 1.70 (m, 1H), 1.42 (d, *J =* 6.6 Hz, 3H), 0.93 - 0.80 (m, 2H), 0.68 - 0.58 (m, 2H).
and isomer 35,(S)-1-(5-cyclopropylpyrimidin-2-yl)-N-(1-(6-oxo-5-(trifluoromethyl)-1,6-dihydropyridin-3-yl)ethoxy)-1,2,3,6-tetrahydropyridin-4-carboxamide (1.57 mg, white solid), yield: 3.9%.

LCMS: *(ESI)* [M+H]⁺ = 450.2.

¹H NMR (400 MHz, DMSO- *d₆*) *δ* 12.28 (s, 1H), 10.96 (s, 1H), 8.19 (s, 2H), 7.97 (s, 1H), 7.69 (s, 1H), 6.45 (s, 1H), 4.76 (q, *J* = 6.4 Hz, 1H), 4.20 (d, *J* = 2.9 Hz, 2H), 3.79 (t, *J* = 5.6 Hz, 2H), 2.25 (t, 2H), 1.81 - 1.71 (m, 1H), 1.41 (d, *J =* 6.6 Hz, 3H), 0.88 - 0.83 (m, 2H), 0.66 - 0.60 (m, 2H).

### Example 36: Synthesis of Compound 36

### (R)-1-(5-acetamidopyrimidin-2-yl)-N-(1-(6-oxo-5-(trifluoromethyl)-1,6-dihydropyridin-3-yl)ethoxy)-1,2, 3,6-tetrahydropyridine-4-carboxamide

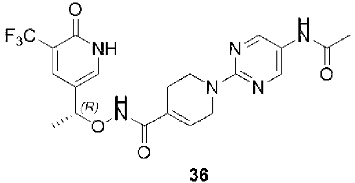

### Step A: Preparation of methyl 1,2,3,6-tetrahydropyridine-4-carboxylic acid hydrochloride

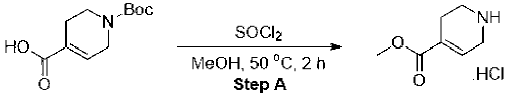

To a solution of 1-(tert-butoxycarbonyl)-1,2,3,6-tetrahydropyridine-4-carboxylic acid (2.00 g, 8.80 mmol, 1.0 eq.) in methanol (50 mL) was added thionyl chloride (5.23 g, 44.00 mmol, 5.0 eq.). The reaction mixture was reacted at 50 °C for 2 h. The reaction mixture was concentrated to give methyl 1,2,3,6-tetrahydropyridine-4-carboxylic acid hydrochloride (1.30 g, white solid), yield: 83.1%.

LCMS: *(ESI)* [M+H]⁺ = 142.2.

### Step B: Preparation of methyl 1-(5-nitropyrimidin-2-yl)-1,2,3,6-tetrahydropyridine-4-carboxylic acid

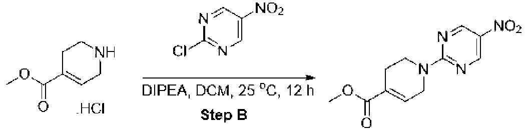

1,2,3,6-Tetrahydropyridine-4-carboxylic acid methyl ester hydrochloride (500 mg, 2.82 mmol, 1.0 eq.) and 2-chloro-5-nitropyrimidine (472 mg, 2.96 mmol, 1.05 eq.) were dissolved in dichloromethane (10 mL), and DIPEA (1.09 g, 8.46 mmol, 3.0 eq.) was added. The reaction mixture was reacted at 25 °C for 12 h. The reaction mixture was diluted with water (30 mL) and extracted with dichloromethane (3 × 40 mL). The organic phases were combined, dried over anhydrous sodium sulfate, and concentrated. The crude product was purified by column chromatography (petroleum ether/EtOAc = 5/1; V/V) to give methyl 1-(5-nitropyrimidin-2-yl)-1,2,3,6-tetrahydropyridine-4-carboxylic acid (0.43 g, yellow solid), yield: 58.1%.

LCMS: *(ESI)* [M+H]⁺ = 265.2.

¹H NMR: (400 MHz, CDCl₃) *δ* 9.03 (s, 2H), 6.96 - 6.90 (m, 1H), 4.48 (q, *J =* 3.2 Hz, 2H), 4.05 (t, *J =* 6.0 Hz, 2H), 3.72 (s, 3H), 2.51 - 2.45 (m, 2H).

### Step C: Preparation of methyl 1-(5-aminopyrimidin-2-yl)-1.2,3,6-tetrahydropyridine-4-carboxylic acid

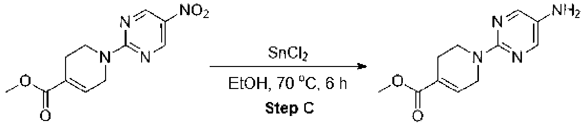

To a solution of Methyl 1-(5-nitropyrimidin-2-yl)-1,2,3,6-tetrahydropyridine-4-carboxylic acid (400 mg, 1.51 mmol, 1.0 eq.) in ethanol (30 mL) was added stannous chloride (1.43 g, 7.56 mmol, 5.0 eq.). The reaction mixture was reacted at 70 °C for 6 h. The reaction mixture was cooled to room temperature and diluted with water (80 mL) and dichloromethane (60 mL). The aqueous phase was adjusted to pH = 8 with saturated sodium bicarbonate solution and extracted with dichloromethane (3 × 60 mL). The organic phases were combined, dried over anhydrous sodium sulfate, and concentrated. The crude product was purified by column chromatography (petroleum ether/EtOAc = 2/1; V/V) to give methyl 1-(5-aminopyrimidin-2-yl)-1,2,3,6-tetrahydropyridine-4-carboxylate (260 mg, pale yellow solid), yield: 74.3%.

LCMS: *(ESI)* [M+H]⁺ = 235.4.

¹H NMR: (400 MHz, MeOD) *δ* 8.28 (s, 2H), 7.02 - 7.00 (m, 1H), 4.40 - 4.34 (m, 2H), 3.94 (t, *J =* 5.6 Hz, 2H), 3.75 (s, 3H), 2.50 - 2.42 (m, 2H).

### Step D: Preparation of methyl 1-(5-acetaminopyrimidin-2-yl)-1,2,3,6-tetrahydropyridine-4-carboxylic acid

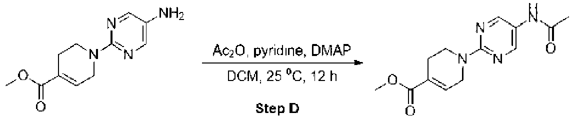

To a solution of Methyl 1-(5-aminopyrimidin-2-yl)-1,2,3,6-tetrahydropyridine-4-carboxylate (220 mg, 0.94 mmol, 1.0 eq.) in dichloromethane (6 mL) was added pyridine (111 mg, 1.41 mmol, 1.5 eq.), 4-dimethylaminopyridine (11 mg, 0.09 mmol, 0.05 eq.), and acetic anhydride (144 mg, 1.41 mmol, 1.5 eq.) sequentially. The reaction mixture was reacted at 25 °C for 12 hours. The reaction mixture was quenched with water (20 mL) and extracted with dichloromethane (3 × 20 mL). The organic phases were combined, dried over anhydrous sodium sulfate, and concentrated. The crude product was purified by column chromatography (petroleum ether / EtOAc = 3 / 1; V / V) to methyl 1-(5-acetaminopyrimidine-2-yl)-1,2,3,6-tetrahydropyridine-4-carboxylic acid (95 mg, yellow solid), yield: 36.5%.

LCMS: *(ESI)* [M+H]⁺ = 277.2.

¹H NMR: (400 MHz, CDCl₃) *δ* 8.43 (s, 2H), 7.08 (s, 1H), 7.03 - 6.99 (m, 1H), 4.38 - 4.33 (m, 2H), 3.92 (t, *J* = 5.6 Hz, 2H), 3.77 (s, 3H), 2.54 - 2.45 (m, 2H), 2.17 (s, 3H).

### Step E: Preparation of 1-(5-acetaminopyrimidin-2-yl)-1,2,3,6-tetrahydropyridine-4-carboxylic acid

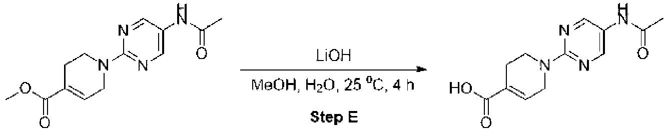

To a solution of Methyl 1-(5-acetaminopyrimidine-2-yl)-1,2,3,6-tetrahydropyridine-4-carboxylic acid (90 mg, 0.33 mmol, 1.0 eq.) in a mixture of methanol (6 mL) and water (2 mL) was added lithium hydroxide (23 mg, 0.97 mmol, 3.0 eq.). The reaction mixture was reacted at 25 °C for 4 hours. The reaction mixture was diluted with water (10 mL), adjusted to pH 6 with dilute hydrochloric acid (1 M), and extracted with a mixture of dichloromethane and methanol (5 × 20 mL, dichloromethane/methanol = 10/1, V/V). The combined organic phases were dried over anhydrous sodium sulfate and concentrated to give 1-(5-acetaminopyrimidin-2-yl)-1,2,3,6-tetrahydropyridine-4-carboxylic acid (45 mg, white solid), yield: 50.0%.

LCMS: *(ESI)* [M+H]⁺ = 263.2.

### Step F: Preparation of (R)-1-(5-acetaminopyrimidin-2-yl)-N-(1-(6-oxo-5-(trifluoromethyl)-1,6-dihydro pyridin-3-yl)ethoxy)-1,2,3,6-tetrahydropyridine-4-carboxamide

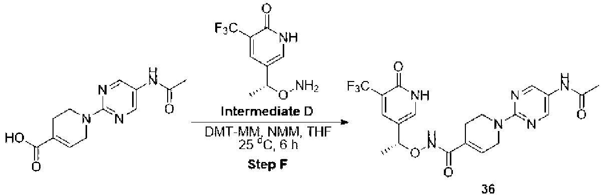

To a solution of 1-(5-acetaminopyrimidin-2-yl)-1,2,3,6-tetrahydropyridine-4-carboxylic acid (39 mg, 0.15 mmol, 1.0 eq.) in THF (3 mL), cooled to 0 °C in an ice bath, was added 4-(4,6-dimetho xytriazine-2-yl)-4-methylmorpholine hydrochloride (57 mg, 0.19 mmol, 1.3 eq.). The reaction mixt ure was reacted at 0 °C for 5 min. N-methylmorpholine (45 mg, 0.45 mmol, 3.0 eq.) and (R)-5-(1-(aminooxy)ethyl)-3-(trifluoromethyl)pyridin-2(1H)-one (50 mg, 0.22 mmol, 1.5 eq.) were added to the reaction mixture. The reaction mixture was reacted at 25 °C for 6 h. The reaction mixture was quenched with saturated ammonium chloride aqueous solution (15 mL) and extracted with EtOAc (3 × 10 mL). The organic phases were combined, dried over anhydrous sodium sulfate, a nd concentrated. The crude product was purified by prep-HPLC to give compound 36,(R)-1-(5-ac etamidopyrimidin-2-yl)-N-(1-(6-oxo-5-(trifluoromethyl)-1,6-dihydropyridin-3-yl)ethoxy)-1,2,3,6-tetrahy dropyridin-4-carboxamide (1.40 mg, white solid), yield: 2.0%.

LCMS: *(ESI)* [M+H]⁺ = 467.2.

¹H NMR: (400 MHz, MeOD) *δ* 8.49 (s, 2H), 8.11 (d, *J =* 2.0 Hz, 1H), 7.68 (d, *J =* 2.4 Hz, 1H), 6.56 - 6.47 (m, 1H), 4.84 - 4.80 (m, 1H), 4.31 - 4.26 (m, 2H), 3.90 (t, *J =* 5.6 Hz, 2H), 2.40 - 2.31 (m, 2H), 2.11 (s, 3H), 1.54 (d, *J =* 6.8 Hz, 3H).

Examples 37, 38:

### Synthesis of Compound 37

### (R)-N-(1-(6-oxo-5-(trifluoromethyl)-1,6-dihydropyridin-3-yl)ethoxy)-5'-(trifluoromethyl)-3,6-dihydro-2H -[1,2'-bipyridine]-4-carboxamide

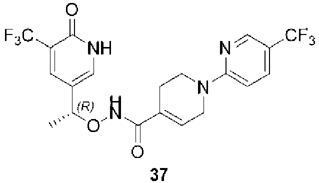

### Synthesis of Compound 38

### (S)-N-(1-(6-oxo-5-(trifluoromethyl)-1,6-dihydropyridin-3-yl)ethoxy)-5'-(trifluoromethyl)-3,6-dihydro-2H -[1,2'-bipyridine]-4-carboxamide

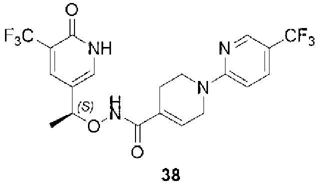

### Step A: Preparation of 5'-(trifluoromethyl)-3,6-dihydro-2H-[1,2'-bipyridine]-4-carboxylic acid

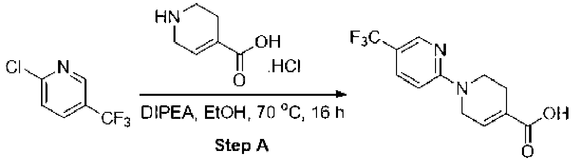

Dissolve 1,2,3,6-tetrahydropyridine-4-carboxylic acid hydrochloride (80 mg, 0.491 mmol, 1.0 eq.) i n a mixture of ethanol (1 mL) and water (0.5 mL), then was added DIPEA (254 g), 2-chloro-5-(trifluoromethyl)pyridine (94 mg, 0.52 mmol, 1.05 eq.) sequentially. The mixture was reacted at 7 0 °C for 16 h. The reaction mixture was diluted with water (20 mL) and extracted with EtOAc (2 × 20 mL). The organic phases were combined, dried over anhydrous sodium sulfate, and conc entrated to give 5'-(trifluoromethyl)-3,6-dihydro-2H-[1,2'-bipyridine]-4-carboxylic acid (70 mg, whit e solid), yield: 58.3%.

LCMS: *(ESI)* [M+H]⁺ = 273.2.

¹H NMR (400 MHz, DMSO-*d₆*) *δ* 8.49 - 8.38 (m, 1H), 7.82 (dd, *J =* 9.1, 2.4 Hz, 1H), 6.95 (d, *J =* 9.2 Hz, 1H), 6.93 - 6.90 (m, 1H), 4.29 - 4.19 (m, 2H), 3.80 (t, *J =* 5.7 Hz, 2H), 2.40 - 2.33 (m, 2H).

### Step B: Preparation of N-(1-(6-oxo-5-(trifluoromethyl)- 1,6-dihydropyridin-3-yl)ethoxy)-5'-(trifluorome thyl)-3,6-dihydro-2H-[1,2'-bipyridine]-4-carboxamide

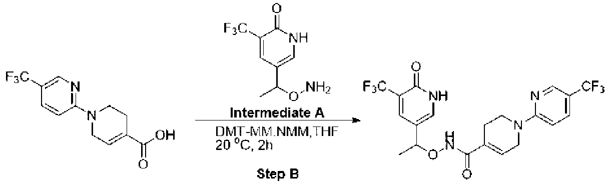

Dissolve 5'-(trifluoromethyl)-3,6-dihydro-2H-[1,2'-bipyridine]-4-carboxylic acid (70 mg, 0.258 mmol, 1.0 eq.) in THF (2 mL), and then was added 5-(1-(aminooxy)ethyl)-3-(trifluoromethyl)pyridin-2(1 H)-one (69 mg, 0.31 mmol, 1.2 eq.) 4-(4,6-dimethoxytriazine-2-yl)-4-methylmorpholine hydrochlori de (99 mg, 0.336 eq.). and N-methylmorpholine (79 mg, 0.78 mmol, 3.0 eq.). The reaction mixt ure was reacted at 20 °C for 2 h. The reaction mixture was quenched with water (30 mL) and extracted with EtOAc (2 × 20 mL). The organic phases were combined, washed with saturated b rine (2 × 30 mL), dried over anhydrous sodium sulfate, and concentrated. The crude product was purified by prep-HPLC to N-(1-(6-oxo-5-(trifluoromethyl)-1,6-dihydropyridin-3-yl)ethoxy)-5'-(trifluo romethyl)-3,6-dihydro-2H-[1,2'-bipyridine]-4-carboxamide (90 mg, white solid), yield: 73.8%. Step C: Preparation of (R)-N-(1-(6-oxo-5-(trifluoromethyl)-1.6-dihydropyridin-3-yl)ethoxy)-5'-(trifluor omethyl)-3,6-dihydro-2H-[1,2'-bipyridinel-4-carboxamide and (S)-N-(1-(6-oxo-5-(trifluoromethyl)-1,6-dihydropyridin-3-yl)ethoxy)-5'-(trifluoromethyl)-3,6-dihydro-2H-[1,2'-bipyridine]-4-carboxamide)

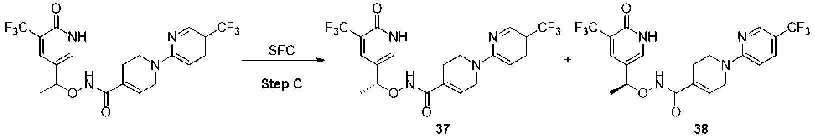

Preparation of N-(1-(6-oxo-5-(trifluoromethyl)-1,6-dihydropyridin-3-yl)ethoxy)-5'-(trifluoromethyl)-3,6 -dihydro-2H-[1,2'-bipyridine]-4-carboxamide (90 mg, white solid). The following isomeric compoun ds were purified by SFC:
Isomer 37, (R)-N-(1-(6-oxo-5-(trifluoromethyl)-1,6-dihydropyridin-3-yl)ethoxy)-5'-(trifluoromethyl)-3,6 -dihydro-2H-[1,2'-bipyridine]-4-carboxamide (18.43 mg, white solid), yield: 20.4%.

LCMS: *(ESI)* [M+H]⁺ = 477.2.

¹H NMR (400 MHz, DMSO-*d₆*) *δ* 12.31 (s, 1H), 11.02 (s, 1H), 8.42 (s, 1H), 8.01 (d, 1H), 7.82 (dd, *J =* 9.1, 2.3 Hz, 1H), 7.69 (s, 1H), 6.95 (d, *J* = 9.1 Hz, 1H), 6.47 (s, 1H), 4.82 - 4.72 (m, 1H), 4.16 (d, *J* = 2.8 Hz, 2H), 3.77 (t, *J* = 5.6 Hz, 2H), 2.32 (t, *J* = 10.1 Hz, 2H), 1.42 (d, *J =* 6.6 Hz, 3H).

And isomer 38, (S)-N-(1-(6-oxo-5-(trifluoromethyl)-1,6-dihydropyridin-3-yl)ethoxy)-5'-(trifluoromethy 1)-3,6-dihydro-2H-[1,2'-bipyridine]-4-carboxamide (0.9 mg, white solid), yield: 1.0%.

LCMS: *(ESI)* [M+H]⁺ = 477.2.

¹H NMR (400 MHz, DMSO-*d₆*) *δ* 12.31 (s, 1H), 11.02 (s, 1H), 8.43 (s, 1H), 8.00 (d, 1H), 7.82 (dd, *J =* 9.1, 2.5 Hz, 1H), 7.69 (s, 1H), 6.95 (d ,*J* = 9.1 Hz, 1H), 6.47 (s, 1H), 4.77 (q, *J =* 6.7 Hz, 1H), 4.15 (d, *J =* 2.6 Hz, 2H), 3.77 (t, *J =* 5.6 Hz, 2H), 2.30 (t, 2H), 1.42 (d, *J* = 6.5 Hz, 3H).

### Examples 39, 40:

### Synthesis of Compound 39

### (R)-5-methyl-N-(1-(6-oxo-5-(trifluoromethyl)-1,6-dihydropyridin-3-yl)ethoxy)-1-(5-(trifluoromethyl)pyri midin-2-yl)-1,2,3,6-tetrahydropyridin-4-carboxamide

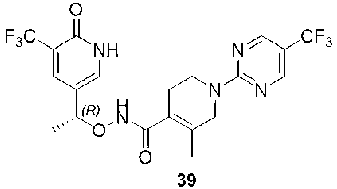

### Synthesis of Compound 40

### 5-methyl-N-((R)-1-(6-oxo-5-(trifluoromethyl)-1,6-dihydropyridin-3-yl)ethoxy)-1-(5-(trifluoromethyl)pyri midin-2-yl)-1,2,3,4-tetrahydropyridin-4-carboxamide

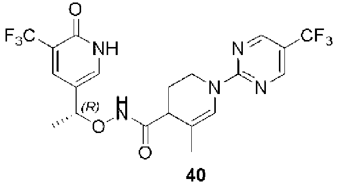

### Step A: Preparation of ethyl 3-oxo-1-(5-(trifluoromethyl)pyrimidin-2-yl)piperidine-4-carboxylic acid

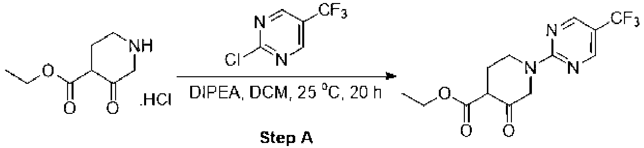

To a solution of ethyl 3-oxopiperidine-4-carboxylic acid hydrochloride (4.00 g, 19.26 mmol, 1.0 e q.) in dichloromethane (20 mL) was added 2-chloro-5-trifluoromethylpyrimidine (3.52 g, 19.26 m mol, 1.0 eq.) and DIPEA (12.45 g, 96.30 mmol, 5.0 eq.). The reaction mixture was reacted at 2 5 °C for 20 h. The reaction mixture was diluted with water (400 mL) and extracted with EtOAc (2 × 350 mL). The organic layer was washed with saturated brine (800 mL), dried over anhydr ous sodium sulfate, and concentrated. The crude product was purified by column chromatography (petroleum ether / EtOAc = 1 / 1; V / V) to give ethyl 3-oxo-1-(5-(trifluoromethyl)pyrimidin-2-yl)piperidine-4-carboxylate (5.30 g, white solid), yield: 86.7%.

¹H NMR: (400 MHz, DMSO-*d₆*) *δ* 11.97 (s, 1H), 8.76 (d, *J* = 0.4 Hz, 2H), 4.44 - 4.39 (m, 2 H), 4.22 (q, *J* = 7.2 Hz, 2H), 3.97 (t, *J* = 5.6 Hz, 2H), 2.39 - 2.32 (m, 2H), 1.25 (t, *J* = 7.2 Hz, 3H).

### Step B: Preparation of ethyl 1-(5-(trifluoromethyl)pyrimidin-2-yl)-5-(((trifluoromethyl)sulfonyl)oxy)-1,2,3,6-tetrahydropyridin-4-carboxylic acid

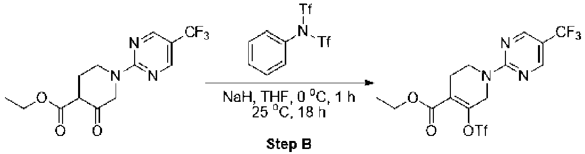

To a solution of Ethyl 3-oxo-1-(5-(trifluoromethyl)pyrimidin-2-yl)piperidine-4-carboxylate (3.00 g, 9.46 mmol, 1.0 eq.) in THF (90 mL), cooled to 0 °C in an ice bath under nitrogen protection, was added NaH (60%, 454 mg, 11.35 mmol, 1.2 eq.). The reaction mixture was reacted at 0 °C for 1 hour. N-phenylbis(trifluoromethanesulfonyl)imide (3.72 g, 10.41 mmol, 1.1 eq.) was added to the reaction mixture. The reaction mixture was reacted at 25 °C for 18 hours. The reaction mixture was quenched with water (400 mL) and extracted with EtOAc (2 × 350 mL). The organ ic phases were combined, washed with saturated brine (800 mL), dried over anhydrous sodium s ulfate, and concentrated. The crude product was purified by column chromatography (petroleum e ther / EtOAc = 10 / 1; V / V) to give ethyl 1-(5-(trifluoromethyl)pyrimidin-2-yl)-5-(((trifluoromet hyl)sulfonyl)oxy)-1,2,3,6-tetrahydropyridine-4-carboxylic acid (3.50 g, yellow oil), yield: 82.4%.

LCMS: *(ESI)* [M+H]⁺ = 450.0.

¹H NMR: (400 MHz, CDCl₃) *δ* 8.48 (d, *J =* 0.4 Hz, 2H), 4.48 (t, *J =* 2.8 Hz, 2H), 4.25 (q, *J =* 7.2 Hz, 2H), 3.98 (t, *J* = 5.6 Hz, 2H), 2.67 - 2.59 (m, 2H), 1.27 (t, *J =* 7.2 Hz, 3H).

### Step C: Preparation of ethyl 5-methyl-1-(5-(trifluoromethyl)pyrimidin-2-yl)-1,2,3,6-tetrahydropyridine -4-carboxylic acid

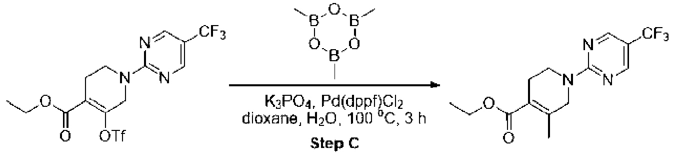

To a solution of Ethyl 1-(5-(trifluoromethyl)pyrimidin-2-yl)-5-(((trifluoromethyl)sulfonyl)oxy)-1,2,3,6 -tetrahydropyridine-4-carboxylic acid (3.50 g, 7.79 mmol, 1.0 eq.) in a mixed solvent of dioxane (80 mL) and water (20 mL),was added potassium phosphate (4.96 g, 23.37 mmol, 3.0 eq.), 1,1'-bis(diphenylphosphine)ferrocene palladium(II) dichloride (570 mg, 0.78 mmol, 0.1 eq.), and trimet hylcycloborone (1.08 g, 8.57 mmol, 1.1 eq.). The reaction mixture was stirred at 100 °C for 3 h ours. The reaction mixture was cooled to room temperature and quenched with water (300 mL), then extracted with EtOAc (2 × 240 mL). The combined organic phases were washed with satur ated brine (500 mL), dried over anhydrous sodium sulfate, and concentrated. The crude product was purified by column chromatography (petroleum ether / EtOAc = 10 / 1; V / V) to ethyl 5-methyl-1-(5-(trifluoromethyl)pyrimidin-2-yl)-1,2,3,6-tetrahydropyridine-4-carboxylic acid (1.60 g, yell ow solid), yield: 65.2%.

LCMS: *(ESI)* [M+H]⁺ = 316.0.

¹H NMR: (400 MHz, DMSO-*d₆*) *δ* 8.73 (d, *J =* 0.8 Hz, 2H), 4.34 - 4.27 (m, 2H), 4.15 (q, *J =* 7.2 Hz, 2H), 3.93 (t, *J* = 5.6 Hz, 2H), 2.45 - 2.37 (m, 2H), 2.05 (s, 3H), 1.23 (t, *J =* 7.2 Hz, 3H).

### Step D: Preparation of ethyl 5-methyl-1-(5-(trifluoromethyl)pyrimidin-2-yl)-1,2,3,6-tetrahydropyridine -4-carboxylic acid

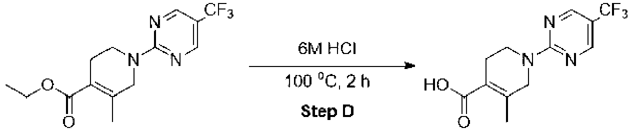

Ethyl 5-methyl-1-(5-(trifluoromethyl)pyrimidin-2-yl)-1,2,3,6-tetrahydropyridine-4-carboxylic acid (400 mg, 1.27 mmol, 1.0 eq.) was dissolved in water (4 mL), and concentrated hydrochloric acid (6 M, 4 mL) was added. The reaction mixture was reacted at 100 °C for 2 h. The reaction mixture was cooled to room temperature and diluted with water (40 mL), and extracted with EtOAc (2 × 35 mL). The organic phases were combined, washed with saturated brine (80 mL), dried over anhydrous sodium sulfate, and concentrated to give 5-methyl-1-(5-(trifluoromethyl)pyrimidin-2-yl)-1, 2,3,6-tetrahydropyridine-4-carboxylic acid (360 mg, yellow solid), yield: 98.8%.

LCMS: *(ESI)* [M+H]⁺ = 288.0.

¹H NMR: (400 MHz, DMSO-*d*₆) *δ* 12.44 (s, 1H), 8.73 (d, *J =* 0.8 Hz, 2H), 4.28 (s, 2H), 3.92 (t, *J =* 5.6 Hz, 2H), 2.43 - 2.34 (m, 2H), 2.05 (s, 3H).

### Step E: (R)-5-methyl-N-(1-(6-oxo-5-(trifluoromethyl)-1,6-dihydropyridin-3-yl)ethoxy)-1-(5-(trifluorom ethyl)pyrimidin-2-yl)-1,2,3,6-tetrahydropyridin-4-carboxamide

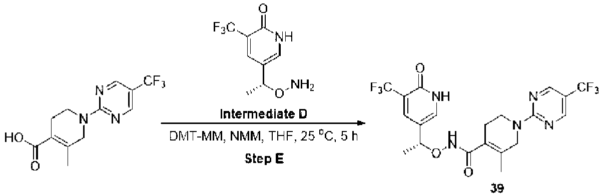

To a solution of 5-Methyl-1-(5-(trifluoromethyl)pyrimidin-2-yl)-1,2,3,6-tetrahydropyridine-4-carboxylic acid (259 mg, 0.90 mmol, 1.0 eq.) in THF (10 mL), cooled to 0 °C in an ice bath was added 4-(4,6-dimethoxytriazine-2-yl)-4-methylmorpholine hydrochloride (324 mg, 1.17 mmol, 1.3 eq.). The reaction mixture was reacted at 0 °C for 20 min. N-methylmorpholine (273 mg, 2.70 mmol, 3.0 eq.) and (R)-5-(1-(aminooxy)ethyl)-3-(trifluoromethyl)pyridine-2(1H)-one (200 mg, 0.90 mmol, 1.0 eq.) were added to the reaction mixture. The reaction mixture was reacted at 25 °C for 5 hours. The reaction mixture was quenched with water (80 mL) and extracted with EtOAc (2 × 70 mL). The organic phases were combined, washed with saturated brine (200 mL), dried over anhydrous sodium sulfate, and concentrated. The crude product was separated by column chromatography (petroleum ether/EtOAc = 1/1; V/V) and further purified by prep-HPLC to give compound 39, (R)-5-methyl-N-(1-(6-oxo-5-(trifluoromethyl)-1,6-dihydropyridin-3-yl)ethoxy)-1-(5-(trifluoromethyl)pyri midin-2-yl)-1,2,3,6-tetrahydropyridin-4-carboxamide (50.84 mg, white solid), yield: 11.5%.

LCMS: *(ESI)* [M+H]⁺ = 492.2.

¹H NMR: (400 MHz, DMSO-*d*₆) *δ* 12.32 (s, 1H), 10.88 (s, 1H), 8.72 (s, 2H), 7.98 (d, *J =* 1.6 Hz, 1H), 7.68 (d, *J* = 2.0 Hz, 1H), 4.82 (q, *J =* 6.4 Hz, 1H), 4.17 (s, 2H), 3.97 - 3.85 (m, 2H), 2.28 - 2.16 (m, 2H), 1.65 (s, 3H), 1.41 (d, *J =* 6.8 Hz, 3H).

### Step F: Preparation of 5-methyl-1-(5-(trifluoromethyl)pyrimidin-2-yl)-1,2,3,4-tetrahydropyridin-4-carb oxylic acid

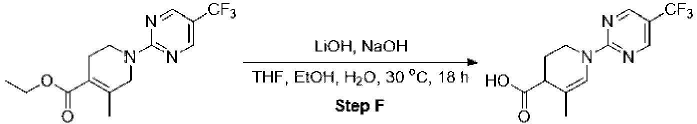

Ethyl 5-methyl-1-(5-(trifluoromethyl)pyrimidin-2-yl)-1,2,3,6-tetrahydropyridin-4-carboxylic acid (800 mg, 2.54 mmol, 1.0 eq.) was dissolved in a mixed solvent of THF (4 mL), water (4 mL), and ethanol (2 mL), lithium hydroxide (533 mg, 12.70 mmol, 5.0 eq) and sodium hydroxide (254 mg, 6.35 mmol, 2.5 eq.) were added. The reaction mixture was reacted at 30 °C for 18 h. The reac tion mixture was acidified with dilute hydrochloric acid (1 M, 30 mL) and extracted with EtOAc (2 × 28 mL). The combined organic phases were washed with saturated brine (60 mL), dried o ver anhydrous sodium sulfate, and concentrated to give 5-methyl-1-(5-(trifluoromethyl)pyrimidin-2-yl)-1,2,3,4-tetrahydropyridine-4-carboxylic acid (702 mg, yellow solid), yield: 96.3%.

LCMS: *(ESI)* [M+H]⁺ = 288.0.

¹H NMR: (400 MHz, DMSO-*d₆*) *δ* 12.51 (s, 1H), 8.81 (s, 2H), 7.42 (s, 1H), 4.19 (dt, *J =* 13.2, 4.4 Hz, 1H), 3.69 - 3.55 (m, 1H), 3.06 (t, *J* = 4.4 Hz, 1H), 2.22 - 2.11 (m, 1H), 2.02 - 1.88 (m, 1H), 1.84 (s, 3H).

### Step G: Preparation of 5-methyl-N-((R)-1-(6-oxo-5-(trifluoromethyl)-1,6-dihydropyridin-3-yl)ethoxy)-1-(5-(trifluoromethyl)pyrimidin-2-yl)-1,2,3,4-tetrahydropyridine-4-carboxamide

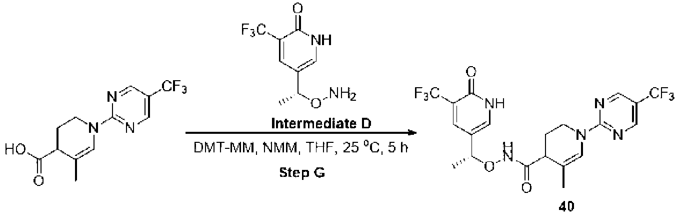

To a solution of 5-Methyl-1-(5-(trifluoromethyl)pyrimidin-2-yl)-1,2,3,4-tetrahydropyridine-4-carboxylic acid (388 mg, 1.35 mmol, 1.0 eq.) in THF (15 mL), cooled to 0 °C in an ice bath was added 4-(4,6-dimethoxytriazine-2-yl)-4-methylmorpholine hydrochloride (487 mg, 1.76 mmol, 1.3 eq.). The reaction mixture was reacted at 0 °C for 20 min. N-methylmorpholine (410 mg, 4.05 mmol, 3.0 eq.) and (R)-5-(1-(aminooxy)ethyl)-3-(trifluoromethyl)pyridine-2(1H)-one (300 mg, 1.35 mmol, 1.0 eq.) were added to the reaction mixture. The reaction mixture was reacted at 25 °C for 5 hours. The reaction mixture was quenched with water (80 mL) and extracted with EtOAc (2 × 70 mL). The organic phases were combined, washed with saturated brine (150 mL), dried over anhydrous sodium sulfate, and concentrated. The crude product was separated by column chromatography (petroleum ether/EtOAc = 1/1; V/V) and further purified by prep-HPLC to give compound 40,5-methyl-N-((R)-1-(6-oxo-5-(trifluoromethyl)-1,6-dihydropyridin-3-yl)ethoxy)-1-(5-(trifluoromethyl) pyrimidin-2-yl)-1,2,3,4-tetrahydropyridin-4-carboxamide (52.13 mg, white solid), yield: 7.9%.

LCMS: *(ESI)* [M+H]⁺ = 492.2.

¹H NMR: (400 MHz, DMSO-*d*₆) *δ* 12.35 (s, 1H), 11.01 (d, *J =* 3.2 Hz, 1H), 8.79 (s, 2H), 7.99 (dd, *J =* 6.4, 2.0 Hz, 1H), 7.70 (dd, *J* = 7.2, 2.0 Hz, 1H), 7.42 - 7.34 (m, 1H), 4.80 (q, *J =* 6.4 Hz, 1H), 4.04 - 3.92 (m, 1H), 3.92 - 3.75 (m, 1H), 2.78 - 2.69 (m, 1H), 1.95 - 1.78 (m, 2H), 1.60 (d, *J =* 24.0 Hz, 3H), 1.42 (dd, *J* = 6.4, 1.6 Hz, 3H).

### Example 41: Synthesis of Compound 41

### (R)-N-(1-(6-oxo-5-(trifluoromethyl)-1,6-dihydropyridin-3-yl)ethoxy)-1-(7-(trifluoromethyl)-1H-imidazo[ 4,5-c]pyridin-4-yl)-1,2,3,6-tetrahydropyridine-4-carboxamide

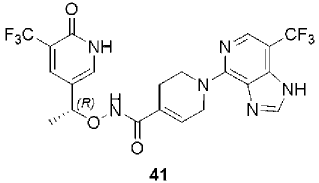

### Step A: Preparation of methyl 1-(7-bromo-1-((2-(trimethylsilyl)ethoxy)methyl)-1H-imidazo[4,5-c]pyri din-4-yl)-1,2,3,6-tetrahydropyridine-4-carboxylic acid

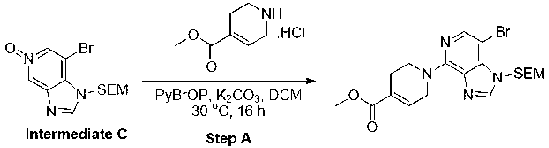

7-Bromo-1-((2-(trimethylsilyl)ethoxy)methyl)-1H-imidazo[4,5-c]pyridine 5-oxide (800 mg, 2.32 mmo 1, 1.0 eq.) was dissolved in dichloromethane (20 mL), followed by the addition of methyl 1,2,3,6 -tetrahydropyridine-4-carboxylic acid hydrochloride (655 mg, 4.64 mmol, 2.0 eq.), tripyrrolylphosp honium hexafluorophosphate bromide (1.41 g, 3.02 mmol, 1.3 eq.), and potassium carbonate (962 mg, 6.96 mmol, 3.0 eq.). The reaction mixture was reacted at 30 °C for 16 h. The reaction mi xture was quenched with water (100 mL) and extracted with EtOAc (2 × 100 mL). The organic phases were combined, washed with saturated brine (200 mL), dried over anhydrous sodium sulf ate, and concentrated. The crude product was purified by column chromatography (petroleum ethe r/EtOAc = 4/1;V/V) to methyl 1-(7-bromo-1-((2-(trimethylsilyl)ethoxy)methyl)-1H-imidazo[4,5-c]pyr idin-4-yl)-1,2,3,6-tetrahydropyridine-4-carboxylic acid (390 mg, yellow solid), yield: 35.9%.

¹H NMR: (400 MHz, DMSO-*d*₆) *δ* 8.49 (s, 1H), 8.03 (s, 1H), 7.17 - 7.07 (m, 1H), 5.86 (s, 2H), 4.76 - 4.70 (m, 2H), 4.32 (t, *J* = 5.6 Hz, 2H), 3.77 (s, 3H), 3.64 - 3.59 (m, 2H), 2.53 - 2.47 (m, 2H), 0.94 - 0.89 (m, 2H), 0.00 (s, 9H).

### Step B: Preparation of methyl 1-(7-(trifluoromethyl)-1-((2-(trimethylsilyl)ethoxy)methyl)-1H-imidazo [4,5-c]pyridin-4-yl)-1,2,3,6-tetrahydropyridine-4-carboxylic acid

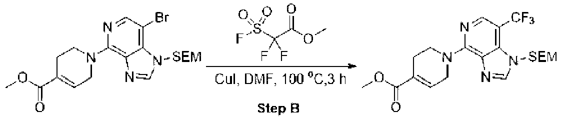

To a solution of Methyl 1-(7-bromo-1-((2-(trimethylsilyl)ethoxy)methyl)-1H-imidazo[4,5-c]pyridin-4-yl)-1,2,3,6-tetrahydropyridine-4-carboxylic acid (340 mg, 0.73 mmol, 1.0 eq.) in N,N-dimethylform amide (5 mL) was added cuprous iodide (70 mg, 0.37 mmol, 0.5 eq.) and methyl fluorosulfonyl difluoroacetate (561 mg, 2.92 mmol, 4.0 eq.). Under nitrogen protection, the reaction mixture w as reacted at 100 °C for 3 hours. The reaction mixture was cooled to room temperature and dilu ted with water (50 mL), extracted with EtOAc (2 × 40 mL). The organic phases were combined, washed with saturated brine (100 mL), dried over anhydrous sodium sulfate, and concentrated. T he crude product was purified by column chromatography (petroleum ether/EtOAc = 4/1; V/V) to obtain methyl 1-(7-(trifluoromethyl)-1-((2-(trimethylsilyl)ethoxy)methyl)-1H-imidazo[4,5-c]pyridin-4-yl)-1,2,3,6-tetrahydropyridine-4-carboxylic acid (75 mg, yellow solid), yield: 22.6%.

LCMS: *(ESI)* [M+H]⁺ = 457.0.

¹H NMR: (400 MHz, DMSO-*d*₆) *δ* 8.57 (s, 1H), 8.30 (s, 1H), 7.14 - 7.09 (m, 1H), 5.69 (s, 2H), 4.96 - 4.86 (m, 2H), 4.47 (t, *J* = 5.6 Hz, 2H), 3.77 (s, 3H), 3.62 - 3.57 (m, 2H), 2.55 - 2.50 (m, 2H), 0.92 - 0.87 (m, 2H), 0.00 (s, 9H).

### Step C: Preparation of methyl 1-(7-(trifluoromethyl)-1-((2-(trimethylsilyl)ethoxy)methyl)-1H-imidazo [4,5-c]pyridin-4-yl)-1,2,3,6-tetrahydropyridine-4-carboxylic acid

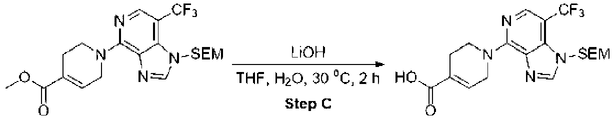

To a solution of methyl 1-(7-(trifluoromethyl)-1-((2-(trimethylsilyl)ethoxy)methyl)-1H-imidazo[4,5-c] pyridin-4-yl)-1,2,3,6-tetrahydropyridine-4-carboxylic acid (70 mg, 0.15 mmol, 1.0 mg) in a mixture of THF (6 mL) and water (3 mL) was added lithium hydroxide (38 mg, 0.90 mmol, 6.0 eq.).

The reaction mixture was reacted at 30 °C for 2 h. The reaction mixture was diluted with water (30 mL), adjusted to pH 6 with dilute hydrochloric acid (1 M), and extracted with EtOAc (2 × 30 mL). The organic phases were combined, washed with saturated brine (80 mL), dried over a nhydrous sodium sulfate, and concentrated to give 1-(7-(trifluoromethyl)-1-((2-(trimethylsilyl)ethoxy) methyl)-1H-imidazo[4,5-c]pyridin-4-yl)-1,2,3,6-tetrahydropyridine-4-carboxylic acid (75 mg, crude, y ellow solid).

LCMS: (*ESI)* [M+H]⁺ =443.0.

### Step D: Preparation of (R)-N-(1-(6-oxo-5-(trifluoromethyl)-1,6-dihydropyridin-3-yl)ethoxy)-1-(7-(trifl uoromethyl)-1-((2-(trimethylsilyl)ethoxymethyl)-1H-imidazo[4,5-c]pyridin-4-yl)-1,2,3,6-tetrahydropyridi n-4-carboxamide)

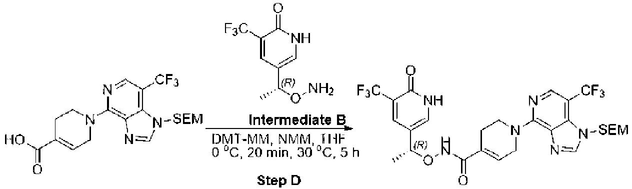

To a solution of 1-(7-(trifluoromethyl)-1-((2-(trimethylsilyl)ethoxy)methyl)-1H-imidazo[4,5-c]pyridin-4-yl)-1,2,3,6-tetrahydropyridine-4-carboxylic acid (70 mg, 0.16 mmol, 1.0 eq.) in THF (5 mL), co oled to 0 °C in an ice bath, was added 4-(4,6-dimethoxytriazine-2-yl)-4-methylmorpholine hydroch loride (58 mg, 0.21 mmol, 1.3 eq.). The reaction mixture was reacted at 0 °C for 20 min. N-me thylmorpholine (49 mg, 0.48 mmol, 3.0 eq.) and (R)-5-(1-(aminooxy)ethyl)-3-(trifluoromethyl)pyrid in-2(1H)-one (36 mg, 0.16 mmol, 1.0 eq.) were added to the reaction mixture. The reaction mixt ure was reacted at 30 °C for 5 h. The reaction mixture was quenched with water (50 mL) and extracted with EtOAc (2 × 50 mL). The organic phases were combined, washed with saturated b rine (120 mL), dried over anhydrous sodium sulfate, and concentrated. The crude product was pu rified by column chromatography (petroleum ether/EtOAc = 1/1; V/V) to give (R)-N-(1-(6-oxo-5-(trifluoromethyl)-1,6-dihydropyridin-3-yl)ethoxy)-1-(7-(trifluoromethyl)-1-((2-(trimethylsilyl)ethoxymet hyl)-1H-imidazo[4,5-c]pyridin-4-yl)-1,2,3,6-tetrahydropyridin-4-carboxamide (55 mg, white solid), yi eld: 53.5%.

LCMS: *(ESI)* [M+H]⁺ = 647.2.

¹H NMR: (400 MHz, DMSO-*d*₆) *δ* 12.37 (s, 1H), 11.04 (s, 1H), 8.54 (s, 1H), 8.28 (s, 1H), 8.08 (s, 1H), 7.75 (s, 1H), 6.62 - 6.54 (m, 1H), 5.67 (s, 2H), 4.91 - 4.75 (m, 3H), 4.49 - 4.39 (m, 2H), 3.59 (t, *J =* 8.0 Hz, 2H), 2.47 - 2.39 (m, 2H), 1.49 (d, *J =* 6.4 Hz, 3H), 0.90 (t, *J =* 8.0 Hz, 2H), 0.00 (s, 9H).

### Step E: Preparation of (R)-N-(1-(6-oxo-5-(trifluoromethyl)-1,6-dihydropyridin-3-yl)ethoxy)-1-(7-(triflu oromethyl)-1H-imidazol4,5-clpyridin-4-yl)-1,2,3,6-tetrahydropyridin-4-carboxamide)

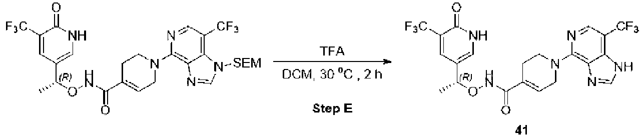

To a solution of (R)-N-(1-(6-oxo-5-(trifluoromethyl)-1,6-dihydropyridin-3-yl)ethoxy)-1-(7-(trifluorome thyl)-1-((2-(trimethylsilyl)ethoxymethyl)-1H-imidazo[4,5-c]pyridin-4-yl)-1,2,3,6-tetrahydropyridin-4-car boxamide (64 mg, 0.10 mmol, 1.0 eq.) in dichloromethane (10 mL) was added trifluoroacetic aci d (2 mL). The reaction mixture was reacted at 30 °C for 2 h. The reaction was quenched with saturated sodium carbonate solution (50 mL), and extracted with dichloromethane (2 × 50 mL). The organic phases were combined, washed with saturated brine (120 mL), dried over anhydrous sodium sulfate, and concentrated. The crude product was washed with column chromatography (petroleum ether / EtOAc = 1/1; V/V) was purified and further purified by prep-HPLC to obtain compound 41, (R)-N-(1-(6-oxo-5-(trifluoromethyl)-1,6-dihydropyridin-3-yl)ethoxy)-1-(7-(trifluoromet hyl)-1H-imidazo[4,5-c]pyridin-4-yl)-1,2,3,6-tetrahydropyridine-4-carboxamide (10.38 mg, white solid), yield: 20.3%.

LCMS: *(ESI)* [M+H]⁺ = 517.2.

¹H NMR: (400 MHz, DMSO-*d*₆) *δ* 13.20 (s, 1H), 12.30 (s, 1H), 10.98 (s, 1H), 8.28 - 8.25 (m, 1H), 8.15 - 8.11 (m, 1H), 8.01 (d, *J =* 2.0 Hz, 1H), 7.69 (s, 1H), 6.55 - 6.48 (m, 1H), 4.90 - 4.70 (m, 3H), 4.38 (t, *J=* 5.6 Hz, 2H), 2.39 - 2.33 (m, 2H), 1.43 (d, *J =* 6.4 Hz, 3H).

### Example 42: Synthesis of Compound 42

### (R)-1-(5-cyanopyrimidin-2-yl)-N-(1-(6-oxo-5-(trifluoromethyl)-1,6-dihydropyridin-3-yl)ethoxy)-1,2,3,6-t etrahydropyridin-4-carboxamide

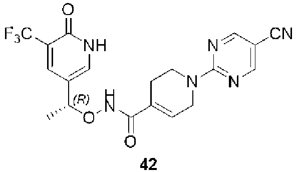

### Step A: Preparation of 1-(5-cyanopyrimidin-2-yl)-1,2,3,6-tetrahydropyridine-4-carboxylic acid

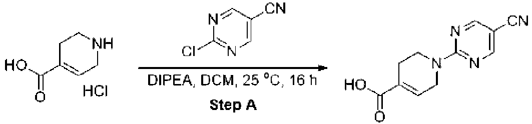

1,2,3,6-tetrahydropyridine-4-carboxylic acid hydrochloride (55 mg, 0.34 mmol, 1.0 eq.) was di ssolved in dichloromethane (5 mL), 2-chloro-5-cyanopyrimidine (47 mg, 0.34 mmol, 1.0 eq.) and DIPEA (200 mg, 1.55 mmol, 5.0 eq.) were added sequentially. The reaction mixture was reacte d at 25 °C for 16 h. The reaction mixture was diluted with water (50 mL) and extracted with E tOAc (2 × 50 mL). The solution was washed with saturated brine (100 mL), dried over anhydro us sodium sulfate, and concentrated. The crude product was purified by column chromatography (dichloromethane/methanol = 10/1; V/V) to obtain 1-(5-cyanopyrimidine-2-yl)-1,2,3,6-tetrahydropyri dine-4-carboxylic acid (75 mg, yellow solid), yield: 96.9%.

LCMS: *(ESI)* [M+H]⁺ = 231.0.

¹H NMR: (400 MHz, DMSO-*d*₆) *δ* 8.86 (s, 2H), 7.00 - 6.95 (m, 1H), 4.51 - 4.46 (m, 2H), 4.03 (t, *J =* 5.6 Hz, 2H), 2.44 - 2.40 (m, 2H).

### Step B: Preparation of (R)-1-(5-cyanopyrimidin-2-yl)-N-(1-(6-oxo-5-(trifluoromethyl)-1,6-dihydropyri din-3-yl)ethoxy)-1,2,3,6-tetrahydropyridin-4-carboxamide

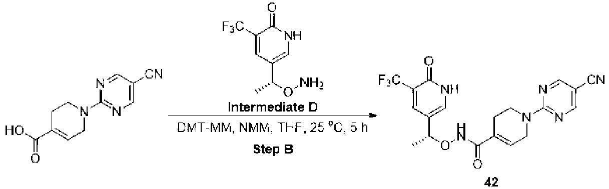

To a solution of 1-(5-cyanopyrimidine-2-yl)-1,2,3,6-tetrahydropyridine-4-carboxylic acid (75 mg, ye llow solid) in THF (5 mL), cooled to 0 °C in an ice bath, was added 4-(4,6-dimethoxytriazine-2 -yl)-4-methylmorpholine hydrochloride (119 mg, 0.43 mmol, 1.3 eq.). The reaction mixture was re acted at 0 °C for 20 min. N-methylmorpholine (100 mg, 0.99 mmol, 3.0 eq.) and (R)-5-(1-(amin ooxy)ethyl)-3-(trifluoromethyl)pyridin-2(1H)-one (73 mg, 0.33 mmol, 1.0 eq.) were added to the r eaction mixture. The reaction mixture was reacted at 25 °C for 5 h. The reaction mixture was q uenched with water (50 mL) and extracted with EtOAc (2 × 45 mL). The organic phases were c ombined and washed with saturated brine (100 mL), dried over anhydrous sodium sulfate and co ncentrated. The crude product was separated by column chromatography (petroleum ether/EtOAc = 2/1; V/V) and further purified by prep-HPLC to give compound 42, (R)-1-(5-cyanopyrimidin-2-yl)-N-(1-(6-oxo-5-(trifluoromethyl)-1,6-dihydropyridin-3-yl)ethoxy)-1,2,3,6-tetrahydropyridine-4-carbox amide (5.30 mg, white solid), yield: 3.7%.

LCMS: *(ESI)* [M+H]⁺ = 435.0.

¹H NMR: (400 MHz, DMSO-*d₆*) *δ* 12.31 (s, 1H), 11.02 (s, 1H), 8.79 (s, 2H), 8.01 (d, *J =* 2.0 Hz, 1H), 7.72 - 7.65 (m, 1H), 6.49 - 6.43 (m, 1H), 4.77 (q, *J =* 6.4 Hz, 1H), 4.40 - 4.33 (m, 2H), 3.94 (t, *J =* 6.0 Hz, 2H), 2.35 - 2.27 (m, 2H), 1.43 (d, *J =* 6.4 Hz, 3H).

### Example 43: Synthesis of Compound 43

### (R)-N-(1-(6-oxo-5-(trifluoromethyl)-1,6-dihydropyridin-3-yl)ethoxy)-1-(quinazolin-2-yl)-1,2,3,6-tetrahyd ropyridin-4-carboxamide

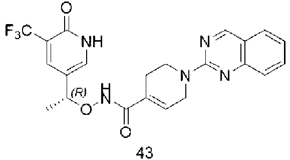

### Step A: Preparation of 1-(quinazolin-2-yl)-1,2,3,6-tetrahydropyridine-4-carboxylic acid

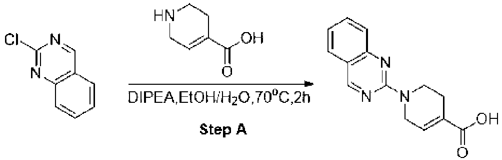

1,2,3,6-Tetrahydropyridine-4-carboxylic acid (150 mg, 0.92 mmol, 1.0 eq.) was dissolved in a mix ture of ethanol (3 mL) and water (1.5 mL), followed by the sequential addition of DIPEA (475 mg, 3.68 mmol, 4.0 eq.) and 2-chloroquinazoline (150 mg, 0.92 mmol, 1.0 eq.). The reaction mi xture was reacted at 70 °C for 2 h. The reaction mixture was diluted with water (30 mL), adjus ted to pH 6 with dilute hydrochloric acid (1 M), and extracted with EtOAc (5 × 20 mL). The c ombined organic phases were dried over anhydrous sodium sulfate and concentrated to give 1-(qu mazoline-2-yl)-1,2,3,6-tetrahydropyridine-4-carboxylic acid (140 mg, yellow solid), yield: 60.1%. LCMS: *(ESI)* [M+H]⁺ = 256.2.

¹H NMR (400 MHz, DMSO-*d₆*) *δ* 12.41 (s, 1H), 9.24 (s, 1H), 7.85 (dd, 1H), 7.78 - 7.69 (m, 1H), 7.54 (d, *J =* 8.5 Hz, 1H), 7.33 - 7.25 (m, 1H), 7.03 - 6.93 (m, 1H), 4.55 - 4.37 (m, 2H), 4.02 (t, *J =* 5.7 Hz, 2H), 2.43 - 2.35 (m, 2H).

### Step B: Preparation of (R)-N-(1-(6-oxo-5-(trifluoromethyl)-1,6-dihydropyridin-3-yl)ethoxy)-1-(quinaz olin-2-yl)-1,2,3,6-tetrahydropyridin-4-carboxamide)

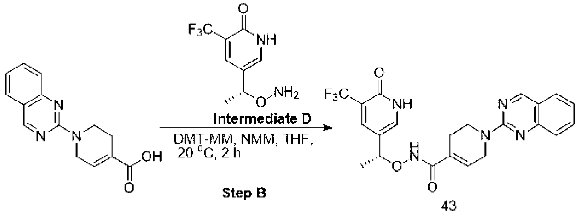

To a solution of 1-(quinazolin-2-yl)-1,2,3,6-tetrahydropyridine-4-carboxylic acid (130 mg, 0.51 mm ol, 1.0 eq.) in THF (3 mL),was added (R)-5-(1-(aminooxy)ethyl)-3-(trifluoromethyl)pyridine-2(1H)-one (115 mg, 0.51 mmol, 1.0 eq.), 4-(4,6-dimethoxytriazine-2-yl)-4-methylmorpholine hydrochlorid e (180 mg, 0.61 mmol, 1.2 eq.), and N-methylmorpholine (178 mg, 1.53 mmol, 3.0 eq.). The re action mixture was reacted at 20 °C for 2 h. The reaction mixture was quenched with water (25 mL) and extracted with EtOAc (2 × 20 mL). The organic phases were combined, washed with saturated brine (2 × 30 mL), dried over anhydrous sodium sulfate, and concentrated. The crude p roduct was purified by prep-HPLC to give compound 43, (R)-N-(1-(6-oxo-5-(trifluoromethyl)-1,6-d ihydropyridin-3-yl)ethoxy)-1-(quinazolin-2-yl)-1,2,3,6-tetrahydropyridin-4-carboxamide (97.65 mg, wh ite solid), yield: 44.9%.

LCMS: *(ESI)* [M+H]⁺ = 460.0.

¹H NMR (400 MHz, DMSO-*d₆*) *δ* 12.30 (s, 1H), 11.00 (s, 1H), 9.24 (s, 1H), 8.02 (d, *J* = 2.0 Hz, 1H), 7.86 (d, *J* = 8.0 Hz, 1H), 7.78 - 7.72 (m, 1H), 7.69 (s, 1H), 7.53 (d, *J =* 8.5 Hz, 1H), 7.29 (t, *J =* 7.4 Hz, 1H), 6.52 (s, 1H), 4.84 - 4.72 (m, 1H), 4.41 (d, *J =* 2.8 Hz, 2H), 4.00 (t, *J* = 5.6 Hz, 2H), 2.33 (s, 2H), 1.43 (d, *J* = 6.6 Hz, 3H).

Example 44: Synthesis of Compound 44

### (R)-1-(5-bromopyrimidin-2-yl)-N-(1-(6-oxo-5-(trifluoromethyl)-1,6-dihydropyridin-3-yl)ethoxy)-1,2,3,6-tetrahydropyridine-4-carboxamide

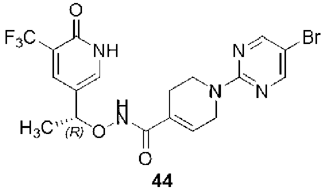

### Step A: Preparation of methyl 1-(5-bromopyrimidin-2-yl)piperidine-4-carboxylic acid

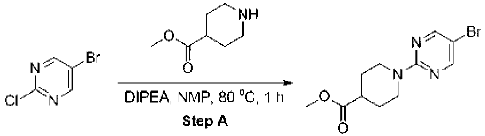

To a solution of 5-bromo-2-chloropyrimidin (11.61 g, 60.00 mL, 1.0 eq.) in N-methylpyrrolidone (100 mL) was added DIPEA (19.39 g, 150.00 mmol, 2.5 eq.) and methyl 4-piperidinecarboxylate (9.45 g, 66.00 mmol, 1.1 eq.) sequentially. The reaction mixture was reacted at 80 °C for 1 h. The reaction mixture was cooled to room temperature and diluted with water (500 mL), and extracted with EtOAc (2 × 200 mL). The organic phases were combined, washed with saturated brine (2 × 300 mL), dried over anhydrous sodium sulfate, and concentrated. The crude product was purified by column chromatography (petroleum ether/EtOAc = 15/1; V/V) to give methyl 1-(5-bromopyrimidin-2-yl)piperidine-4-carboxylate (15.63 g, white solid), yield: 86.8%.

LCMS: *(ESI)* [M+H]⁺ = 300.0.

¹H NMR: (400 MHz, CDCl₃) *δ* 8.27 (s, 2H), 4.56 (dt, *J =* 13.6, 3.6 Hz, 2H), 3.69 (s, 3H), 3.10 - 2.99 (m, 2H), 2.63 - 2.53 (m, 1H), 2.01 - 1.92 (m, 2H), 1.74 - 1.64 (m, 2H).

### Step B: Preparation of methyl 1-(5-bromopyrimidin-2-yl)-4-(phenylselenoyl)piperidine-4-carboxylic acid

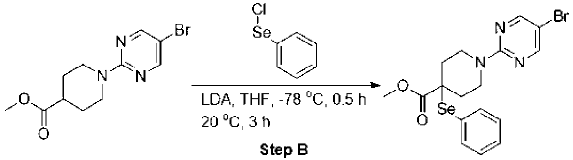

Methyl 1-(5-bromopyrimidin-2-yl)piperidine-4-carboxylic acid (1.80 g, 6.00 mmol, 1.0 eq.) was dis solved in THF (40 mL). Under nitrogen protection, the mixture was cooled to -78 °C, and LDA (2 M, 3.30 mL, 6.60 mmol, 1.1 eq.) was added dropwise. The reaction mixture was stirred at - 78 °C for 0.5 h. A solution of phenylselenyl chloride (1.26 g, 6.60 mmol, 1.1 eq.) in THF (5 m L) was added dropwise. The reaction mixture was then stirred at 20 °C for 3 h. The reaction m ixture was quenched with saturated ammonium chloride (100 mL) and extracted with EtOAc (2 × 100 mL). The organic phases were combined, dried over anhydrous sodium sulfate, and concen trated. The crude product was washed with column chromatography (petroleum ether / EtOAc = 15/1; V/V) to give methyl 1-(5-bromopyrimidin-2-yl)-4-(phenylselenoyl)piperidine-4-carboxylic acid (0.83 g, pale yellow solid) was obtained by separation and purification, yield: 30.4%.

LCMS: *(ESI)* [M+H]⁺ = 456.0.

¹H NMR: (400 MHz, CDCl₃) *δ* 8.27 (s, 2H), 7.58 - 7.53 (m, 2H), 7.44 - 7.38 (m, 1H), 7.35 - 7.29 (m, 2H), 4.25 - 4.15 (m, 2H), 3.66 (s, 3H), 3.46 - 3.35 (m, 2H), 2.24 - 2.13 (m, 2H), 1.97 - 1.87 (m, 2H). Step C: Preparation of methyl 1-(5-bromopyrimidin-2-yl)-1,2,3,6-tetrahydropyridine-4-carboxylic acid

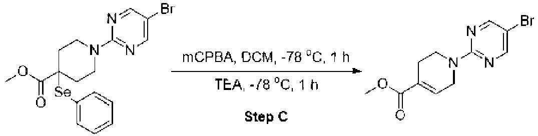

Methyl 1-(5-bromopyrimidin-2-yl)-4-(phenylselenoyl)piperidine-4-carboxylic acid (400 mg, 0.88 mmol, 1.0 eq.) was dissolved in dichloromethane (10 mL). Under nitrogen protection, the solution was cooled to -78 °C, and m-chloroperoxybenzoic acid (80%, 190 mg, 0.88 mmol, 1.0 eq.) was added. The reaction mixture was reacted at -78 °C for 1 hour. TEA (267 mg, 2.64 mmol, 3.0 eq.) was added to the reaction mixture. The reaction mixture was further reacted at -78 °C for 1 hour. The reaction mixture was quenched with saturated sodium bicarbonate aqueous solution (50 mL) and extracted with EtOAc (2 × 70 mL). The organic phases were combined, dried over anhydrous sodium sulfate, and concentrated. The crude product was purified by column chromatography (petroleum ether/EtOAc = 20/1; V/V) to give methyl 1-(5-bromopyrimidin-2-yl)-1,2,3,6-tetrahydropyridine-4-carboxylic acid (230 mg, pale yellow solid), yield: 87.8%.

LCMS: *(ESI)* [M+H]⁺ = 298.0.

¹H NMR: (400 MHz, CDCl₃) *δ* 8.32 (s, 2H), 7.03 - 6.97 (m, 1H), 4.34 (q, *J =* 3.2 Hz, 2H), 3.92 (t, *J =* 5.6 Hz, 2H), 3.77 (s, 3H), 2.53 - 2.44 (m, 2H).

### Step D: Preparation of 1-(5-bromopyrimidin-2-yl)-1,2,3,6-tetrahydropyridine-4-carboxylic acid

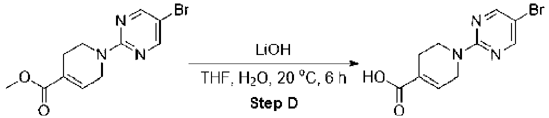

To a solution of Methyl 1-(5-bromopyrimidin-2-yl)-1,2,3,6-tetrahydropyridine-4-carboxylic acid (119 mg, 0.40 mmol, 1.0 eq.) in a mixture of THF (3 mL) and water (1.5 mL) was added lithium hydroxide (67 mg, 1.60 mmol, 4.0 eq.). The reaction mixture was reacted at 20 °C for 6 h. The reaction mixture was diluted with water (50 mL) and washed with EtOAc (20 mL). The aqueous phase was collected, adjusted to pH 6 with dilute hydrochloric acid (1 M), and extracted with EtOAc (2 × 50 mL). The organic phases were combined, dried over anhydrous sodium sulfate, and concentrated to give 1-(5-bromopyrimidin-2-yl)-1,2,3,6-tetrahydropyridine-4-carboxylic acid (91 mg, pale yellow solid), yield: 80.1%.

LCMS: *(ESI)* [M+H]⁺ = 284.0.

¹H NMR: (400 MHz, CDCl₃) *δ* 8.33 (s, 2H), 7.16 - 7.10 (m, 1H), 4.43 - 4.33 (m, 2H), 3.94 (t, *J =* 5.6 Hz, 2H), 2.55 - 2.45 (m, 2H).

### Step E: Preparation of (R)-1-(5-bromopyrimidin-2-yl)-N-(1-(6-oxo-5-(trifluoromethyl)-1,6-dihydropyri din-3-yl)ethoxy)-1,2,3,6-tetrahydropyridine-4-carboxamide

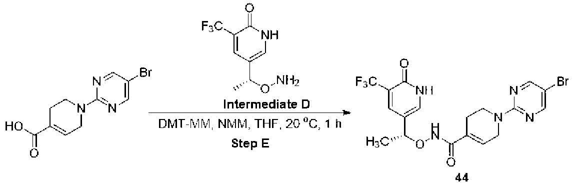

1-(5-bromopyrimidin-2-yl)-1,2,3,6-tetrahydropyridine-4-carboxylic acid (71 mg, 0.25 mmol, 1.0 eq.) was dissolved in THF (3.5 mL), cool to 0 °C in an ice bath, was added 4-(4,6-dimethoxytriazine-2-yl)-4-methylmorpholine hydrochloride (96 mg, 0.33 mmol, 1.3 eq.). The reaction mixture is reacted at 0 °C for 5 min. N-methylmorpholine (76 mg, 0.75 mmol, 3.0 eq.) and (R)-5-(1-(aminooxy)ethyl)-3-(trifluoromethyl)pyridin-2(1H)-one (58 mg, 0.26 mmol, 1.05 eq.) were added to the reaction mixture. The reaction mixture was reacted at 20 °C for 1 h. The reaction mixture was quenched with saturated ammonium chloride aqueous solution (50 mL) and extracted with EtOAc (2 × 50 mL). The organic phases were combined, dried over anhydrous sodium sulfate, and concentrated. The crude product was purified by prep-HPLC to give compound 44, (R)-1-(5-bromopyrimidin-2-yl)-N-(1-(6-oxo-5-(trifluoromethyl)-1,6-dihydropyridin-3-yl)ethoxy)-1,2,3,6-tetrahydropyridine-4-carboxamide (20.53 mg, white solid), yield: 16.8%.

LCMS: *(ESI)* [M+H]⁺ = 488.0.

¹H NMR: (400 MHz, DMSO-*d₆*) *δ* 12.15 (s, 1H), 11.01 (s, 1H), 8.48 (s, 2H), 8.01 (d, *J =* 1.6 Hz, 1H), 7.68 (d, *J* = 2.0 Hz, 1H), 6.50 - 6.41 (m, 1H), 4.78 (q, *J =* 6.4 Hz, 1H), 4.27 - 4.18 (m, 2H), 3.82 (t, *J =* 5.6 Hz, 2H), 2.32 - 2.22 (m, 2H), 1.43 (d, *J =* 6.4 Hz, 3H).

### Example 45: Synthesis of Compound 45

### (R)-1-(5-(4-fluorophenyl)pyrimidin-2-yl)-N-(1-(6-oxo-5-(trifluoromethyl)-1,6-dihydropyridin-3-yl)ethox y)-1,2,3,6-tetrahydropyridine-4-carboxamide

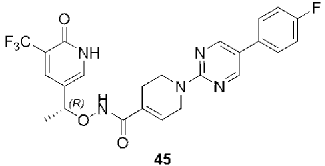

### Step A: Preparation of methyl 1-(5-bromopyrimidin-2-yl)piperidine-4-carboxylic acid

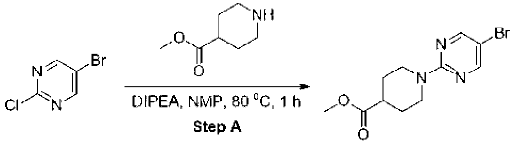

To a solution of 5-bromo-2-chloropyrimidin (11.61 g, 60.00 mL, 1.0 eq. ) in N-methylpyrrolidone (100 mL) was added DIPEA (19.39 g, 150.00 mmol, 2.5 eq.) and methyl 4-piperidinecarboxylate (9.45 g, 66.00 mmol, 1.1 eq.) sequentially. The reaction mixture was reacted at 80 °C for 1 h. The reaction mixture was cooled to room temperature and diluted with water (500 mL), and extracted with EtOAc (2 × 200 mL). The organic phases were combined, washed with saturated brine (2 × 300 mL), dried over anhydrous sodium sulfate, and concentrated. The crude product was purified by column chromatography (petroleum ether/EtOAc = 15/1; V/V) to give methyl 1-(5-bromopyrimidin-2-yl)piperidine-4-carboxylate (15.63 g, white solid), yield: 86.8%.

LCMS: *(ESI)* [M+H]⁺ = 300.0.

¹H NMR: (400 MHz, CDCl₃) *δ* 8.27 (s, 2H), 4.56 (dt, *J =* 13.6, 3.6 Hz, 2H), 3.69 (s, 3H), 3.10 - 2.99 (m, 2H), 2.63 - 2.53 (m, 1H), 2.01 - 1.92 (m, 2H), 1.74 - 1.64 (m, 2H).

### Step B: Preparation of methyl 1-(5-(4-fluorophenyl)pyrimidin-2-yl)piperidine-4-carboxylate

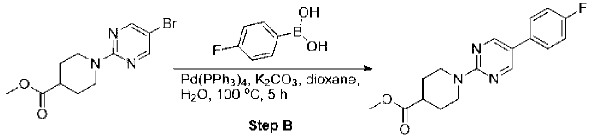

To a solution of Methyl 1-(5-bromopyrimidin-2-yl)piperidine-4-carboxylic acid (2.50 g, 8.36 mmol, 1.0 eq.) in 1,4-dioxane (25 mL) was added (4-fluorophenyl)boronic acid (1.28 g, 9.19 mmol, 1.1 eq.), potassium carbonate (3.46 g, 25.08 mmol, 3.0 eq.), water (3 mL), and tetra(triphenylphosphine)palladium (0.482 g, 0.42 mmol, 0.05 eq.). The reaction mixture was reacted at 100 °C for 5 hours under nitrogen protection. The reaction mixture was diluted with water (30 mL) and extracted with dichloromethane (2 × 30 mL). The combined organic phases were washed with saturated brine (10 mL), dried over anhydrous sodium sulfate, and concentrated. The crude product was purified by column chromatography (petroleum ether/EtOAc = 4/1; V/V) to give methyl 1-(5-(4-fluorophenyl)pyrimidin-2-yl)piperidine-4-carboxylic acid (2.1 g, yellow solid), yield: 81.9%.

LCMS: *(ESI)* [M+H]⁺ = 316.2.

### Step C: Preparation of methyl 1-(5-(4-fluorophenyl)pyrimidin-2-yl)-4-(phenylselenoyl)piperidine-4-ca rboxylate

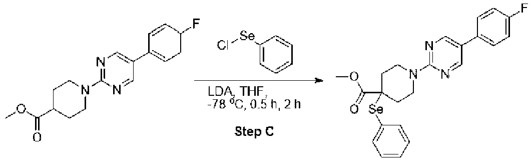

Methyl 1-(5-(4-fluorophenyl)pyrimidin-2-yl)piperidine-4-carboxylic acid (1.9 g, 6.03 mmol, 1.0 eq.) in THF (20 mL). Under nitrogen protection, the mixture was cooled to -78 °C, and LDA (2 M, 6 mL, 12.06 mmol, 6.0 eq.) was added dropwise. After reacting for half an hour, phenylselenyl chloride (1.4 g, 7.23 mmol, 1.2 eq.) was added to the reaction mixture, and the mixture was re acted at -78 °C for 2 hours. The reaction mixture was quenched with saturated ammonium chlori de aqueous solution (30 mL) and extracted with EtOAc (3 × 20 mL). The organic phases were combined, dried over anhydrous sodium sulfate, and concentrated. The crude product was purified by column chromatography (petroleum ether/EtOAc = 4/1; V/V) to give methyl 1-(5-(4-fluorophe nyl)pyrimidin-2-yl)-4-(phenylseleno)piperidine-4-carboxylic acid (1.4 g, white solid), yield: 49.2%. LCMS: *(ESI)* [M+H]⁺ = 472.2.

¹H NMR: (400 MHz, CDCl₃) *δ* 8.41 (s, 2H), 7.53 - 7.48 (m, 2H), 7.38 - 7.31 (m, 3H), 7.29 - 7.23 (m, 2H), 7.10 - 7.02 (m, 2H), 4.30 - 4.17 (m, 2H), 3.60 (s, 3H), 3.47 - 3.34 (m, 2H), 2.22 - 2.10 (m, 2H), 1.97 - 1.84 (m, 2H).

### Step D: Preparation of methyl 1-(5-(4-fluorophenyl)pyrimidin-2-yl)-1,2,3,6-tetrahydropyridine-4-carb oxylate

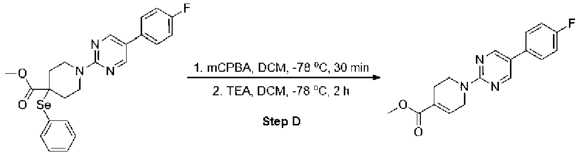

Methyl 1-(5-(4-fluorophenyl)pyrimidin-2-yl)-4-(phenylseleno)piperidine-4-carboxylic acid (1.4 g, 2.9 7 mmol, 1.0 eq.) in dichloromethane (15 mL), cooled to -78 °C, and m-chloroperoxybenzoic acid (1.0 g, 5.94 mmol, 2.0 eq.) was added. The reaction mixture was reacted at -78 °C for 30 min. TEA (3.0 g, 29.7 mmol, 10.0 eq.) was added to the reaction mixture. The reaction mixture was reacted at -78 °C for 2 hours. The reaction mixture was quenched with 30 mL of ammonium c hloride aqueous solution and extracted with EtOAc (3 × 30 mL). The combined organic phases were dried over anhydrous sodium sulfate and concentrated. The crude product was purified by c olumn chromatography (petroleum ether / EtOAc = 4 / 1; V / V) to methyl 1-(5-(4-fluorophenyl) pyrimidin-2-yl)-1,2,3,6-tetrahydropyridine-4-carboxylic acid (800 mg, white solid), yield: 86.0%. LCMS: *(ESI)* [M+H]⁺ = 314.2.

¹H NMR: (400 MHz, DMSO-*d₆*) *δ* 8.72 (s, 2H), 7.75 - 7.65 (m, 2H), 7.29 (t, *J =* 8.5 Hz, 2H), 7.02 (s, 1H), 4.41 (s, 2H), 3.99 - 3.91 (m, 2H), 3.70 (s, 3H), 2.40 (s, 2H).

### Step E: Preparation of 1-(5-(4-fluorophenyl)pyrimidin-2-yl)-1,2,3,6-tetrahydropyridine-4-carboxylic acid

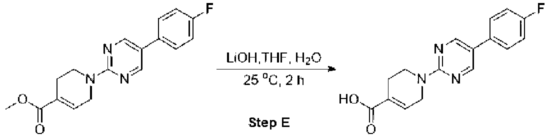

To a solution of Methyl 1-(5-(4-fluorophenyl)pyrimidin-2-yl)-1,2,3,6-tetrahydropyridine-4-carboxylic acid (200 mg, 0.63 mmol, 1.0 eq.) in a mixed solvent of THF (8 mL) and water (4 mL) was added lithium hydroxide (30 mg, 1.27 mmol, 2.0 eq.). The reaction mixture was reacted at 25 ° C for 2 h. The reaction mixture was diluted with water (10 mL), adjusted to pH = 6 with dilut e hydrochloric acid (1 M), and extracted with EtOAc (3 × 20 mL). The organic phases were co mbined, dried over anhydrous sodium sulfate, and concentrated to give 1-(5-(4-fluorophenyl)pyrimi din-2-yl)-1,2,3,6-tetrahydropyridine-4-carboxylic acid (170 mg, white solid), yield: 89.1%.

¹H NMR: (400 MHz, DMSO-*d₆*) *δ* 12.46 (s, 1H), 8.72 (s, 2H), 7.72 - 7.67 (m, 2H), 7.31 - 7.26 (m, 2H), 6.99 - 6.93 (m, 1H), 4.43 - 4.34 (m, 2H), 3.93 (t, J = 5.7 Hz, 2H), 2.42 - 2.30 (m, 2H).

### Step F: Preparation of (R)-1-(5-(4-fluorophenyl)pyrimidin-2-yl)-N-(1-(6-oxo-5-(trifluoromethyl)-1,6-di hydropyridin-3-yl)ethoxy)-1,2,3,6-tetrahydropyridine-4-carboxamide

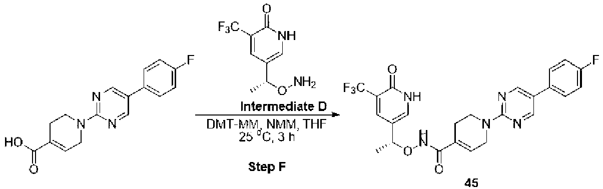

To a solution of 1-(5-(4-fluorophenyl)pyrimidin-2-yl)-1,2,3,6-tetrahydropyridine-4-carboxylic acid (8 0 mg, 0.26 mmol, 1.0 eq.) in THF (3 mL), cooled to 0 °C in an ice bath, was added 4-(4,6-di methoxytriazine-2-yl)-4-methylmorpholine hydrochloride (88 mg, 0.32 mmol, 1.2 eq.). The reaction mixture was reacted at 0 °C for 5 min. N-methylmorpholine (81 mg, 0.80 mmol, 3.0 eq.) and (R)-5-(1-(aminooxy)ethyl)-3-(trifluoromethyl)pyridin-2(1H)-one (60 mg, 0.26 mmol, 1.0 eq.) were a dded to the reaction mixture. The reaction mixture was reacted at 25 °C for 3 h. The reaction mixture was quenched with saturated ammonium chloride aqueous solution (15 mL) and extracted with EtOAc (3 × 10 mL). The organic phases were combined, dried over anhydrous sodium sul fate, and concentrated. The crude product was purified by prep-HPLC to give compound 45, (R)-1-(5-(4-fluorophenyl)pyrimidin-2-yl)-N-(1-(6-oxo-5-(trifluoromethyl)-1,6-dihydropyridin-3-yl)ethoxy)-1, 2,3,6-tetrahydropyridine-4-carboxamide (36.38 mg, white solid), yield: 27.0%.

LCMS: *(ESI)* [M+H]⁺ = 504.2.

¹H NMR: (400 MHz, DMSO-*d*₆) *δ* 12.30 (s, 1H), 11.00 (s, 1H), 8.71 (s, 2H), 8.01 (d, *J =* 2.0 Hz, 1H), 7.73 - 7.65 (m, 3H), 7.33 - 7.23 (m, 2H), 6.49 (s, 1H), 4.78 (d, *J =* 6.4 Hz, 1H), 4.31 (d, *J* = 2.9 Hz, 2H), 3.90 (t, *J* = 5.7 Hz, 2H), 2.30 (s, 2H), 1.43 (d, *J =* 6.6 Hz, 3H).

### Example 46: Synthesis of Compound 46

### N-(2-hydroxy-1-(6-oxo-5-(trifluoromethyl)-1,6-dihydropyridin-3-yl)ethoxy)-1-(5-(trifluoromethyl)pyrimi din-2-yl)-1,2,3,6-tetrahydropyridin-4-carboxamide

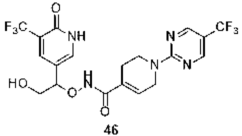

### Step A: Preparation of 2-methoxy-3-(trifluoromethyl)-5-vinylpyridine

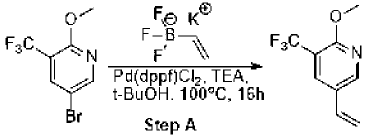

To a solution of 5-Bromo-2-methoxy-3-(trifluoromethyl)pyridine (5 g, 19.6 mmol, 1.0 eq.) in tert-butanol (50 mL) was added potassium ethylenetrifluoroborate (3.9 g, 29.3 mmol, 1.5 eq.), 1,1-bis (diphenylphosphine)dimerferropalladium(II) chloride (731 mg, 1.0 mmol, 0.05 eq.), and TEA (2 g, 19.6 mmol, 1.0 eq.). The reaction mixture was reacted at 100 °C for 16 h. The reaction mixtur e was cooled to room temperature and quenched with water (60 mL), and extracted with EtOAc (2 × 50 mL). The organic phases were combined, dried over anhydrous sodium sulfate, and con centrated. The crude product was purified by column chromatography (petroleum ether/EtOAc = 95/5; V/V) to give 2-methoxy-3-(trifluoromethyl)-5-vinylpyridine (1.3 g, yellow oil), yield: 33.3%. LCMS: *(ESI)* [M+H]⁺ = 204.2.

### Step B: Preparation of 2-methoxy-5-(ethylene oxide-2-yl)-3-(trifluoromethyl)pyridine

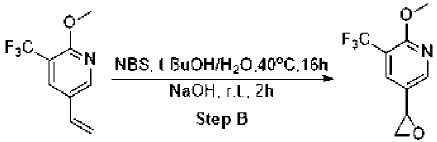

To a solution of 2-methoxy-3-(trifluoromethyl)-5-vinylpyridine (800 mg, 3.95 mmol, 1.0 eq.) in a mixture of tert-butanol (8 mL) and water (8 mL) was added N-bromosuccinimide (698 mg, 3.95 mmol, 1.0 eq.). The reaction mixture was reacted at 40 °C for 16 h. The reaction mixture was cooled to 0 °C, and sodium hydroxide solution (2.37 mL, 11.85 mmol, 5 M, 3.0 eq.) was added. The reaction mixture was reacted at room temperature for 2 h. The reaction mixture was quenched with water (30 mL) and extracted with EtOAc (2 × 25 mL). The organic phases were combined, dried over anhydrous sodium sulfate, and concentrated. The crude product was purified by column chromatography (petroleum ether/EtOAc = 98/2; V/V) to give 2-methoxy-5-(ethylene oxide-2-yl)-3-(trifluoromethyl)pyridine (590 mg, yellow oil), yield: 77.5%.

LCMS: *(ESI)* [M+H]⁺ = 220.2.

¹H NMR (400 MHz, CDCl₃) *δ* 8.22 (d, *J =* 2.0 Hz, 1H), 7.61 (d, *J =* 2.1 Hz, 1H), 3.97 (s, 3H), 3.84 - 3.78 (m, 1H), 3.13 - 3.11 (m, 1H), 2.79 - 2.70 (m, 1H).

### Step C: Preparation of 1-(6-methoxy-5-(trifluoromethyl)pyridin-3-yl)ethane-1,2-diol

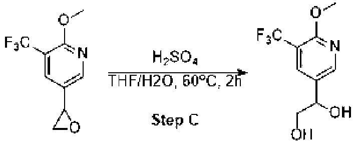

2-methoxy-5-(ethylene oxide-2-yl)-3-(trifluoromethyl)pyridine (590 mg, 2.53 mmol, 1.0 eq.) was dissolved in a mixed solvent of THF (4 mL) and water (2 mL). Sulfuric acid (2 mL) was added at 0 °C. The reaction mixture was reacted at 60 °C for 2 hours. The reaction mixture was adjusted to pH 7 with sodium bicarbonate aqueous solution, and extracted with EtOAc (2 × 35 mL). The organic phases were combined, dried over anhydrous sodium sulfate, and concentrated to give 1-(6-methoxy-5-(trifluoromethyl)pyridin-3-yl)ethane-1,2-diol (660 mg, yellow oil), yield: 100%.

LCMS: *(ESI)* [M+H]⁺ = 238.2.

### Step D: Preparation of 2-(tert-butyldimethylsiloxy)-1-(6-methoxy-5-(trifluoromethyl)pyridin-3-yl)etha ne-1-ol

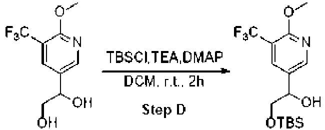

To a solution of 1-(6-methoxy-5-(trifluoromethyl)pyridin-3-yl)ethane-1,2-diol (660 mg, 2.79 mmol, 1.0 eq.) in dichloromethane (8 mL),was added tert-butyldimethylchlorosilane (627 mg, 4.18 mmol, 1.5 eq.), TEA (705 mg, 6.98 mmol, 2.5 eq.), and 4-dimethylaminopyridine (66 mg, 0.54 mmol, 0.2 eq.). The reaction mixture was reacted at room temperature for 2 hours. The reaction mixture was quenched with water (30 mL) and extracted with EtOAc (3 × 25 mL). The organic phases were combined, dried over anhydrous sodium sulfate, and concentrated. The crude product was purified by column chromatography (petroleum ether / EtOAc = 4 / 1; V / V) to give 2-(tert-but yldimethylsiloxy)-1-(6-methoxy-5-(trifluoromethyl)pyridin-3-yl)ethane-1-ol (560 mg, colorless oil), yi eld: 57.3%.

LCMS: *(ESI)* [M+H]⁺ = 352.2.

¹H NMR (400 MHz, CDCl₃) *δ* 8.21 (d, *J =* 2.2 Hz, 1H), 7.84 (d, *J =* 2.2 Hz, 1H), 4.74 - 4.60 (m, 1H), 3.96 (s, 3H), 3.77 - 3.62 (m, 1H), 3.51 - 3.44 (m, 1H), 2.90 (d, *J =* 2.8 Hz, 1H), 0.84 (s, 9H), 0.00 (d, *J =* 2.6 Hz, 6H).

### Step E: Preparation of 2-(2-(tert-butyldimethylsiloxy)-1-f6-methoxy-5-(trifluoromethyl)-1,6-dihydropy ridin-3-yl)ethoxy)isodihydroindole-1,3-dione

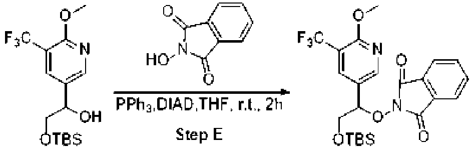

2-(tert-butyldimethylsiloxy)-1-(6-methoxy-5-(trifluoromethyl)pyridin-3-yl)ethane-1-ol (560 mg, 1.6 m mol, 1.0 eq.) was dissolved in THF (8 mL), followed by the sequential addition of 2-hydroxyiso dihydroindole-1,3-dione (274 mg, 1.68 mmol, 1.05 eq.), triphenylphosphine (462 mg, 1.76 mmol, 1.1 eq.), and diisopropyl azodicarbonate (356 mg, 1.76 mmol, 1.1 eq.) was added dropwise at 0 °C. The reaction mixture was reacted at room temperature for 2 hours. The reaction mixture wa s quenched with water (30 mL) and extracted with EtOAc (3 × 25 mL). The organic phases wer e combined, dried over anhydrous sodium sulfate, and concentrated. The crude product was purifi ed by column chromatography (petroleum ether/EtOAc = 7/3; V/V) to give 2-(2-(tert-butyldimethy lsiloxy)-1-(6-methoxy-5-(trifluoromethyl)-1,6-dihydropyridin-3-yl)ethoxy)isodihydroindole-1,3-dione (7 60 mg, colorless oil), yield: 100%.

LCMS: *(ESI)* [M+H]⁺ = 497.2.

### Step F: Preparation of 2-(2-(tert-butyldimethylsiloxy)-1-(6-oxo-5-(trifluoromethyl)-1,6-dihydropyridin-3-yl)ethoxy)isodihydroindole-1,3-dione

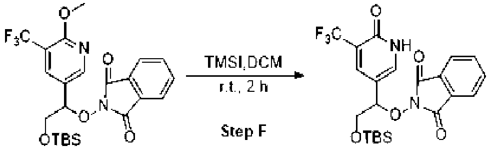

To a solution of 2-(2-(tert-butyldimethylsiloxy)-1-(6-methoxy-5-(trifluoromethyl)-1,6-dihydropyridin-3 -yl)ethoxy)isodihydroindole-1,3-dione (760 mg, 1.52 mmol, 1.0 eq.) in dichloromethane (7 mL) w as added TMSI (0.7 mL). The reaction mixture was reacted at room temperature for 2 hours. Th e reaction mixture was quenched with water (40 mL) and extracted with dichloromethane (2 × 3 0 mL). The organic phases were combined, dried over anhydrous sodium sulfate, and concentrate d. The crude product was purified by column chromatography (dichloromethane/methanol = 94/6; V/V) to give 2-(2-(tert-butyldimethylsiloxy)-1-(6-oxo-5-(trifluoromethyl)-1,6-dihydropyridin-3-yl)eth oxy)isodihydroindole-1,3-dione (240 mg, colorless oil), yield: 32.6%.

LCMS: *(ESI)* [M+H]⁺ = 483.0.

¹H NMR (400 MHz, DMSO-*d*₆) *δ* 12.32 (s, 1H), 8.19 (d, *J =* 2.0 Hz, 1H), 7.83 (s, 4H), 7.80 (s, 1H), 5.13 (t, *J* = 4.4 Hz, 1H), 4.18 - 3.91 (m, 2H), 0.82 (s, 9H), 0.01 (d, *J =* 4.0 Hz, 6H).

### Step G: Preparation of 5-(1-(aminooxy)-2-(tert-butyldimethylsiloxy)ethyl)-3-(trifluoromethyl)pyridin-2 (1H)-one

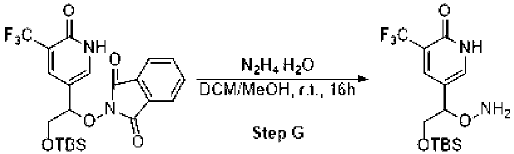

To a solution of 2-(2-(tert-butyldimethylsiloxy)-1-(6-oxo-5-(trifluoromethyl)-1,6-dihydropyridin-3-yl)ethoxy)isodihydroin dole-1,3-dione (220 mg, 0.576 mmol, 1.0 eq.) in a mixture of dichloromethane (2 mL) and methanol (1 mL) was added hydrazine hydrate (85%, 173 mg, 3.46 mmol, 6.0 eq.). The reaction mixture was reacted at room temperature for 16 hours. The reaction mixture was concentrated, and the residue was added to dichloromethane (20 mL) and filtered. The filtrate was concentrated again. The crude product was purified by column chromatography (petroleum ether/EtOAc = 1/9; V/V) to obtain 5-(1-(aminooxy)-2-(tert-butyldimethylsiloxy)ethyl)-3-(trifluoromethyl)pyridin-2(1H)-one (100 mg, white solid), yield: 62.5%.

LCMS: *(ESI)* [M+H]⁺ =353.2.

¹H NMR (400 MHz, DMSO-*d*₆) *δ* 12.27 (s, 1H), 7.89 (d, *J =* 2.0 Hz, 1H), 7.63 (d, *J =* 2.2 Hz, 1H), 6.07 (s, 2H), 4.39 (t, *J* = 4.7 Hz, 1H), 3.81 - 3.75 (m, 2H), 0.84 (s, 9H), -0.00 (s, 6H).

### Step H: Preparation of N-(2-(tert-butyldimethylsiloxy)-1-(6-oxo-5-(trifluoromethyl)-1,6-dihydropyridin -3-yl)ethoxy)-1-(5-(trifluoromethyl)pyrimidin-2-yl)-1,2,3,6-tetrahydropyridin-4-carboxamide

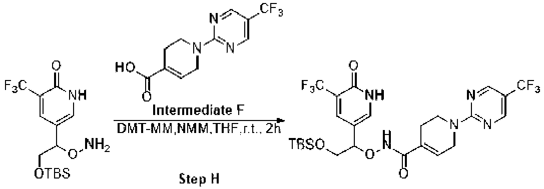

1-(5-(trifluoromethyl)pyrimidin-2-yl)-1,2,3,6-tetrahydropyridine-4-carboxylic acid (65 mg, 0.228 mm ol, 1.0 eq.) was dissolved in THF (2 mL), followed by the addition of 5-(1-(aminooxy)-2-(tert-bu tyldimethylsiloxy)ethyl)-3-(trifluoromethyl)pyridine-2(1H)-one (80 mg, 0.228 mmol, 1.0 eq.), 4-(4,6-dimethoxytriazine-2-yl)-4-methylmorpholine hydrochloride (80 mg, 0.274 mmol, 1.2 eq.), and N-m ethylmorpholine (70 mg, 0.684 mmol, 3.0 eq.). The reaction mixture was allowed to react at roo m temperature for 2 hours. The reaction mixture was quenched with water (20 mL) and extracte d with EtOAc (2 × 15 mL). The combined organic phases were dried over anhydrous sodium su lfate and concentrated to give N-(2-(tert-butyldimethylsiloxy)-1-(6-oxo-5-(trifluoromethyl)-1,6-dihydr opyridin-3-yl)ethoxy)-1-(5-(trifluoromethyl)pyrimidin-2-yl)-1,2,3,6-tetrahydropyridin-4-carboxamide (1 60 mg, yellow solid), yield: 100%.

LCMS: *(ESI)* [M+H]⁺ = 608.1.

### Step I: Preparation of N-(2-hydroxy-1-(6-oxo-5-(trifluoromethyl)-1,6-dihydropyridin-3-yl)ethoxy)-1-(5 -(trifluoromethyl)pyrimidin-2-yl)-1,2,3,6-tetrahydropyridine-4-carboxamide)

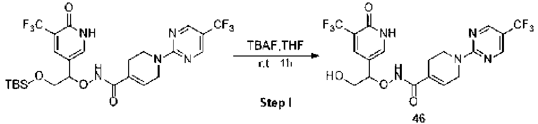

To a solution of N-(2-(tert-butyldimethylsiloxy)-1-(6-oxo-5-(trifluoromethyl)-1,6-dihydropyridin-3-yl) ethoxy)-1-(5-(trifluoromethyl)pyrimidin-2-yl)-1,2,3,6-tetrahydropyridine-4-carboxamide (120 mg, 0.19 8 mmol, 1.0 eq.) in THF (1 mL) was added tetrabutylammonium fluoride (0.4 mL, 0.396 mmol, 2.0 eq.). The reaction mixture was reacted at room temperature for 1 hour. The reaction mixtur e was quenched with water (10 mL) and extracted with EtOAc (2 × 10 mL). The organic phase s were combined, dried over anhydrous sodium sulfate, and concentrated. The crude product was purified by prep-HPLC to give compound 46, N-(2-hydroxy-1-(6-oxo-5-(trifluoromethyl)-1,6-dihyd ropyridin-3-yl)ethoxy)-1-(5-(trifluoromethyl)pyrimidin-2-yl)-1,2,3,6-tetrahydropyridin-4-carboxamide (3 7.37 mg, white solid), yield: 38.1%.

LCMS: *(ESI)* [M+H]⁺ = 494.2.

¹H NMR (400 MHz, DMSO-*d*₆) *δ* 12.33 (s, 1H), 11.19 (s, 1H), 8.74 (s, 2H), 8.02 (s, 1H), 7.71 (s, 1H), 6.55 - 6.48 (m, 1H), 4.67 (t, *J =* 5.1 Hz, 1H), 4.36 (d, *J =* 2.8 Hz, 2H), 3.95 (t, *J =* 5.7 Hz, 2H), 3.76 - 3.62 (m, 2H), 2.32 (s, 2H).

### Example 47: Synthesis of Compound 47

### N-(2-(6-oxo-5-(trifluoromethyl)-1,6-dihydropyridin-3-yl)propoxy)-1-(5-(trifluoromethyl)pyrimidin-2-yl)-1,2,3,6-tetrahydropyridin-4-carboxamide

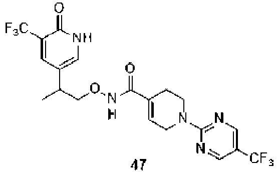

### Step A: Preparation of 2-methoxy-5-(isopropenyl)-3-(triflucromethyl)pyridine

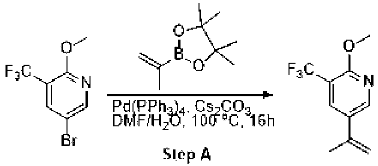

To a solution of 5-bromo-2-methoxy-3-(trifluoromethyl)pyridine (300 mg, 1.18 mmol, 1.0 eq.) in a mixed solvent of N,N-dimethylformamide (6 mL) and water (2 mL) was added pinacol isopropenylborate (296 mg, 1.76 mmol, 1.5 eq.), tetrakis(triphenylphosphine)palladium (138 mg, 0.12 mmol, 0.1 eq.) and cesium carbonate (770 mg, 2.36 mmol, 2.0 eq.). The reaction mixture was reacted at 100 °C for 16 h. The reaction mixture was cooled to room temperature and quenched with water (30 mL), and extracted with EtOAc (2 × 25 mL). The organic phases were combined, dried over anhydrous sodium sulfate, and concentrated. The crude product was purified by column chromatography (petroleum ether/EtOAc = 99/1; V/V) to give 2-methoxy-5-(isopropenyl)-3-(trifluoromethyl)pyridine (160 mg, colorless oil), yield: 62.5%.

LCMS: *(ESI)* [M+H]⁺ = 218.2.

¹H NMR (400 MHz, CDCl₃) *δ* 8.32 (d, *J =* 2.0 Hz, 1H), 7.86 (d, *J =* 2.4, 0.5 Hz, 1H), 5.27 (s, 1H), 5.10 - 5.00 (m, 1H), 3.97 (s, 3H), 2.15 - 1.95 (m, 3H).

### Step B: Preparation of 2-methoxy-5-(2-methylethyleneoxy-2-yl)-3-(trifluoromethyl)pyridine

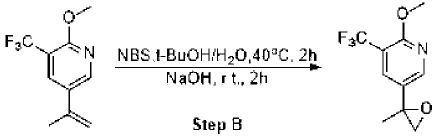

To a solution of 2-methoxy-5-(isopropenyl)-3-(trifluoromethyl)pyridine (320 mg, 1.48 mmol, 1.0 e q.) in a mixed solvent of tert-butanol (5 mL) and water (3 mL) was added N-bromosuccinimide (262 mg, 1.48 mmol, 1.0 eq.). The reaction mixture was reacted at 40 °C for 2 h. The reaction mixture was cooled to 0 °C, and sodium hydroxide aqueous solution (0.89 mL, 4.44 mmol, 5 M, 3.0 eq.) was added. The reaction mixture was reacted at room temperature for 2 h. The react ion mixture was quenched with water (30 mL) and extracted with EtOAc (2 × 25 mL). The org anic phases were combined, dried over anhydrous sodium sulfate, and concentrated. The crude pr oduct was purified by column chromatography (petroleum ether/EtOAc = 97/3; V/V) to give 2-m ethoxy-5-(2-methylepoxyethylene-2-yl)-3-(trifluoromethyl)pyridine (240 mg, yellow oil), yield: 68. 5%.

LCMS: *(ESI)* [M+H]⁺ = 234.2.

¹H NMR (400 MHz, CDCl₃) *δ* 8.26 (d, *J =* 2.0 Hz, 1H), 7.72 (dd, *J =* 2.3, 0.5 Hz, 1H), 3.96 (s, 3H), 2.94 (d, *J* = 5.1 Hz, 1H), 2.73 (dd, *J* = 5.1, 0.5 Hz, 1H), 1.66 (s, 3H).

### Step C: Preparation of 2-(6-methoxy-5-(trifluoromethyl)pyridin-3-yl)propane-1-ol

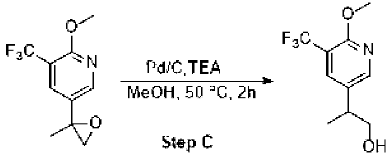

To a solution of 2-methoxy-5-(2-methylepoxyethylene-2-yl)-3-(trifluoromethyl)pyridine (160 mg, 0.69 mmol, 1.0 eq.) in methanol (3 mL) was added palladium on carbon (16 mg) and TEA (346 mg, 3.44 mmol, 5.0 eq.). The reaction mixture was degassed with a hydrogen balloon and reacted at 50 °C for 2 h under a hydrogen atmosphere. The reaction mixture was filtered through a Celite pad, and the filtrate was concentrated. The crude product was purified by column chromatography (petroleum ether/EtOAc = 4/1; V/V) to obtain 2-(6-methoxy-5-(trifluoromethyl)pyridin-3-yl)propane-1-ol (160 mg, colorless oil), yield: 99.3%.

LCMS: *(ESI)* [M+H]⁺ = 236.2.

¹H NMR (400 MHz, CDCl₃) *δ* 8.12 (d, *J =* 2.1 Hz, 1H), 7.66 (d, *J =* 2.3 Hz, 1H), 3.95 (s, 3H), 3.73 - 3.51 (m, 2H), 2.98 - 2.75 (m, 1H), 1.56 (s, 1H), 1.22 (d, *J* = 7.1 Hz, 3H).

### Step D: Preparation of 2-(2-(6-methoxy-5-(trifluoromethyl)pyridin-3-yl)propoxy)isodihydroindole-1,3-dione

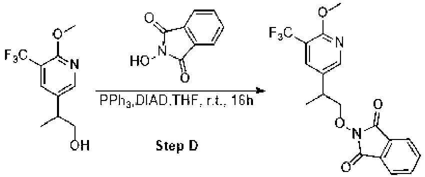

2-(6-methoxy-5-(trifluoromethyl)pyridin-3-yl)propane-1-ol (160 mg, 0.68 mmol, 1.0 eq.) was dissol ved in THF (3 mL), followed by the sequential addition of 2-hydroxyisodihydroindole-1,3-dione (117 mg, 0.72 mmol, 1.05 eq.) and triphenylphosphine (197 mg, 0.75 mmol, 1.1 eq.), and finally, diisopropyl azodicarbonate (152 mg, 0.75 mmol, 1.1 eq.) was added dropwise at 0 °C. The reac tion mixture was reacted at room temperature for 16 hours. The reaction mixture was quenched with water (30 mL) and extracted with EtOAc (3 × 25 mL). The organic phases were combined, dried over anhydrous sodium sulfate, and concentrated. The crude product was purified by colu mn chromatography (petroleum ether/EtOAc = 78/22; V/V) to obtain 2-(2-(6-methoxy-5-(trifluorom ethyl)pyridin-3-yl)propoxy)isodihydroindole-1,3-dione (240 mg, colorless oil), yield: 93%.

LCMS: *(ESI)* [M+H]⁺ = 381.2.

### Step E: Preparation of O-(2-(6-methoxy-5-(trifluoromethyl)pyridin-3-yl)propyl)hydroxylamine

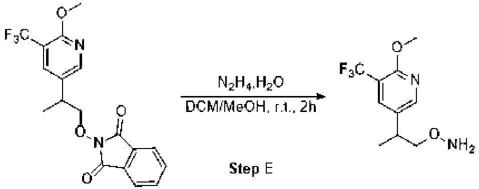

To a solution of 2-(2-(6-methoxy-5-(trifluoromethyl)pyridin-3-yl)propoxy)isodihydroindole-1,3-dione (240 mg, 0.64 mmol, 1.0 eq.) in a mixed solvent of dichloromethane (2 mL) and methanol (1 mL) was added hydrazine hydrate (85%, 190 mg, 3.79 mmol, 6.0 eq.). The reaction mixture was reacted at room temperature for 2 hours, and the reaction mixture was concentrated. Dichloromethane (20 mL) was added to the residue, stirred, and filtered. The filtrate was concentrated again. The crude product was purified by column chromatography (petroleum ether/EtOAc = 7/3; V/V) to obtain O-(2-(6-methoxy-5-(trifluoromethyl)pyridin-3-yl)propyl)hydroxylamine (150 mg, colorless oil), yield: 95.5%.

LCMS: *(ESI)* [M+H]⁺ = 251.2.

¹H NMR (400 MHz, CDCl₃) *δ* 8.11 (d, *J =* 2.0 Hz, 1H), 7.64 (d, *J =* 2.1 Hz, 1H), 3.95 (s, 3H), 3.70 - 3.58 (m, 2H), 3.15 - 2.93 (m, 1H), 1.18 (d, *J* = 1.9 Hz, 3H).

### Step F: Preparation of N-(2-(6-methoxy-5-(trifluoromethyl)pyridin-3-yl)propoxy)-1-(5-(trifluoromethyl) pyrimidin-2-yl)-1,2,3,6-tetrahydropyridin-4-carboxamide

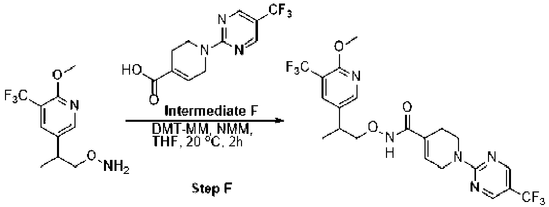

1-(5-(trifluoromethyl)pyrimidin-2-yl)-1,2,3,6-tetrahydropyridine-4-carboxylic acid (140 mg, 0.52 mmol, 1.0 eq.) was dissolved in THF (3 mL), followed by the addition of O-(2-(6-methoxy-5-(trifluoromethyl)pyridin-3-yl)propyl)hydroxylamine (120 mg, 0.52 mmol, 1.0 eq.), 4-(4,6-dimethoxytriazine-2-yl)-4-methylmorpholine hydrochloride (184 mg, 0.63 mmol, 1.2 eq.), and N-methylmorpholine (158 mg, 1.56 mmol, 3.0 eq.). The reaction mixture was reacted at 20 °C for 2 h. The reaction mixture was quenched with water (20 mL) and extracted with EtOAc (2 × 15 mL). The organic phases were combined, dried over anhydrous sodium sulfate, and concentrated. The crude product was purified by column chromatography (petroleum ether/EtOAc = 6/4; V/V) to obtain N-(2-(6-methoxy-5-(trifluoromethyl)pyridin-3-yl)propoxy)-1-(5-(trifluoromethyl)pyrimidin-2-yl)-1,2,3,6-tetrahydropyridine-4-carboxamide (70 mg, yellow oil), yield: 26.7%.

LCMS: *(ESI)* [M+H]⁺ = 506.2.

### Step G: Preparation of N-(2-(6-oxo-5-(trifluoromethyl)-1,6-dihydropyridin-3-yl)propoxy)-1-(5-(trifluor omethyl)pyrimidin-2-yl)-1,2,3,6-tetrahydropyridin-4-carboxamide

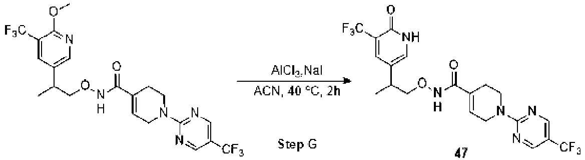

To a solution of N-(2-(6-methoxy-5-(trifluoromethyl)pyridin-3-yl)propoxy)-1-(5-(trifluoromethyl)pyri midin-2-yl)-1,2,3,6-tetrahydropyridine-4-carboxamide (50 mg, 0.1 mmol, 1.0 eq.) in acetonitrile (1 mL) was added aluminum trichloride (33 mg, 0.25 mmol, 2.5 eq.) and sodium iodide (30 mg, 0. 2 mmol, 2.0 eq.). The reaction mixture was reacted at 40 °C for 2 h. The reaction mixture was quenched with water (10 mL) and extracted with EtOAc (2 × 10 mL). The organic phases wer e combined, dried over anhydrous sodium sulfate, and concentrated. The crude product was purifi ed by prep-HPLC to obtain compound 47, N-(2-(6-oxo-5-(trifluoromethyl)-1,6-dihydropyridin-3-yl)p ropoxy)-1-(5-(trifluoromethyl)pyrimidin-2-yl)-1,2,3,6-tetrahydropyridine-4-carboxamide (8.86 mg, whi te solid), yield: 17.9%.

LCMS: *(ESI)* [M+H]⁺ = 492.0.

¹H NMR (400 MHz, DMSO-*d*₆) *δ* 12.25 (s, 1H), 11.19 (s, 1H), 8.74 (d, *J* = 0.6 Hz, 2H), 7.95 (d, 1H), 7.65 (s, 1H), 6.54 (s, 1H), 4.37 (d, *J* = 2.8 Hz, 2H), 3.95 (t, 2H), 3.90 - 3.82 (m, 2H), 3.03 - 2.90 (m, 1H), 2.34 (s, 2H), 1.16 (d, *J* = 7.1 Hz, 3H).

### Example 48

### Biological Detection

### Determination of the inhibitory effect of the compounds on PARP7 enzyme activity

Recombinant human PARP7 (Abcam, catalog number ab271651) was used for assay. First, the 384-well reaction plate was coated with 30 µL of histone coating buffer (1.5 µg/mL, purchased from Active Motif, Cat. No. 81167) at 37°C for 2 hours. After washing away the histone coating buffer, 30 µL of blocking buffer (PBS solution containing 0.05% Tween-20 and 5% BSA) was added to each well, and the plate was stirred at 25°C for 60 minutes before washing away the blocking buffer. Prepare the compound (1000×), starting from 1 mM and diluting it three-fold down to 0.05 µM. Transfer 25 nL of the prepared compound to each well in a 384-well plate using an Echo liquid handler, so that the final concentrations of the compound in the reaction system are 1000 nM, 333.33 nM, 111.11 nM, 37.03 nM, 12.34 nM, 4.11 nM, 1.37 nM, 0.45 nM, 0.15 nM, and 0.05 nM, which serve as the test wells; at the same time, add 25 nL of DMSO to the max control well. Prepare PARP7 enzyme solution (1.67×). Add 15 µL of the prepared PARP7 enzyme solution to each well. Simultaneously, add 15 µL of reaction buffer (20 mM HEPES solution, pH 7.5, containing 100 mM NaCl, 0.002% Tween-20, 2 mM DTT, and 0.1% BSA) to min control well. Incubate at 25°C for 15 minutes. Then was added 10 µL of Biotin-NAD+ substrate solution (2.5×, purchased from R&D, Cat. No. 6573) and incubate at 25°C for 60 minutes. Wash away the reaction mixture. Dilute horseradish peroxidase (HRP)-labeled streptavidin (Thermo Pierce, Cat. No. 21127) with blocking buffer at a ratio of 1:5000, add 30 µL to each well, and incubate at 25°C for 30 minutes. After washing away the HRP buffer, add 50 µL of HRP chemiluminescent substrate mixture (purchased from Thermo Pierce, Cat. No. 15159) to each well, and detect the luminescence signal (RLU) of each well using a microplate reader (Multi-Mode Detection Platform, manufacturer MOLECULAR DEVICES, model Paradigm).

Calculation formula: Inhibition rate % = (max control average - RLU of the compound) / (max control average - min control average)) × 100%. The dose-response curves of each compound were fitted using GraphPad Prism 8 and the IC₅₀ values were obtained.

### Experimental results are shown in Table 1.

**Table 1. Determination of the inhibitory activity of each compound on PARP7 enzyme ("-" indicates not measured)**

| Compounds | PARP7 IC₅₀ (nM) | Compounds | PARP 7 IC₅₀ (nM) | Compounds | PARP 7 IC₅₀ (nM) |
|---|---|---|---|---|---|
| 2 | 4.1 | 18 | 51 | 34 | 1.6 |
| 3 | - | 19 | 16 | 35 | 83 |
| 4 | 1.5 | 20 | 1.3 | 36 | 24 |
| 5 | 10.2 | 21 | - | 37 | 1.1 |
| 6 | 5.1 | 22 | - | 38 | 122 |
| 7 | 1.1 | 23 | - | 39 | 26 |
| 8 | 25 | 24 | 6.9 | 40 | 31 |
| 9 | - | 25 | 16 | 41 | 0.64 |
| 10 | 0.79 | 26 | - | 42 | 3.2 |
| 11 | 0.72 | 27 | 1.9 | 43 | 1.2 |
| 12 | - | 28 | 18 | 44 | 1.4 |
| 13 | 1 | 29 | 1.3 | 45 | 3 |
| 14 | 4.6 | 30 | 14 | 46 | 5.8 |
| 15 | 0.55 | 31 | 6.6 | 47 | 8.7 |
| 16 | 2.1 | 32 | 4.1 | | |
| 17 | - | 33 | 84 | | |

### Determination of the inhibitory activity of compounds 4 and 27 on PARP1 and PARP12 enzymes

In this example, the inhibitory activities of compounds 4 and 27 on other PARP enzymes (PARP1 and PARP12) were tested, and the inhibitory activities of compound RBN-2397 (Cat. No: HY-136174, MedChemExpress, Shanghai, China) on PARP7, PARP1, and PARP12 enzymes were tested to detect their selectivity in inhibiting PARP7.

The inhibition of PARP12 enzyme activity by the compounds was tested using recombinant human PARP12 (BPS, catalog number 80513). Except for the enzyme added, the other steps were the same as the test method for PARP7 enzyme activity, see the above test method for PARP7 enzyme activity for details.

The inhibition of PARP1 enzyme activity by the compound was determined using recombinant human PARP1 protein (BPS, catalog number 80501). First, a 384-well reaction plate was coated overnight at 4°C with 25 µL of histone coating buffer (1.5 µg/mL). After washing away the histone coating buffer, 50 µL of blocking buffer was added to each well, and the plate was stirred at 25°C for 60 minutes before washing away the blocking buffer. A stock solution of the compound (2000×) was prepared and tripled from 2 mM down to 0.1 µM, resulting in final concentrations of 1000 nM, 333.33 nM, 111.11 nM, 37.03 nM, 12.34 nM, 4.11 nM, 1.37 nM, 0.45 nM, 0.15 nM, and 0.05 nM in the reaction system. Add 50 nL of the prepared compound stock solution to 20 µL of reaction buffer, vortex to mix thoroughly, and then transfer 5 µL to a 384-well plate as the test wells; simultaneously, add an equal amount of DMSO to the max control well. Incubate the mixture of PARP1 and activated DNA (purchased from Genscript, Cat. No. L05182-01&02&03) at 25°C for 10 minutes. Then, add 10 µL of the mixture to the test wells and the max control well, and add 10 µL of DNA to the min control well, and incubate at 25°C for 10 minutes. Next, add 10 µL of Biotin-NAD+ substrate solution (2.5×), and incubate at 25°C for 60 minutes. Wash away the reaction mixture, add 20 µL of rabbit monoclonal antibody against poly/mono-ADP) ribose (purchased from CST, Cat. No. 83732S), and incubate at 25°C for 90 minutes. Wash three times with PBST (PBS solution containing 0.05% Tween-20). HRP-labeled anti-rabbit secondary antibody (purchased from CST, Cat. No. 7074P2) was diluted 1:2000 with blocking buffer, and 20 µL was added to each well. The mixture was stirred at 25°C for 60 minutes. After washing away the HRP buffer, 25 µL of HRP chemiluminescent substrate mixture (purchased from Thermo Pierce, Cat. No. 15159) was added to each well, and the luminescence signal (RLU) of each well was detected using a microplate reader (Multi-Mode Detection Platform, manufacturer: MOLECULAR DEVICES, model: Paradigm).

Calculation formula: Inhibition rate % = (max control average - RLU of the compound) / (max control average - min control average)) × 100%. The dose-response curves of each compound were fitted using GraphPad Prism 8 and the IC₅₀ values were obtained.

Experimental results are shown in Table 2.

**Table 2. Comparison of the enzyme inhibitory activities of compounds RBN-2397, 4, and 27 against PARP1 and PARP12 and their selectivity with PARP7**

| Compounds | PARP 7 | PARP 1 | PARP1 IC₅₀/PARP7 | PARP12 | PARP 12 IC₅₀/PARP7 |
|---|---|---|---|---|---|
| | IC₅₀ (nM) | IC₅₀ (nM) | IC₅₀(fold) | IC₅₀ (nM) | IC₅₀(fold) |
| RBN-2397 | 0.45 | 29 | 64.4 | 16 | 35.6 |
| 4 | 1.5 | 451 | 300.7 | 80 | 53.3 |
| 27 | 1.9 | 801 | 421.6 | 229 | 120.5 |

As shown in Table 2, compounds 4 and 27 exhibited fold selectivity for inhibiting PARP1 relative to PARP7, at 300.7 and 421.6 times respectively, significantly higher than the 64.4-fold selectivity of the reference compound RBN-2397. Similarly, compounds 4 and 27 showed fold selectivity for inhibiting PARP12 relative to PARP7, at 53.3 and 120.5 times respectively, significantly higher than the 35.6-fold selectivity of the reference compound RBN-2397. These results indicate that compounds 4 and 27 possess extremely high selective inhibitory activity against PARP7 enzymes.

### Determination of the compounds' ability to restore the type I interferon signaling pathway in cells

In the examples, the expression of STAT1 and p-STAT1 proteins in CT26 mouse colon cancer cells was measured to demonstrate whether the compounds could restore the type I interferon signaling pathway in cells.

CT26 cells in the exponential growth phase (purchased from Wuhan Pronosai Life Science Technology Co., Ltd., catalog number CL-0071) were digested into single-cell suspensions with trypsin (purchased from Thermo Fisher, catalog number 25300120), counted, and seeded into 12-well plates at 2 × 10⁵cells per well, and cultured overnight at 37°C. The day after seeding, stock solutions (1000×) of compounds 4 and 27 were prepared using DMSO (purchased from Sigma, catalog number D4540), and triple-diluted from 10 mM down to 0.1 mM. The prepared stock solutions were added to the 12-well plates to achieve final concentrations of 0.1, 0.3, 1, 3, and 10 µM for compounds 4 and 27, respectively. The negative control wells were treated with the same amount of DMSO as the drug-treated wells (final concentration 1‰), and the positive control wells were treated with RBN-2397 (prepared as 1000× stock solution in DMSO, final concentration 1 µM). After 24 hours of treatment, protein was extracted using SDS lysis buffer (Beyotime, catalog number P0013G) with protease inhibitor (Roche, catalog number 11836170001). Protein quantification was performed using a BCA kit (Thermo Fisher, catalog number 23227). After adjusting the protein concentration, routine Western blot experiments were conducted (p-STAT1 antibody: Santa Cruz, catalog number sc-136229; STAT1 antibody: Cell Signaling, catalog number 9172; β-actin antibody: Beyotime, catalog number AF2811).

The experimental results are shown in Figure 1. Both compounds 4 and 27 dose-dependently increased the expression of STAT1 and p-STAT1 proteins, indicating that both compounds could restore the type I interferon signaling pathway in cells.

### Pharmacokinetic Testing of Compounds

In this embodiment, pharmacokinetic tests were performed on compounds RBN-2397, 4, and 27 in mice to illustrate their pharmacokinetic properties in mice.

Preparation Method of Test Compound Solution:
Preparation of intravenous and oral gavage solutions: The analyte in a solution prepared with 5% DMSO, 50% PEG300, and 45% saline.

Dosage (based on the amount of compound in the administration solution (mg) / mouse body weight (kg): Intravenous injection (IV) 3 mg/kg, oral gavage (PO) 10 mg/kg.

Experimental Method for Pharmacokinetic Study in Mice:
Eighteen male CD-1 mice (Shanghai Jihui Experimental Animal Breeding Co., Ltd.), weighing 28-31 g, were fasted for 12 hours and randomly divided into two groups (Group A and Group B), with 9 mice in each group. Group A animals were administered the test compound solution by gavage at a dose of 10 mg/kg; Group B animals were administered the test compound solution by tail vein injection at a dose of 3 mg/kg. Blank blood samples were collected before administration. For Group A animals, approximately 110 µL of venous blood was collected at 0.25 h, 0.5 h, 1 h, 2 h, 4 h, 8 h, and 24 h after administration. For Group B animals, approximately 110 µL of venous blood was collected at 0.083 h, 0.25 h, 0.5 h, 1 h, 2 h, 4 h, 8 h, and 24 h after administration. All samples were placed in heparinized tubes, centrifuged, and the plasma was collected and stored at -70°C for testing.

Experimental Results are shown in Table 3.

**Table 3: Comparison of pharmacokinetic data for compounds RBN-2397, 4, and 27**

| **Compounds** | **IV 3mg/kg** | | | **PO 10mg/kg** | | |
|---|---|---|---|---|---|---|
| | AUC ₗₐₛₜ (ng/mL*hr) | CL(iv) (L/hr/kg) | T_{1/2} (hr) | AUC ₗₐₛₜ (ng/mL*hr) | Cₘₐₓ (ng/mL) | F% |
| RBN-2397 | 732 | 4.10 | 0.2 | 582 | 682 | 23.9 |
| Compound 4 | 6037 | 0.487 | 1.63 | 19377 | 7527 | 94.3 |
| Compound 27 | 3224 | 0.919 | 1.08 | 9388 | 5163 | 88.4 |

AUCₗₐₛₜ represents the area under the drug-time curve from the start of administration to the last point, i.e., the area enclosed by the plasma drug concentration curve on the time axis; CL(iv) represents the drug clearance rate after intravenous administration; T_{1/2} represents the plasma half-life of the drug, which refers to the time required for half of the drug to be eliminated from the body; Cₘₐₓ represents the maximum drug concentration reached in plasma after administration; F% represents the oral bioavailability of the drug.

As shown in Table 3, after tail vein injection of 3 mg/kg, the area under the curve (AUCₗₐₛₜ) of compound 4, i.e., the plasma exposure, was approximately 8.2 times that of RBN-2397, and the plasma exposure of compound 27 was approximately 4.4 times that of RBN-2397; the drug clearance rate (CL(iv)) of RBN-2397 after intravenous administration was 8.4 and 4.5 times that of compounds 4 and 27, respectively; and the plasma half-life (T_{1/2}) of compounds 4 and 27 was 8.2 and 5.4 times that of RBN-2397, respectively. Following oral administration of 10 mg/kg, the plasma exposures of compounds 4 and 27 were approximately 33.3 times and 16.1 times that of RBN-2397, respectively; the maximum plasma concentrations (Cₘₐₓ) were 11.0 times and 7.6 times that of RBN-2397, respectively; and the oral bioavailability (F%) were 3.9 times and 3.7 times that of RBN-2397, respectively. These results indicate that compounds 4 and 27 possess superior pharmacokinetic properties compared to RBN-2397, overcoming the shortcomings of RBN-2397 such as short half-life and poor oral bioavailability. They hold promise for superior clinical efficacy, and their excellent target inhibitory activity and pharmacokinetic properties offer greater potential for expanding their clinical indications.

### Industrial Applicability

This invention provides a PARP7 inhibitor and its use thereof, which can selectively inhibit the enzyme activity of PARP7 and can be used to treat or prevent diseases, disorders, or conditions regulated by or affected by PARP7 activity, or involving PARP7 activity or overexpression, and is therefore suitable for industrial applications.

## Claims

1. A PARP7 inhibitor, which is a compound of formula (I) or a pharmaceutically acceptable salt, solvate, ester, acid, metabolite or prodrug thereof, 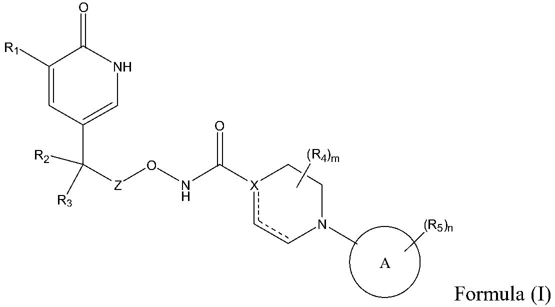 wherein,
R₁ is selected from halogen, C1-6 haloalkyl, and C2-6 alkanoyl;
R₂ is selected from hydrogen and deuterium;
R₃ is selected from hydrogen, C1-6 alkyl, C1-6 hydroxyalkyl, and C1-6 haloalkyl; or,
R₂ and R₃ together with adjacent carbon atom form C3-6 cycloalkyl;
Z is selected from direct bond or methylene;
X is selected from carbon and nitrogen, indicates a double bond or a single bond, provided that when X is nitrogen, the bond connected to X is a single bond, and when X is carbon, the bond connected to X is a double bond or a single bond, and at most one double bond is connected to any atom;
m is 0, 1, or 2, and when m is 1, R₄ is selected from C1-6 alkyl; when m is 2, the two R₄ are located on different ring atoms, and the two R₄ together form an alkylene bridging bond containing 1-3 carbon atoms;
ring A is a 5-6 membered monocyclic heteroaryl or a 9-10 membered bicyclic heteroaryl having 1-3 nitrogen atoms;
n is 0, 1, or 2, and each R₅ is independently selected from nitrile, halogen, C3-6 cycloalkyl, C1-6 alkyl, C2-6 alkenyl, C2-6 alkynyl, C1-6 haloalkyl, C2-6 alkylamido, C1-4 alkylsulfinamide, C1-4 alkylsulfonamide, and phenyl optionally substituted with one or two halogens.

2. The PARP7 inhibitor of claim 1, wherein,
ring A is selected from pyrimidinyl, pyridinyl, pyrrolopyridinyl, imidazopyridinyl, and quinazolinyl; ring A is more preferably selected from pyrimidin-2-yl, pyridin-2-yl, pyrrolo[2,3-c]pyridin-7-yl, imidazo[4,5-c]pyridin-4-yl, and quinazolin-2-yl; ring A is most preferably pyrimidin-2-yl or imidazo[4,5-c]pyridin-4-yl.

3. The PARP7 inhibitor of claim 1 or 2, wherein,
n is 1; and /or,
R₅ is selected from halogen, C3-6 cycloalkyl, C1-3 haloalkyl, and phenyl optionally substituted with one halogen; R₅ is more preferably selected from trifluoromethyl or cyclopropyl; and /or,
the substitution position of R₅ on ring A is para-position relative to the connection position of ring A with the nitrogen-containing six-membered heterocyclic group.

4. The PARP7 inhibitor of any one of claims 1-3, wherein,
X is N, and both are single bonds; and /or,
m is 0, 1, or 2, more preferably 0 or 1;
when m is 1, R₄ is a C1-3 alkyl, more preferably methyl; and /or, R₄ is located ortho to the X atom of the nitrogen-containing six-membered heterocyclic group;
when m is 2, the two R₄ on different ring atoms together form a bridging bond selected from methylene or ethylene; and /or, the two R₄ are respectively located ortho to the X atom of the nitrogen-containing six-membered heterocyclic group or respectively located meta to the X atom of the nitrogen-containing six-membered heterocyclic group.

5. The PARP7 inhibitor of any one of claims 1-3, wherein,
X is C or CH, and one of the two - - - is a double bond and the other is a single bond; more preferably, X is C, and one of the two - - - bonds that connects to the X atom is a double bond, and the other is a single bond; and /or,
m is 0 or 1, more preferably 0;
when m is 1, R₄ is C1-3 alkyl, more preferably methyl.

6. The PARP7 inhibitor of any one of claims 1-5, wherein Z is a direct bond.

7. The PARP7 inhibitor of any one of claims 1-6, wherein,
R₂ is hydrogen; and /or,
R₃ is selected from hydrogen, C1-3 alkyl, and C1-3 hydroxyalkyl; R₃ is more preferably methyl or ethyl.

8. The PARP7 inhibitor of any one of claims 1-7, wherein,
R₁ is C1-3 haloalkyl, more preferably trifluoromethyl.

9. The PARP7 inhibitor of claim 1, wherein the compound of formula (I) is selected from:

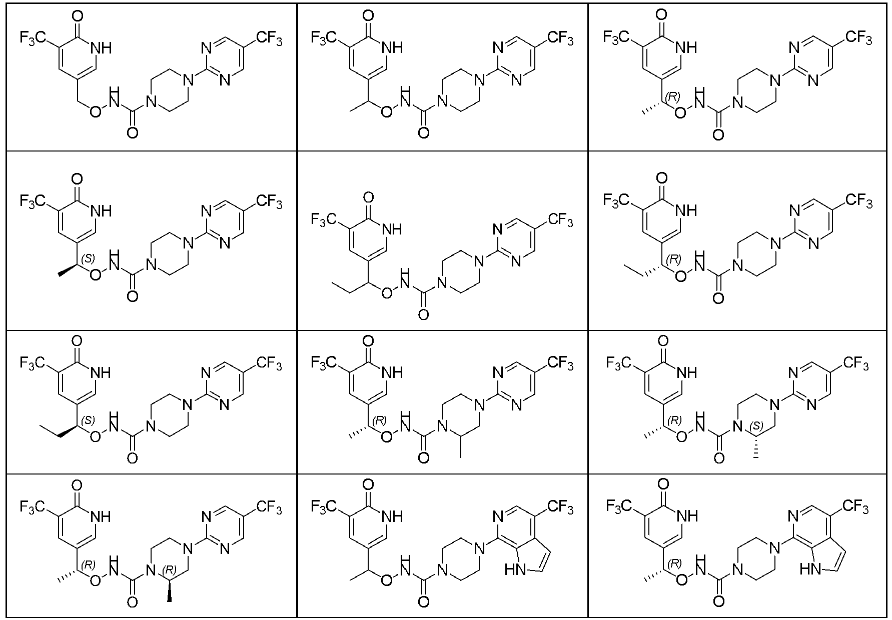

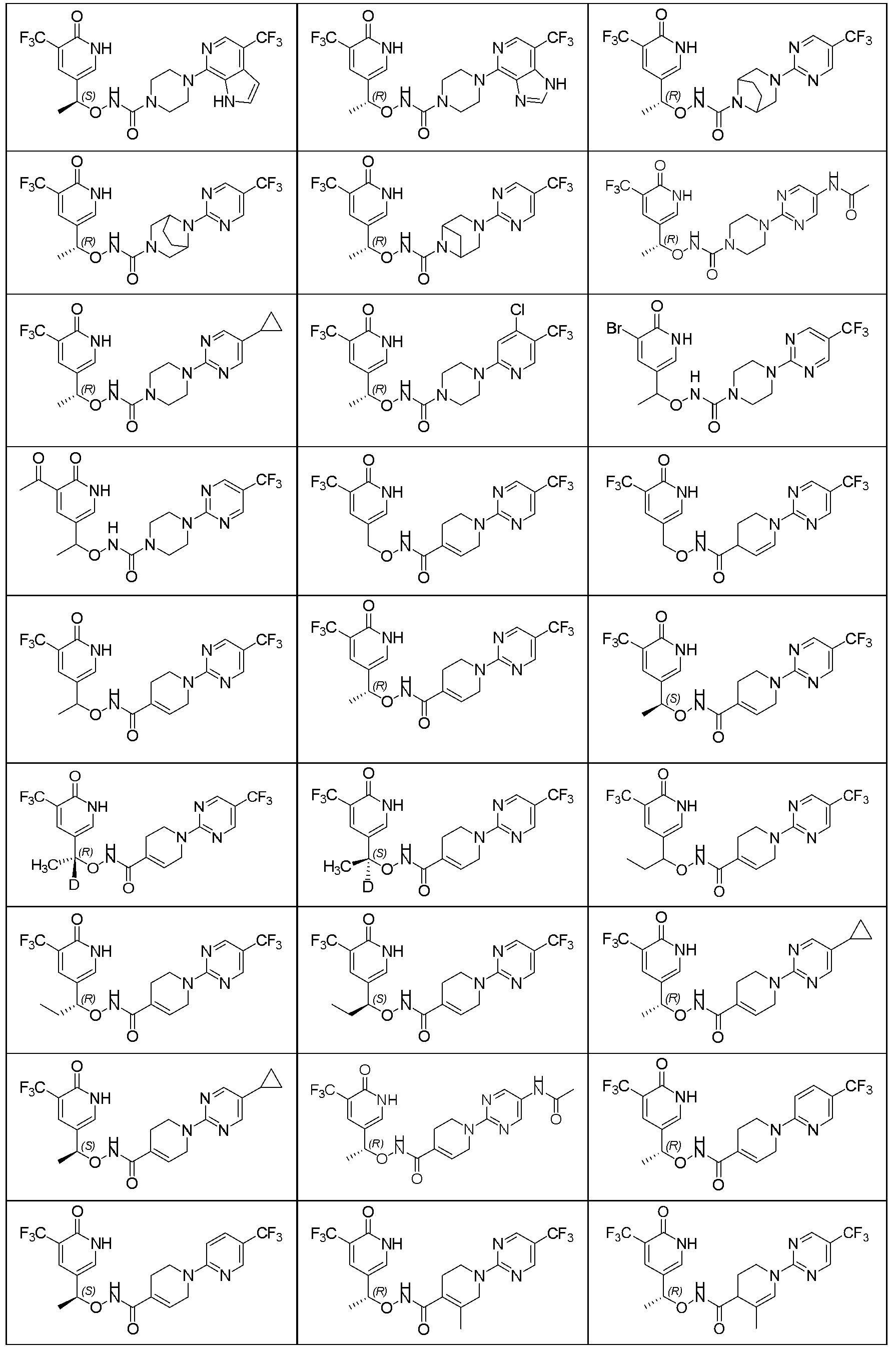

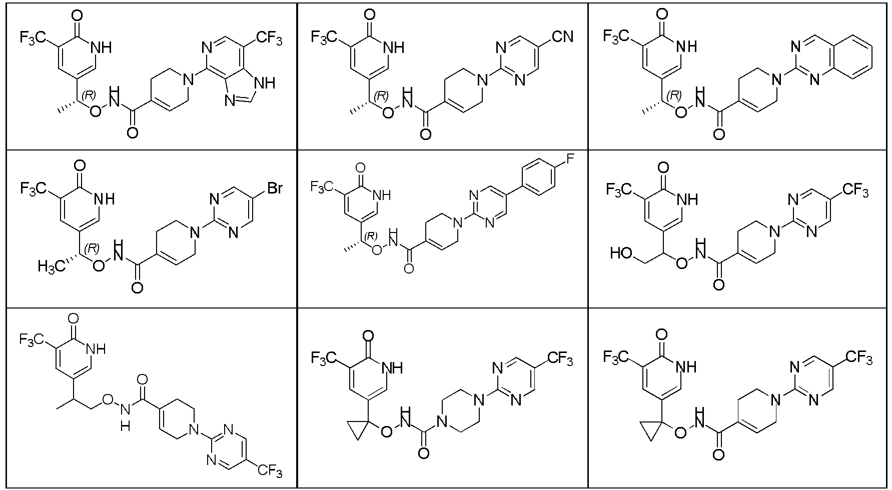

10. A pharmaceutical composition comprising the PARP7 inhibitor according to any one of claims 1-9, and a pharmaceutically acceptable carrier or excipient, and optionally other therapeutic agents.

11. Use of the PARP7 inhibitor according to any one of claims 1-9 in the preparation of a medicament for treating or preventing a disease, disorder, or condition regulated or affected by PARP7 activity, or in which PARP7 activity or overexpression is involved.

12. The use as claimed in claim 11, wherein the disease, disorder, or condition is a hyperproliferative disease, an autoimmune disease, or an inflammatory disease.

13. The use as claimed in claim 11 or 12, wherein the disease, disorder, or condition is a cancer selected from lung squamous cell carcinoma, lung adenocarcinoma, large cell lung cancer, small cell lung cancer, head and neck squamous cell carcinoma, breast cancer, pancreatic cancer, pancreatic ductal adenocarcinoma, colorectal cancer, melanoma, ovarian cancer, esophageal squamous cell carcinoma, gastric cancer, liver cancer, oral cancer, urothelial carcinoma, prostate cancer, bladder cancer, renal cell carcinoma, gastrointestinal stromal tumor, cervical cancer, endometrial cancer, rhabdomyosarcoma, fibrosarcoma, neuroendocrine tumor, mesothelioma, brain cancer, or malignant glioma, or
the disease, disorder or condition is an autoimmune disease or an inflammatory disease selected from ulcerative colitis, Crohn's disease, multiple sclerosis, autoimmune liver disease, type 1 diabetes mellitus, bronchial asthma, systemic lupus erythematosus, rheumatoid arthritis, ankylosing spondylitis, juvenile idiopathic arthritis, psoriasis, polymyositis, or dermatomyositis.

14. The PARP7 inhibitor according to any one of claims 1-9 for use in the treatment or prevention of a disease, disorder, or condition regulated or affected by PARP7 activity, or in which PARP7 activity or overexpression is involved.

15. The PARP7 inhibitor for use as claimed in claim 14, wherein the disease, disorder, or condition is a hyperproliferative disease, an autoimmune disease, or an inflammatory disease.

16. The PARP7 inhibitor for use as claimed in claim 14 or 15, wherein the disease, disorder, or condition is a cancer selected from lung squamous cell carcinoma, lung adenocarcinoma, large cell lung cancer, small cell lung cancer, head and neck squamous cell carcinoma, breast cancer, pancreatic cancer, pancreatic ductal adenocarcinoma, colorectal cancer, melanoma, ovarian cancer, esophageal squamous cell carcinoma, gastric cancer, liver cancer, oral cancer, urothelial carcinoma, prostate cancer, bladder cancer, renal cell carcinoma, gastrointestinal stromal tumor, cervical cancer, endometrial cancer, rhabdomyosarcoma, fibrosarcoma, neuroendocrine tumor, mesothelioma, brain cancer, or malignant glioma, or
the disease, disorder or condition is an autoimmune disease or an inflammatory disease selected from ulcerative colitis, Crohn's disease, multiple sclerosis, autoimmune liver disease, type 1 diabetes mellitus, bronchial asthma, systemic lupus erythematosus, rheumatoid arthritis, ankylosing spondylitis, juvenile idiopathic arthritis, psoriasis, polymyositis, or dermatomyositis.
